(19) Europäisches Patentamt
European Patent Office
Office européen des brevets

(11) EP 0 409 410 B1

(12) **EUROPEAN PATENT SPECIFICATION**

(45) Date of publication and mention of the grant of the patent:
**20.03.1996 Bulletin 1996/12**

(51) Int. Cl.$^6$: **C07D 209/70**, A61K 31/40

(21) Application number: **90306718.9**

(22) Date of filing: **20.06.1990**

(54) **Indenoindole compounds**

Indenoindolverbindungen

Composés de l'indénoindole

(84) Designated Contracting States:
**AT BE CH DE DK ES FR GB GR IT LI LU NL SE**

(30) Priority: **22.06.1989 SE 8902274**

(43) Date of publication of application:
**23.01.1991 Bulletin 1991/04**

(73) Proprietors:
- **UNIVERSITY OF BATH**
  **Claverton Down, Bath, BA2 7AY (GB)**
- **UNIVERSITY OF CINCINNATI**
  **Cincinnati, Ohio 45221-0627 (US)**

(72) Inventors:
- **Sainsbury, Malcolm**
  **Saltford, Bristol BS18 3EF (GB)**
- **Shertzer, Howard G.**
  **Cincinnati, OH 45255 (US)**

(74) Representative: **Goldin, Douglas Michael**
**J.A. KEMP & CO.**
**14, South Square**
**Gray's Inn**
**London WC1R 5LX (GB)**

(56) References cited:
- **CHEMICAL ABSTRACTS, vol. 100, 1984, page 517, abstract 191091w, Columbus, OH, US; K. BILL et al.: "The coupling reactions of 3-acylindoles and proof of structure of the palladium(II) acetate meiated cyclization reaction product of 3-benzoyl-1-methylindole"**
- **FASEB JOURNAL, vol. 2, 1988, page A407, abstract 648; H.G. SHERTZNER et al.: "M. SAINSBURY: 'Amellioration of carbon tetrachloride hepatotoxicity in mice by indenoindole compounds'"**
- **FD CHEM. TOXIC, vol. 26, no. 6, 1988, pages 517-522; H.G. SHERTZNER et al.: "Protection agains carbon tetrachloride hepatotoxicity by 5,10-dihydroindeno[1,2-b] indole, a potent inhibitor of lipid peroxidation"**
- **ANNALEN DER CHEMIE, vol. 61, 1928; LEUCHS et al., pp. 27-45**

Remarks:
The file contains technical information submitted after the application was filed and not included in this specification

EP 0 409 410 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

The present invention relates to a novel type of hydrophobic antioxidant, based on the indenoindole structure, which is highly efficient in reducing, i.e. quenching, free radicals in lipids or lipid biphases, thereby terminating the lipid peroxidation process and preventing conditions and diseases initiated by this or related processes. The invention also relates to compositions, especially pharmaceutical compositions, containing at least one compound of the invention, or a salt thereof, especially a therapeutically acceptable salt thereof, as active ingredient. In a further aspect, the invention relates to processes for the preparation of such compounds. In a further aspect, the invention relates to processes in the preparation of such compounds as well as to non-medical applications. and prevention as well as in non-medical applications. Especially important in non-medical applications would be the use in controlling or terminating free-radical mediated processes.

### Background of the Invention

Some biological processes generate more or less stable intermediates that contain an unpaired electron, which can either be donated, or paired with an additional electron from the surroundings. Such intermediates are called free radicals, and they may be the products of various enzymatic and non-enzymatic reactions, some of which are vital for body functions, e.g. reduction of ribonucleoside diphosphates for DNA synthesis and the generation of prostaglandins in the prostaglandin synthase reaction. The latter is essential for inflammatory response following cell injury, and a number of other functions. Other radical reactions include the myeloperoxidase reaction in neutrophils and macrophages which destroy bacteria and other invading particles, and the electron transport in the mitochondrial respiratory chain. Most organisms contain chemical antioxidants such as $\alpha$-tocopherol (vitamin E), ascorbic acid and different radical and peroxide-inactivating enzymes, e.g. superoxide dismutase, catalase and glutathione peroxidase.

Free radicals of various types are becoming increasingly associated with a broad range of conditions and diseases such as ischemic or reperfusion injury, atherosclerosis, thrombosis and embolism, allergic/inflammatory conditions such as bronchial asthma, rheumatoid arthritis, conditions related to Alzheimer's disease, Parkinson's disease and ageing, cataract, diabetes, neoplasms and toxicity of anti-neoplastic or immunosuppresive agents and chemicals. One possible explanation for these conditions and diseases is that, for unknown reasons, the endogeneous protecting agents against radical damage are not sufficiently active to protect the tissue against radical damage. Lipid peroxidation caused by excess generation of radicals may constitute one significant damaging pathway in the above conditions and diseases. Administration of additional antioxidants, which inhibit radical reactions, e.g. lipid peroxidation, would thus provide a way of preventing or curing the above conditions and diseases. The present invention describes new antioxidants of the indenoindole type that fulfils both the requirement to accumulate in membranes, i.e. they are sufficiently hydrophobic, and they are potent inhibitors of lipid peroxidation. These new antioxidants compare favourably with other antioxidants, e.g. $\alpha$-tocopherol. The compounds of the present invention may also be used in non-medical applications for stabilising compounds susceptable to oxidative deterioration, e.g. in skin care products, food preservation, food additives and for preservation of other products. The present invention extends to both a method of stabilisation using the tetrahydroindenoindoles and the resulting stabilised compositions.

### Prior art

N-methyl-4b,5,9b,10-tetrahydroindeno[1,2-b]indole is disclosed in J.Chem. Soc. Chem. Commun. p. 647-48. (1981) 4b,5,9b,10-tetrahydro-9b-ethylindeno[1,2-b]indole is disclosed in Beilsteins Handbuch Der Organischen Chemie, 4:e Auflage, It.20 EII, p. 310-311 (1953).

### Disclosure of the Invention

It has been found that compounds with the tetrahydroindeno-indole structures of formulae IA (THII) and IB (iso-THII) are effective as inhibitors of the lipid peroxidation process and useful as antioxidants, IA or IB may be present as a racemic mixture or in the enantiomeric form,

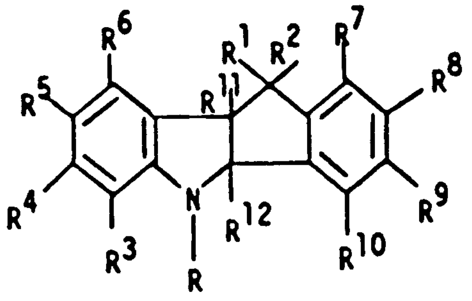

IA

or

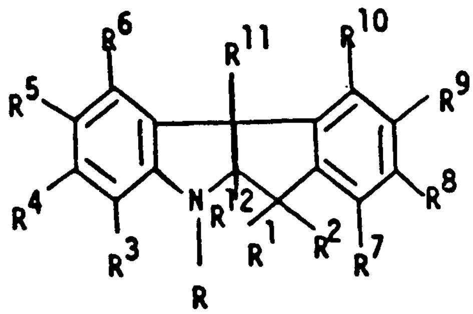

IB

wherein R is hydrogen, an alkyl group or $COR^{15}$,
$R^1$, $R^2$,$R^{11}$ and $R^{12}$ are independently selected from hydrogen, or a lower alkyl group,
$R^3$, $R^4$, $R^5$ and $R^6$ are independently selected from hydrogen, hydroxy, halogen, a lower alkyl group, a lower alkoxy group, a mono- or di-lower alkylamino group, $NH_2$ or $NR^{13}COR^{14}$,
$R^7$, $R^8$, $R^9$ and $R^{10}$ are independently selected from hydrogen, hydroxy, a lower alkyl group, a lower alkoxy group, a mono- or di-lower alkylamino group, $NH_2$ or $NR^{13}COR^{14}$, $R^{13,}$ $R^{14}$ and $R^{15}$ independently selected from are hydrogen or a lower alkyl group, with the proviso that when R is $COR^{14}$ then at least one of $R^3$ to $R^{10}$ are hydroxy or a mono- or di-lower alkylamino group,
and enantiomers or salts thereof.

The novel compounds of the present invention have either formula IA or IB

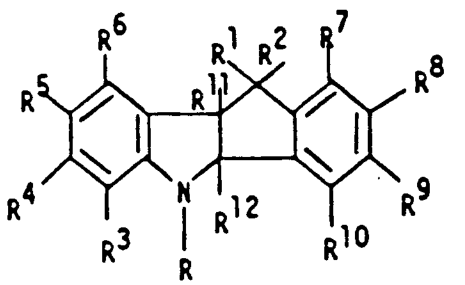

IA

or

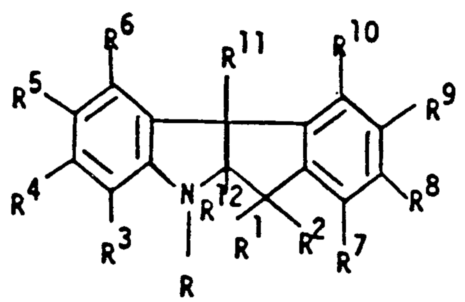

IB

wherein R is hydrogen, an alkyl group or $COR^{15}$,
$R^1$, $R^2$, $R^{11}$ and $R^{12}$ are independently selected from hydrogen, or a lower alkyl group,
$R^3$, $R^4$, $R^5$ and $R^6$ are independently selected from hydrogen, hydroxy, halogen, a lower alkyl group, a lower alkoxy group, a mono- or di-lower alkylamino group, $NH_2$ or $NR^{13}COR^{14}$,
$R^7$, $R^8$, $R^9$ and $R^{10}$ are independently selected from hydrogen, hydroxy, a lower alkyl group, a lower alkoxy group, a mono- or di-lower alkylamino group, $NH_2$ or $NR^{13}COR^{14}$, $R^{13}$, $R^{14}$ and $R^{15}$ are independently selected from hydrogen or a lower alkyl group, with the following provisos:

i) when R is methyl in formula IA then at least one of the radicals $R^1$ to $R^{12}$ is not hydrogen;

ii, when R is hydrogen or acetyl and $R^{11}$ is ethyl in formula IA then at least one of the radicals $R^1$ to $R^{10}$ or $R^{12}$ is not hydrogen, and enantiomers and salts thereof.

The following compounds of formula IA (THII) and IB (iso-THII) which are effective as inhibitors of the lipid peroxidation process are particularly useful as anti-oxidants in the medical therapy,

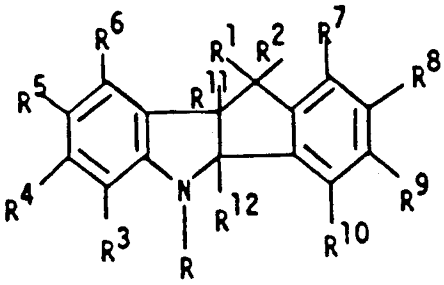

or

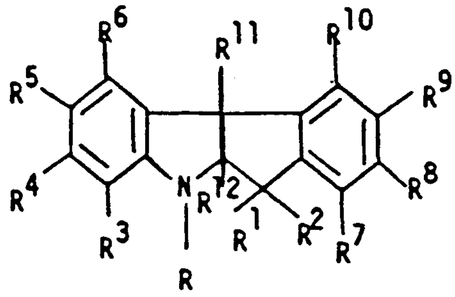

IA IB

wherein R is hydrogen, an alkyl group or $COR^{15}$,
$R^1$, $R^2$, $R^{11}$ and $R^{12}$ are independently selected from hydrogen, or a lower alkyl group,
$R^3$, $R^4$, $R^5$ and $R^6$ are independently selected from hydrogen, hydroxy, halogen, a lower alkyl group, a lower alkoxy group, a mono- or di-lower alkylamino group, $NH_2$ or $NR^{13}COR^{14}$,
$R^7$, $R^8$, $R^9$ and $R^{10}$ are independently selected from hydrogen, hydroxy, a lower alkyl group, a lower alkoxy group, a mono- or di-lower alkylamino group, $NH_2$ or $NR^{13}COR^{14}$,
$R^{13}$, $R^{14}$ and are independently selected from hydrogen or a lower alkyl group,
with the proviso that when R is $COR^{15}$ then at least one of $R^3$ to $R^{10}$ are hydroxy or a mono- or di-lower alkylamino group, and enantiomers and pharmaceutically acceptable salts thereof.

The indenole and iso-indenoindole structures of the present invention have the following numbering in the rings.

INDENOINDOLE STRUCTURE

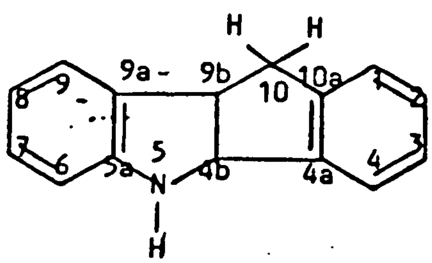

4b,5,9b,10-Tetrahydroindeno[1,2-b]indole(THII)

ISO-INDENOINDOLE STRUCTURE

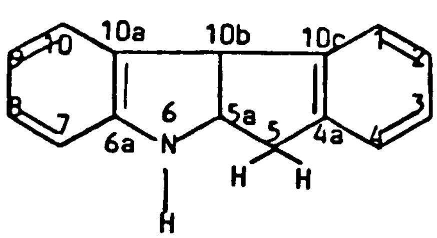

5,5A,6,10b-tetrahydroindeno[2,1-b]indole(iso-THII)

The alkyl group in the definition of R is an alkyl group having 1 - 24 carbon atoms e.g. methyl, ethyl, n-propyl, iso-propyl, n-butyl, sec-butyl, tert-butyl, pentyl, hexadecyl, octadecyl.

The term "lower" in the definition of substituents in the compound of the present invention means a number of carbon atoms not more than 6, preferably not more than 4.

The lower alkyl group in the definition of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$ and $R^{15}$ is an alkyl group having 1 - 6 carbon atoms, preferably 1 - 4 carbon atoms e.g. methyl, ethyl, n-propyl, isopropyl, n-butyl, sec-butyl or tert-butyl, preferred are methyl and ethyl.

The lower alkoxy group in the definition of $R^3$, $R^4$, $R^5$ and $R^6$, $R^7$, $R^8$, $R^9$ and $R^{10}$ is an alkoxy group having 1 - 6 carbon atoms, preferably 1 - 4 carbon atoms e.g. methoxy, ethoxy, n-propoxy, iso-propoxy, n-butoxy, sec-butoxy or tert-butoxy, preferred are methoxy and ethoxy.

Halogen in the definition of $R^3$, $R^4$, $R^5$ and $R^6$ is chlorine, bromine, iodine or fluorine.

The mono- or di-lower alkylamino group in the definition of $R^3$, $R^4$, $R^5$, $R^6$, $R^7$, $R^8$, $R^9$ and $R^{10}$ include methylamino, dimethylamino, ethylamino, diethylamino, propylamino, dipropylamino, butylamino, dibutylamino, preferred are ethylamino and diethylamino.

Preferred groups of compounds of the invention are those wherein R, $R^1$, $R^2$, $R^4$, $R^6$ and $R^{10}$ are hydrogen and $R^5$ and/or $R^8$ are a lower alkoxy group, particularly methoxy and/or $R^3$, $R^5$, $R^7$, $R^9$, $R^{11}$ and/or $R^{12}$ are a lower alkyl group, particularly methyl, ethyl, i-propyl and those compounds wherin $R^5$ and/or $R^8$ are a mono- or di-alkylamino group, particularly ethylamino or diethylamino.

Examples of compounds of the tetrahydroindenoindoles having formula IA and IB, which are included in the present invention are the following:

cis-4b,5,9b,10-tetrahydroindeno[1,2-b]indole
cis-4b,5,9b,10-tetrahydro-6,8-dimethylindeno[1,2-b]indole
cis-4b,5,9b,10-tetrahydro-5,8-dimethylindeno[1,2-b]indole
cis-4b,5,9b,10-tetrahydro-8-methylindeno[1,2-b]indole
cis-4b,5,9b,10-tetrahydro-4b,6,8,9b,tetramethylindeno[1,2-b]indole
cis-4b,5,9b,10-tetrahydro-8-isopropylindeno[1,2-b]indole
cis-4b,5,9b,10-tetrahydro-8-methoxy-5-methylindeno[1,2-b]indole
cis-4b,5,9b,10-tetrahydro-8-methoxyindeno[1,2-b]indole
cis-4b,5,9b,10-tetrahydro-10,10-dimethylindeno[1,2-b]indole
cis-4b,5,9b,10-tetrahydro-9b-methylindeno[1,2-b]indole
cis-4b,5,9b,10-tetrahydro-4b,9b-dimethylindeno[1,2-b]indole
cis-4b,5,9b,10tetrahydro-4b,5,9b,trimethylindeno[1,2-b]indole
cis-4b,5,9b,10-tetrahydro-2-methoxy-1,3-dimethylindeno[1,2-b]indole
cis-4b,5,9b,10-tetrahydro-2-methoxy-1,3-dimethyl-8-isopropylindeno-[1,2-b]indole
cis-4b,5,9b,10-tetrahydro-4b-methylindeno[1,2-b]indole
cis-4b,5,9b,10-tetrahydro-2-hydroxy-1,3-dimethyl-8-isopropylindeno[1,2-b]indole
cis-4b,5,9b,10-tetrahydro-2-hydroxy-1,3-dimethylindeno[1,2-b]indole
cis-4b,5,9b,10-tetrahydro-4b,8,9b-trimethylindeno[1,2-b]indole
cis-4b,5,9b,10-tetrahydro-8-isopropyl-4b,9b-dimethylindeno[1,2-b]indole
cis-4b,5,9b,10-tetrahydro-8-isopropyl-4b-methylindeno[1,2-b]indole
cis-4b,5,9b,10-tetrahydro-2,8-dimethoxy-1,3-dimethylindeno[1,2-b]indole
cis-4b,5,9b,10-tetrahydro-4b,5,8,9b-tetramethylindeno[1,2-b]indole
cis-4b,5,9b,10-tetrahydro-8-tert.butylindeno[1,2-b]indole
cis-4b,5,9b,10-tetrahydro-8-methoxy-7,9-dimethylindeno[1,2-b]indole
cis-4b,5,9b,10-tetrahydro-8-methoxy 6-methylindeno[1,2-b]indole
cis-4b,5,9b,10-tetrahydro-8-diethylamino-5 ethylindeno[1,2-b]indole
cis-4b,5,9b,10-tetrahydro-2-diethylaminoindeno[1,2-b]indole
cis-4b,5,9b,10-tetrahydro-8-tert.butyl-4b-methylindeno[1,2-b]indole
cis-4b,5,9b,10-tetrahydro-8-fluoroindeno[1,2-b]indole
cis-5,5a,6,10b-tetrahydroindeno[2,1-b]indole
cis-5,5a,6,10b-tetrahydro-9-methoxyindeno[2,1-b]indole
cis-5,5a,6,10b-tetrahydro-9-isopropylindeno[2,1-b]indole
cis-4b,5,9b,10-tetrahydro-8-methoxy-4b,6-dimethylindeno[1,2-b]indole
cis-4b,5,9b,10-tetrahydro-8-methoxy-4b,5,6-trimethylindeno[1,2-b]indole
cis-4b,5,9b,10-tetrahydro-8-methoxy-5,6-dimethylindeno[1,2-b]indole
cis-4b,5,9b,10-tetrahydro-8-methoxy-6-isopropylindeno[1,2-b]indole
cis-4b,5,9b,10-tetrahydro-8-methoxy-4b-methyl-6-isopropylindeno[1,2-b]indole
cis-4b,5,9b,10-tetrahydro-8-methoxy-4,6-dimethylindeno[1,2-b]indole
cis-4b,5,9b,10-tetrahydro-8-methoxy-4,4b,6-trimethylindeno[1,2-b]indole

cis-4b,5,9b,10-tetrahydro-4b,5,6,8,9b-pentamethylindeno[1,2-b]indole
cis-4b,5,9b,10-tetrahydro-8-diethylamino-6-methylindeno[1,2-b]indole
cis-4b,5,9b,10-tetrahydro-8-diethylamino-4b,6-dimethylindeno[1,2-b]indole
cis-4b,5,9b,10-tetrahydro-8-diethylamino-4b,5,6-trimethylindeno[1,2-b]indole
cis-4b,5,9b,10-tetrahydro-8-methoxy-6,10,10-trimethylindeno[1,2-b]indole
cis-4b,5,9b,10-tetrahydro-8-methoxy-4b,6,10,10-tetramethylindeno[1,2-b]indole
cis-4b,5,9b,10-tetrahydro-8-diethylamino-6,10,10-trimethylindeno[1,2-b]indole
cis-4b,5,9b,10-tetrahydro-8-diethylamino-4b,6,10,10-tetramethylindeno[1,2-b]indole
cis-4b,5,9b,10-tetrahydro-8-hydroxy-7,9-dimethylindeno[1,2-b]indole
cis-4b,5,9b,10-tetrahydro-8-hydroxy-4b,7,9-trimethylindeno[1,2-b]indole
cis-4b,5,9b,10-tetrahydro-8-hydroxy-7,9-ditert.butylindeno[1.2-b]indole
cis-4b,5,9b,10-tetrahydro-8-hydroxy-6,7,9-trimethylindeno[1,2-b]indole
cis-4b,5,9b,10-tetrahydro-8-hydroxy-4b,6,7,9-tetramethylindeno[1,2-b]indole
cis 4b,5,9b,10-tetrahydro-8-methoxy-4b,6,9b-trimethylindeno[1,2-b]indole
cis-4b,5,9b,10-tetrahydro-8-methoxy-4b,5,6,9b-tetramethylindeno[1,2-b]indole
cis-4b,5,9b,10-tetrahydro-8-diethylamino-4b,6,9b-trimethylindeno[1,2-b]indole
cis-4b,5,9b,10-tetrahydro-8-diethylamino-4b,5,6,9b-tetramethylindeno[1,2-b]indole
cis-5,5a,6,10b-tetrahydro-9-methoxy-7-methylindeno[2,1-b]indole,
cis-5,5a,6,10b-tetrahydro-9-methoxy-5a,7-dimethylindeno[2,1-b]indole
cis-5,5a,6,10b-tetrahydro-9-diethylamino-7-methylindeno[2,1-b]indole
cis-5,5a,6,10b tetrahydro-9-diethylamino-5a,7-dimethylindeno[2,1-b]indole
cis-5,5a,6,10b-tetrahydro-9-hydroxy-8,10-dimethylindeno[2,1-b]indole
cis-5,5a,6,10b-tetrahydro-9-hydroxy-7,8,10-trimethylindeno[2,1-b]indole
cis-5,5a,6,10b-tetrahydro-9-hydroxy-5a,7,8,10-tetramethylindeno[2,1-b]indole
cis-5,5a,6,10b-tetrahydro-9-diethylaminoindeno [2,1-b]indole
cis-4b,5,9b,10-tetrahydro-6-isopropylindeno[1,2-b]indole
cis-5,5a,6,10b-tetrahydro-9-methoxy-5,5,7-trimethylindeno[2,1 b]indole
cis-5,5a,6,10b-tetrahydro-9-diethylamino-5,5,7-trimethylindeno[2,1-b]indole
cis-4b,5,9b,10-tetrahydro-2-diethylamino-8-methoxy-6-methylindeno[1,2-b]indole
cis-4b,5,9b,10-tetrahydro-8-methoxy-4b-isopropylindeno[1,2-b]indole
cis-4b,5,9b,10-tetrahydro-8-methoxy-4b-isopropyl-6-methylindeno[1,2-b]indole
cis-4b,5,9b,10-tetrahydro-8-isopropyl-5-methylindeno[1,2-b]indole
cis-4b,5,9b,10-tetrahydro-8-methoxy-6-ethylindeno[1,2-b]indole
cis-4b,5,9b,10-tetrahydro-3-methoxy-4b-methylindeno[1,2-b]indole
cis-4b,5,9b,10-tetrahydro-7-methoxy-4b-methylindeno[1,2-b]indole
cis-5,5b,6,10b,tetrahydro-3-hydroxy-2,4-dimethylindeno[2,1-b]
cis-4b,5,9b,10-tetrahydro-8-acetamido-6-methylindeno[1,2-b]indole
cis-4b,5,9b,10-tetrahydro-2-acetamido-8-methoxy-6-methylindeno[1,2-b]indole
cis-4b,5,9b,10-tetrahydro-8-tert.butyl-5-methylindeno[1,2-b]indole
cis-5,5b,6,10b tetrahydro-3-acetamidoindeno[2,1-b]indole
cis-4b,5,9b,10tetrahydro-2-acetamidoindeno[1,2-b]indole
cis-5,5a,6,10b-tetrahydro-6-methylindeno[2,1-b]indole
cis-5,5a,6,10b-tetrahydro-6-ethyl-9-isopropylindeno[2,1-b]indole
cis-5,5a,6,10b-tetrahydro-9-fluoroindeno[2,1-b]indole
cis-5,5a,6,10b-tetrahydro-9-tert.butylindeno[2,1-b]

Preferred tetrahydroindenoindole compounds of the present invention having antioxidant activity are the following:

cis-4b,5,9b,10-tetrahydroindeno[1,2-b]indole
cis-4b,5,9b,10-tetrahydro-6,8-dimethylindeno[1,2-b]indole
cis-4b,5,9b,10-tetrahydro-5,8-dimethylindeno[1,2-b]indole
cis-4b,5,9b,10-tetrahydro-8-methylindeno[1,2-b]indole
cis-4b,5,9b,10-tetrahydro-4b,6,8,9b,tetramethylindeno[1,2-b]indole
cis-4b,5,9b,10-tetrahydro-8-isopropylindeno[1,2-b]indole
cis-4b,5,9b,10-tetrahydro-8-methoxy-5-methylindeno[1,2-b]indole
cis-4b,5,9b,10-tetrahydro-8-methoxyindeno[1,2-b]indole
cis-4b,5,9b,10-tetrahydro-10,10-dimethylindeno[1,2-b]indole
cis-4b,5,9b,10-tetrahydro-9b-methylindeno[1,2-b]indole
cis-4b,5,9b,10-tetrahydro-4b,9b-dimethylindeno[1,2-b]indole
cis-4b,5,9b,10-tetrahydro-4b,5,9b,trimethylindeno[1,2-b]indole
cis-4b,5,9b,10-tetrahydro-2-methoxy-1,3-dimethylindeno[1,2-b]indole

cis-4b,5,9b,10-tetrahydro-2-methoxy-1,3-dimethyl-8-isopropylindeno[1,2-b]indole
cis-4b,5,9b,10-tetrahydro-4b-methylindeno[1,2-b]indole
cis-4b,5,9b,10-tetrahydro-2-hydroxy-1,3-dimethyl-8-isopropylindeno[1,2-b]indole
cis-4b,5,9b,10-tetrahydro-2-hydroxy-1,3-dimethylindeno[1,2-b]indole
cis-4b,5,9b,10-tetrahydro-4b,8,9b-trimethylindeno[1,2-b]indole
cis-4b,5,9b,10-tetrahydro-8-isopropyl-4b,9b-dimethylindeno[1,2-b]indole
cis-4b,5,9b,10-tetrahydro-8-isopropyl-4b-methylindeno[1,2-b]indole
cis-4b,5,9b,10-tetrahydro-2,8-dimethoxy-1,3-dimethylindeno[1,2-b]indole
cis-4b,5,9b,10-tetrahydro-4b,5,8,9b-tetramethylindeno[1,2-b]indole
cis-4b,5,9b,10-tetrahydro-8-tert.butylindeno[1,2-b]indole
cis-4b,5,9b,10-tetrahydro-8-methoxy-7,9-dimethylindeno[1,2-b]indole
cis-4b,5,9b,10-tetrahydro-8-methoxy-6-methylindeno[1,2-b]indole
cis-4b,5,9b,10-tetrahydro-8-diethylamino-5-ethylindeno[1,2-b]indole
cis-4b,5,9b,10-tetrahydro-2-diethylaminoindeno[1,2-b]indole
cis-4b,5,9b,10-tetrahydro-8-tert.butyl-4b-methylindeno[1,2-b]indole
cis-4b,5,9b,10-tetrahydro-8-fluoroindeno[1,2-b]indole
cis-5,5a,6,10b-tetrahydroindeno[2,1-b]indole
cis-5,5a,6,10b-tetrahydro-9-methoxyindeno[2,1-b]indole
cis-5,5a,6,10b-tetrahydro-9-isopropylindeno[2,1-b]indole

The compounds having formula IA and IB can exist either as such or as pharmaceutically acceptable salts.

For the compounds with the general formula IA and IB which are asymmetric, both the pure enantiomers, mixtures of enantiomers and the racemic mixtures are within the scope of the present invention.

Pharmaceutical preparations

The compounds of the formula IA or IB will normally be administered orally, rectally, dermally or by injection, in the form of pharmaceutical preparations comprising the active ingredient either as a free base or a pharmaceutically acceptable non-toxic acid addition salt, e.g. the hydrochloride, hydrobromide, lactate, acetate, phosphate, sulfate, sulfamate, citrate, tartrate, or oxalate in a pharmaceutically acceptable dosage form.

The dosage form may be a solid, semisolid or liquid preparation. Usually the active substance will constitute between 0.1 and 99% by weight of the preparation, more specifically between 0.5 and 20% by weight for preparations intended for injection and between 0.2 and 50% by weight for preparations suitable for oral administration. Dermal administration would normally utilize 0,1 - 5% by weight of the active ingredient in a suitable vehicle.

To produce pharmaceutical preparations containing a compound of the formula I in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, and paraffin, and then compressed into tablets. If coated tablets are required, the cores, prepared as described above, may be coated with a concentrated sugar solution which may contain e.g. gum arabic, gelatine, talcum, and titanium dioxide. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compounds.

For the preparation of soft gelatine capsules, the active substance may be admixed with e.g. a vegetable oil or polyethylene glycol. Hard gelatine capsules may contain granules of the active substance using either the above-mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from 0.2% to 20% by weight of the active substance herein described, the balance being sugar and mixture of ethanol, water, glycerol and propylene glycol. Optionally such liquid preparations may contain colouring agents, flavouring agents, saccharine and carboxymethyl-cellulose as a thickening agent or other excipients known to the man in the art.

Solutions for parenteral applications by injection can be prepared in an aqueous solution of a water-soluble pharmaceutically acceptable salt of the active substance, preferably in a concentration of from 0.5% to 10% by weight. These solutions may also contain stabilizing agents and/or buffering agents and may conveniently be provided in various dosage unit ampoules.

Suitable daily doses of the compounds of the invention in therapeutical treatment of humans are about 0.01-100 mg/kg body weight at peroral administration and 0.001-100 mg/kg body weight at parenteral administration.

Method of Preparation

The compounds of the invention may be prepared as outlined below, however, the invention is not limited to these methods, the compounds may be prepared by processes described in known art.

Methods involving preparation of THII and iso-THII compounds from non THII or iso-THII materials.

a. 4b,5,9b,10-tetrahydroindeno[1,2-b]indole (THII, IA) and analogues containing functional groups on the atoms of the benzenoid rings and/or radicals at C-10 such as lower alkyl, lower alkoxy, may be prepared by reduction of corresponding 5,10-dihydroindeno[1,2-b]indole (DHII)

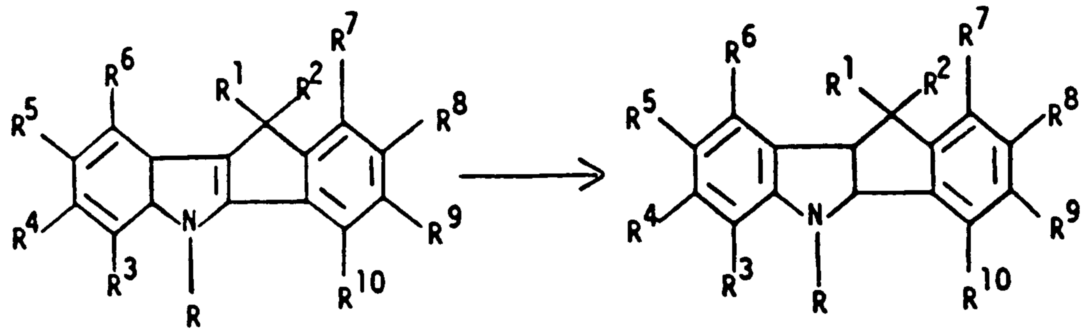

XI IA

R, $R^1$, $R^2$, $R^3$, $R^4$, $R^5$, $R^6$, $R^7$, $R^8$, $R^9$ and $R^{10}$, are as defined under formula IA.
DHII or an analogues are reduced by reaction with zinc and an aqueous mineral acid, such as hydrochloric acid, or more efficiently by reaction with a boron based reductant such as sodium cyanoborohydride in a solvent, often acetic acid or $BH_3$ in tetrahydrofurane. Alternatively morpholino borane in a solvent, often tetrahydrofurane or dioxane, and in the presence of a strong acid e.g. hydrochloric acid, can be used. Alternatively a trialkylsilane can be used. At the end of the reaction the product is isolated by dilution of the reaction mixture with water, neutralisation, and either filtration or solvent extraction. Alternatively reduction is achieved by hydrogenation over a catalyst such as palladium, in this case the DHII compound is dissolved in a suitable solvent, for example ethanol, acetic acid, or ethyl acetate. In such case the product is isolated by removal of the catalyst and evaporation of the solvent under reduced pressure. THII and its analogues may be purified by crystallisation from a suitable solvent, or by column chromatography using silica. DHII and its analogues are synthesized by the Fischer indolisation reaction from phenylhydrazines of formula II and 1-indanones of formula III, wherein $R^{11}$ is hydrogen. 5-Alkyl-4b,5,9b,10-tetrahydroindeno[1,2-b]indoles (N-Alkyl THIIs) are either obtained by the N-alkylation of the corresponding DHII compounds prior to reduction, or from the corresponding 5H-THII compounds by direct N-alkylation. In both cases it is preferable to form the intermediate anions of the tetracyclic amines by treating them with base prior to reaction with an alkyl halide or an alkyl sulphate.

b. 4b,5,9b,10-tetrahydroindeno[1,2-b]indole and analogues bearing a substituent at C-9b can be synthesized by the Fischer indolisation followed by reduction of the intermediate indolenines (IV)

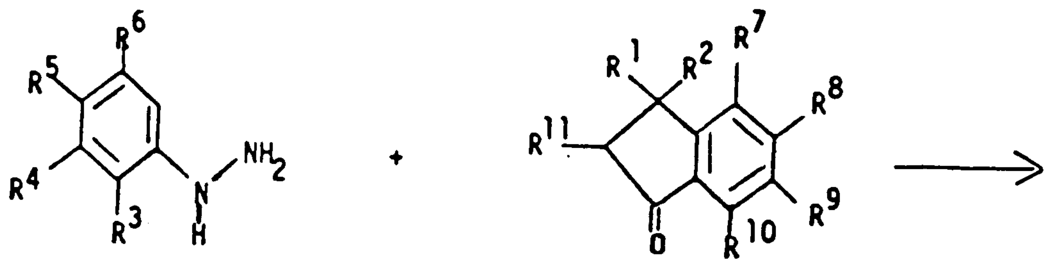

II III

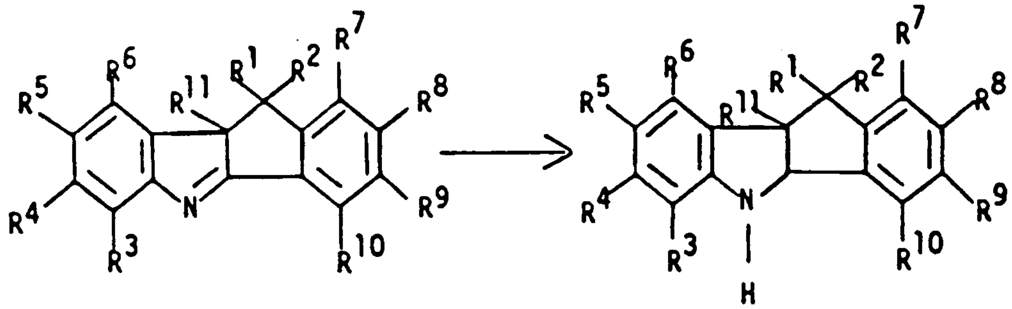

IV V

$R^1$, $R^2$, $R^3$, $R^4$, $R^5$, $R^6$, $R^7$, $R^8$, $R^9$, $R^{10}$, and $R^{11}$ are as defined under formula IA, and if appropriate followed by N-alkylation with R-halide or R-sulphate, where R is as defined in formula IA.

2-Substituted-1-indanones (III) or equivalent starting materials, with appropriate functional group substitution in the benzenoid ring and at C-3, may be reacted with phenylhydrazines (II) either as the free base, or as a salt, often the hydrochloride. Normally the reactants are dissolved in a solvent preferably an alcoholic solvent such as ethanol or propanol. In some cases heat is not required, whereas in others it is necessary to heat the reaction mixture to reflux for up to 1 hour, or more. The phenylhydrazone product can be isolated by dilution of the reaction mixture with water and separated by filtration, or by extraction with a suitable solvent. Further purification is achieved by crystallisation or by chromatography. In the last case column chromatography on silica is satisfactory and a range of eluting solvents may be used.

Cyclisation of the phenylhydrazones to the indolenines (IV) is achieved by redissolving them in a suitable solvent, preferably an alcohol such as ethanol or propanol, and treating the solution with an acid, for example, hydrochloric acid, acetic acid, or trifluroacetic acid. Heat may or may not be required. Other cyclisation reagents including Lewis acids such as zinc chloride, or reagents containing a phosphorus atom, for example phosphorus trichloride, phosphorus oxytrichloride, polyphosphoric acid, or polyphosphonates, can also be used.

Should the salts of phenylhydrazines be used in place of phenylhydrazines in reactions with the indanones then cyclisation of the intermediate phenylhydrazones to the indolenines may occur spontaneously.

In some instances it is observed that the phenylhydrazones obtained from the reactions of phenylhydrazines and 2-substituted-1-indanones on heating in a high boiling solvent such as diethylene glycol, afford the corresponding THII derivatives.

Reduction of the indolenines (IV) to the THII derivatives (V) substituted at C-9b is achieved using standard reducing agents such as sodium borohydride in an appropriate solvent such as ethanol. The products are then isolated and purified in the usual way.

c. 4b,9b-Dialkyl-4b,5,9b,10-tetrahydroindeno[1,2-b]indoles (VI) and analogues may be prepared directly by reacting indolenines (IV) with lithium alkyls ($R^{12}Li$) in an aprotic solvent such as dry tetrahydrofuran.

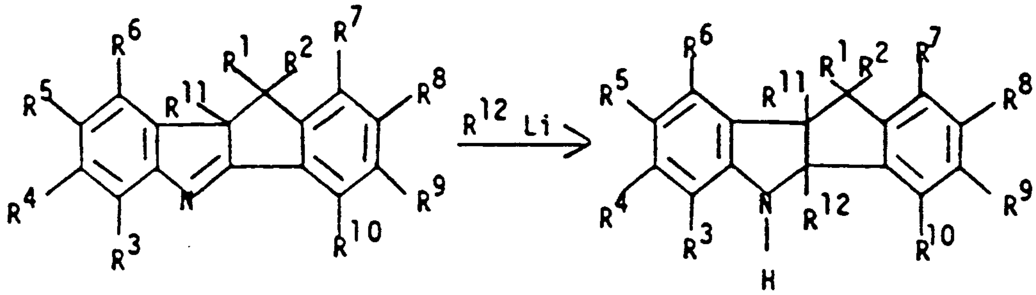

IV VI

wherein $R^1$ to $R^{12}$ are as defined under formula IA, and if appropriate followed by N-alkylation with R-halide or R-sulphate, where R as is defined in formula IA.

d. 5,5a,6,10b-tetrahydroindeno[2,1-b]indole (iso-THII) and analogues may be prepared by reduction of the corresponding 5,6,-dihydroindeno[2,1-b]indole (iso-DHII) by the same methods as outlined in method a. above.

e. 10b-substituted-5,5a,6,10b-tetrahydroindeno[2,1-b]indoles (IX) and analogues can be synthesised from indan-2-ones (XII) bearing a substituent group at C-3, by reacting them with suitable phenylhydrazines (II) under the same conditions as described for the preparation of the indolenines (IV). The intermediate products are the corresponding indolenines (VIII) which when dissolved in a suitable solvent, often ethanol, and reacted with a reducing agent, such as sodium borohydride, yield iso-THII compounds (IX) bearing an alkyl substituent at position 10b. These compounds

can be isolated from the reaction mixtures by dilution and filtration, or by extraction with a suitable solvent.

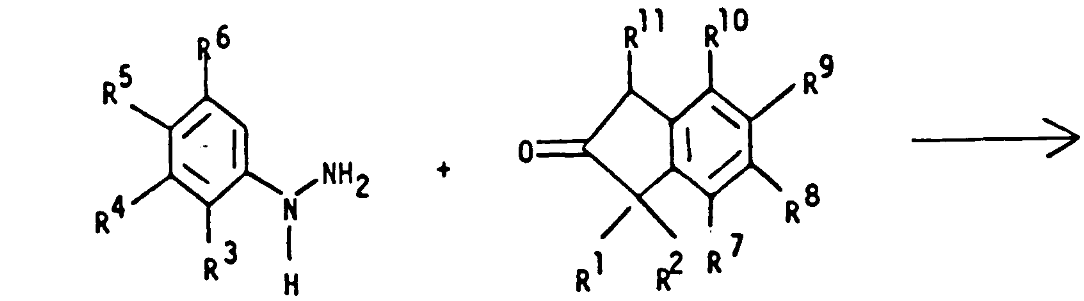

II XII

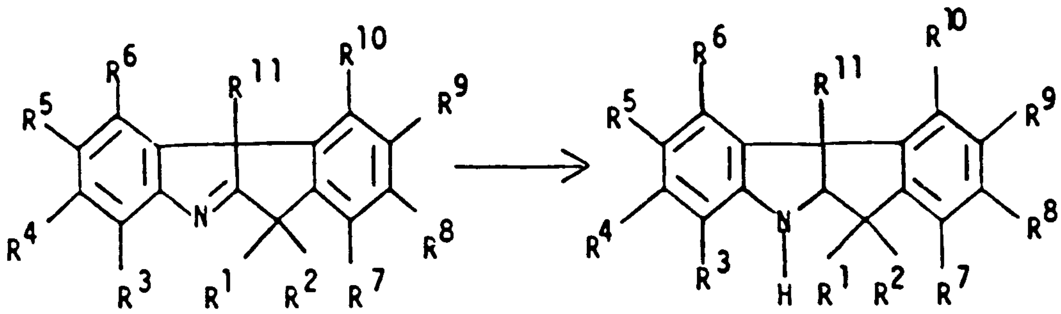

VIII IX

$R^1$, $R^2$, $R^3$, $R^4$, $R^5$, $R^6$, $R^7$, $R^8$, $R^9$, $R^{10}$, $R^{11}$ are as defined under formulae IB.

f. Iso-THII compounds (X) bearing alkyl substituents at C-5a and at C-10b are obtained from the corresponding indolenines (VIII), through reaction with alkyl lithiums ($R^{12}Li$). Using the same procedures, previously described for the 4b,9b dialkylated THII compounds (VI),

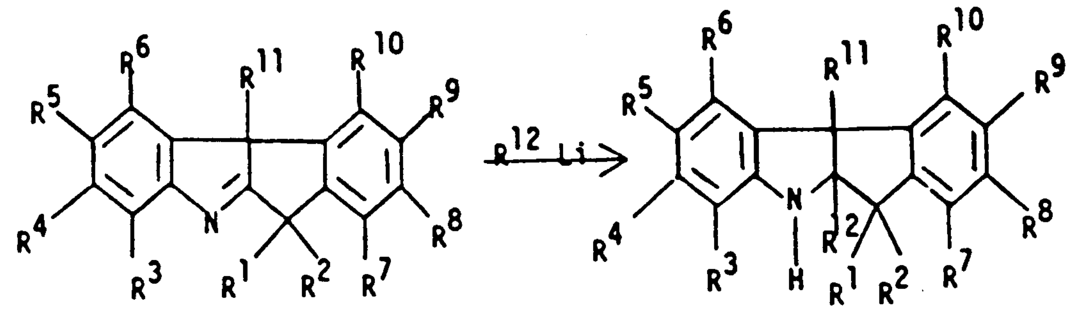


VIII X

$R^1$ to $R^{12}$ is defined as under formula IB.

Methods involving preparation of THII or iso-THII compounds by modification of other THII or iso-THII compounds.

g. 5-Alkyl THII or 6-alkyl iso-THII derivatives are synthesized by N-alkylation of corresponding 5H-THII or 6H iso-THII compounds dissolved in an aprotic solvent e.g. acetone, acetonitrile, dimethylsulfoxide (DMSO), dimethylformamide (DMF) optionally but preferably in the presence of a strong base, e.g. with sodium hydride and the reaction mixture then treated with an alkyl halide or an alkyl sulphate. Alternatively the corresponding 5-alkyl DHII or 6-alkyl iso-DHII compound may be reduced by reaction with zinc and an aqueous mineral acid such as hydrochloric acid, or more efficiently by reduction with a boron based reductant such as sodium cyanoborohydride in a solvent, often acetic acid or $BH_3$ in tetrohydrofuran. Alternatively morpholino borane in a solvent, often tetrahydrofuran or dioxane, and in the presence of a strong acid e.g. hydrochloric acid, can be used. Alternatively a trialkyl silane can be used. At the end of the reaction the product is isolated by dilution with water and either filtration or solvent extraction. Alternatively reduction may be achieved by hydrogenation over a catalyst such as palladium, in this case the 5-alkyl-DHII or the 6-alkyl iso-DHII compound is dissolved in a suitable solvent, for example, ethanol, acetic acid, or ethyl acetate. In such a case the product is isolated by removal of the catalyst and evaporation of the solvent under reduced pressure. 5-Alkyl-THII or 6-alkyl iso-THII compounds may be purified by crystallisation from a suitable solvent, or by column chromatography using silica.

h. 5-Alkyl THII or 6-alkyl iso-THII are synthesized by simple reduction of the corresponding 5-acyl or 6-acyl derivatives using standard methods e.g. by use of lithiumaluminium hydride.

i. THII or iso-THII compounds with alkylamino groups in $R^3$-$R^6$ and/or $R^7$-$R^{10}$ can be prepared from the corresponding 5-acyl THII or 6-acyl iso-THII nitro compounds by standard reduction techniques, e.g. using $TiCl_3$/HCl, followed by one standard N-alkylation, optionally followed by acidic hydrolysis of the 5- or 6-acyl groups for generation of 5- or 6-unsubstituted compounds. The nitro compounds used can either be prepared from the corresponding DHII or iso-DHII-compounds according to methods a. or d. above or via nitration of suitably substituted THII or iso-THII compounds.

j. Hydroxy substituted compounds can be prepared from the corresponding alkoxy substituted ones by standard ether dealkylation methods, e.g. using different Lewis acids.

k. 4b-Alkyl THII and iso-THII i.e. wherein $R^{12}$ is a lower alkyl group and R, $R^1$ to $R^{11}$ are as defined in formula I can be prepared from the corresponding 4b-unsubstituted analogue by a sequence of metallation, e.g. using butyl lithium, carbonation with carbon dioxide, a second directed metallation, e.g. using butyl lithium, and an alkylation with $R^{12}$-halide or $R^{12}$-sulphate followed by a final hydrolysis of the resulting N-carboxylated intermediate.

Processes for preparation of starting materials such as 5,10-dihydroindeno[1,2-b]indole (DHII) and 5,6-dihydroindeno[2,1-b]indole and analogs containing functional groups are described in the co-pending application EP A 404 536

The following illustrates the principle and the adaption of the invention. Temperature is given degrees Celsius.

## Working Examples

### Example 1

cis-4b,5,9b,10-Tetrahydroindeno[1,2-b]indole

To a suspension of 5,10-dihydroindeno[1,2-b]indole 19.16g, 93 mmol in glacial acetic acid (300 $cm^3$) was added portionwise over half an hour, sodium cyanoborohydride (24g, 400 mmol). The mixture was stirred for 3 hours, until all the material had dissolved. The solution was poured into ice water (500 $cm^3$) and stirred for 1 hour to break down the borohydride complex. The clear solution was carefully neutralised with sodium hydroxide causing a white precipitate to form. This was filtered and washed with water until the washings were free from cyanide ion. Drying yielded the title compound as a white solid. Yield: 19 g, (98%). M.p. 107°C. $^1H$ NMR ($CDCl_3$) δ: 3.20 (1H, dd,), 3.51 (1H, dd), 3.99 IH, br,) 4.18 (1H, ddd,), 5.25 (1H, d), 6.60 (1H, d,), 6.74 (1H, dd,), 6.99 (1H, dd,), 7.15-7.22 (4H, m,), 7.32 (1H, d,),

### Example 2

cis-4b,5,9b,10-Tetrahydro-5-methylindeno[1,2-b]indole

A flame dried flask was charged with sodium hydride (60 mg, 2.5 mmol), and tetrahydrofuran (THF) (5 $cm^3$) protected under an atmosphere of nitrogen. To the stirring suspension was added cis-4b,5,9b,10-tetrahydroindeno[1,2-b]indole (500 mg, 2.4 mmol) in THF (5 $cm^3$) dropwise. The reaction was stirred for 1 hour, a pink colour developing. Iodomethane (0.2 $cm^3$) was added, and the solution was stirred overnight. Water (5 $cm^3$) was added, and the THF removed in vacuo. The colourless solid thus obtained was filtered, and dried in a vacuum desiccator. The product was dissolved in 5% ethyl acetate petrol (60-80°C) and filtered through a pad of flash silica. After evaporation of the solvent in vacuo, the title

compound was obtained as a colourless solid. Yield: 450 mg (85%). M.p. 76-77°C. $^1$H NMR ($CDCl_3$) δ: 3.0 (3H, s,), 3.1 (1H, dd,), 3.4 (1H, dd,), 4.1 (1H, ddd,), 4.9 (1H, d,), 6.4 (1H, d,), 6.7 (1H, dd,), 7.1-7.5 (6H, m,).

Example 3

cis-4b,5,9b,10-Tetrahydro-8-methoxyindeno[1,2-b]indole

5,10-Dihydro-8-methoxyindeno[1,2-b]indole (770 mg, 3.3 mmol) was reacted with sodium cyanoborohydride (1.0 g, 16 mmol), in glacial acetic acid (17 $cm^3$) solution. After 30 minutes, the solution was poured into ice/water, stirred for 1 hour, and neutralised with sodium hydroxide. The colourless reaction mixture was extracted into diethylether, the organic layers, dried ($Na_2SO_4$), and concentrated in vacuo. The residue was column chromatographed (10% ethyl acetate/petrol 60-80°C) to yield the title compound as a colourless solid. Yield: 520 mg (66%). M.p. 101°C. H1 NMR ($CDCl_3$) δ: 3.28 (1H, dd,), 3.57 (1H, dd,), 3.80 (3H, s,), 3.85 (1H, br,), 4.24 (1H, dd,), 5.30 (1H, d,), 6.6-7.4 (7H, m,).

Example 4

cis-4b.5.9b,10-Tetrahydro-8-methoxy-5-methylindeno[1,2-b]indole

Using the same procedure as described in Example 2 for cis-4b,5,9b,10-tetrahydro-8-methoxyindeno[1,2-b]indole (239 mg, 1.0 mmol) was methylated with iodomethane, using sodium hydride (25 mg, 1.1 mmol) as the base, in THF (2 $cm^3$). Extraction work-up (into diethylether), and purification by "suction" flash chromatography, yielded a clear gum. Yield: 158 mg, (63%) which solidified after bulb to bulb distillation (180°C at 0.2 mm Hg), giving the title compound M.p. 72°C. $^1$H NMR ($CDCl_3$)δ: 2.87 (3H, s,), 3.03 (1H, dd,), 3.36 (1H, dd,), 3.70 (3H, s,), 4.08 (1H, ddd,), 4.80 (1H, d,), 6.28 (1H, d,), 6.61 (1H, dd,), 6.77 (1H, dd,), 7.1-7.5 (4H, m,).

Example 5

cis-5,5a,6,10b-Tetrahydroindeno[2,1-b]indole

5,6-Dihydroindeno[2,1-b]indole (185 mg, 0.9 mmol) was reacted with sodium cyanoborohydride (310 mg, 5 mmol), in glacial acetic acid (5 $cm^3$), for six hours. The solution was poured into ice/water, and stirred for one hour. It was then neutralised with sodium hydroxide, and the white solid which formed was collected by filtration, washed with water, dried and purified by "flash" chromatography (10% EtOAc/petrol 60-80°C, $R_f$ 30% EtOAc/petrol (60-80°C) 0.6) to yield the title compound as a colourless solid. Yield: (81 mg, (43%) M.p. 85-86°C. $^1$H NMR ($CDCl_3$) δ: 3.09 (1H, dd,), 3.33 (1H, dd,), 3.45 (1H, br,), 4.74 (1H, d,), 4.82 (1H, ddd,), 6.55 (1H, d,), 6.73 (1H, ddd,), 7.00 (1H, ddd,), 7.1-7.4 (4H, m).

Example 6

cis-4b,5,9b,10-Tetrahydro-10,10-dimethylindeno [1,2-b] indole

5,10-Dihydro-10,10-dimethylindeno [1,2-b] indole (1.00 g, 4.29 mmol) was reacted with sodium cyanoborohydride (1.0 g, 16 mmol) in glacial acetic acid (20 $cm^3$), for 10 minutes. The solution was poured into water, stirred for 30 minutes, and extracted into diethylether. The organic phase was washed 10 times with water, dried ($Na_2SO_4$), and the solvent removed in vacuo. The residue was dissolved in 5% ethylacetate/petrol (60-80°C) and filtered through a pad of "flash" silica, yielding, on removal of solvent, a gum which solidified on the oil pump, to give the title compound as a colourless solid. Yield: 0.98 g, (98%) M.p. 57-59°C. $^1$H NMR ($CDCl_3$)δ: 1.17 (3H, s,), 1.43 (3H, s,), 3.86 (1H, d,), 3.9 (1H, br,), 5.29 (1H, d,), 6.59 (1H, d,), 6.71 (1H, ddd,), 7.02 (1H, ddd,), 7.2-7.3 (5H, m,).

Example 7

cis-4b,5,9b,10-Tetrahydro-9b-methylindeno [1,2-b]-indole

The phenylhydrazone of 2-methyl-1-indanone (1.44 g, 6.1 mmol) was heated in diethylene glycol (20 $cm^3$) to near its reflux temperature, until ammonia started to evolve from the air condenser. Heating was continued overnight, or until the ammonia ceased to evolve. The solution was cooled, poured into an equal volume of water, and extracted into diethylether. The ethereal solution was back-extracted with 2M hydrochloric acid, which was made basic with sodium hydroxide, and re-extracted with diethylether. The extracts were evaporated and the residue was purified by column chromatography using silica eluting with 5% EtOAc petrol to yield the title compound as a colourless solid ($R_f$ 30%

EtOAc/petrol 0.8). Yield: (28%). M.p. 72°C. $^1$H NMR ($CDCl_3$) δ: 1.46 (3H, s,), 3.10 (1H, d), 3.30 (1H, dd,), 4.05 (1H, s,), 4.69 (1H, s,), 6.52 (1H, dd,), 6.71 (1H, ddd,), 6.95 (1H, ddd,), 7.0-7.2 (5H, m,).

Example 8

cis-4b,5,9b,10-Tetrahydro-4b,9b-dimethylindenol[1,2-b]indole

Methyllithium (1.5 ml, 2eq of 1.5 m solution in hexanes) was added dropwise at -78°C to a solution of 9b,10-dihydro 9b-methylindeno[1,2-b]indole (260 mg, 1.19 mmol) in THF (10 $cm^3$). After stirring at -78°C for one hour, water (1 $cm^3$) was added to the dark red solution, and the reaction allowed to warm. On approaching room temperature, the colour of the solution was quenched. The reaction was quenched with saturated ammonium chloride solution (10 $cm^3$), the organic phase separated, and dried ($Na_2SO_4$). Evaporation of solvent and "flash" chromatography (10% EtOAc/petrol [60-80°C]) gave a colourless gum ($R_f$[10% EtOAc/petrol (60-80°C)] 0.5) which solidified after all remaining solvent had been removed with an oil pump to give the title compound as a colourless solid. Yield: 87 mg (31%). $^1$H NMR ($CDCl_3$) δ: 1.35 (3H, s,), 1.46 (3H, s,), 3.07 (1H, d,), 3.36 (1H, d,), 4.27 (1H, br,), 6.53 (1H,), 6.71 (1H, ddd,), 6.96 (1H, ddd,), 7.1-7.3 (5H, m,).

Example 9

cis-4b,5,9b,10-Tetrahydro-6,8-dimethylindeno[1,2-b]indole

5,10 Dihydro-6,8-dimethylindeno[1,2-b]indole (323 mg, 1.38 mmol) was reacted with sodium cyanoborohydride (400 mg, 5eq) in glacial acetic acid solution (7 $cm^3$) for 30 minutes. The solution was poured into ice/water, and stirred for a further 30 minutes. The aqueous solution was neutralised with sodium hydroxide, and the suspension was extracted into diethylether. The organic extracts were washed with water, dried ($Na_2SO_4$) and evaporated in vacuo. Purification by "suction flash" chromatography, gave the title compound as a colourless solid. Yield: 2.44 mg, (75%). M.p. 147°C (from EtOAc/petrol [60-80°C]). $^1$H NMR ($CDCl_3$) δ: 2.03 and 2.07 (3H, s,), 3.18, (1H, dd,), 3.48 (1H, dd,), 4.16 (1H, ddd,), 5.24 (1H, d,), 6.66 (1H, s,), 6.84 (1H, s,), 7.1-7.4 (4H, m,).

Example 10

cis-4b,5,9b,10-Tetrahydro-8 methylindeno[1,2-b]indole

5,10-Dihydro-8-methylindeno[1,2-b]indole (10 g, 46 mmol) was stirred at room temperature in glacial acetic acid (150 $cm^3$). Sodium cyanoborohydride (8.6 g, 3 equivalents) was added portionwise over a period of 30 minutes. The reaction was stirred for a further hour, and then poured into ice/water (200 $cm^3$). After stirring for 30 minutes, the acidic solution was made basic by the addition of sodium hydroxide, and the colourless solid thus formed collected by filtration. This solid was washed copiously with water until the residue was free from cyanide ion, and then dried in a vacuum oven to yield the title compound as a colourless solid. Yield: 7.5 g (73%) M.p. 110°C (from ethanol/water). $^1$H NMR ($CDCl_3$) δ: 2.24 (3H, s,), 3.20 (1H, dd,), 3.50 (1H, dd,) 3.9 (1H, br,), 4.16 (1H, dd,), 5.23 (1H, d,), 6.52 (1H,d,), 6.80 (1H, d,), 6.99 (1H, s,), 7.1-7.4 (4H, m,).

Example 11

cis-4b,5,9b,10-Tetrahydro-5,8-dimethylindenol[1,2-b]indole

A solution of 4b,5,9b,10-Tetrahydro-8-methylindeno[1,2-b]indole (1.8 g, 8.1 mmol) in THF (20 $cm^3$) was cooled to -78°C, and n-butyllithium (5.6 $cm^3$ of 1.6 M solution in hexane, 9.0 rmol) added dropwise. The temperature of the solution was allowed to warm to room temperature, and stirred for 30 minutes. The reaction was then cooled to -78°C, and iodomethane (0.6 $cm^3$, 0.9 mmol) added. The reaction was again allowed to warm slowly to room temperature, and then quenched with a saturated solution of ammonium chloride (5 $cm^3$). After stirring the mixture overnight, the organic layer was diluted with dichloromethane, separated, washed with brine, and dried ($MgSO_4$). After removal of solvent in vacuo, the residue was purified by column chromatography to give a gum, which gave the title compound as a colourless solid on trituration with ethanol. Yield: 1.0 g, (53%). M.P. 54°C (from ethanol). $^1$H NMR ($CDCl_3$) δ: 2.25 (3H, s,), 2.94 (3H, s,), 3.07 (1H, dd,), 3.41 (1H, dd,), 4.11 (1H, m), 4.88 (1H, d,), 6.30 (1H, d,), 6.88 (1H, d,), 6.96 (1H, d,), 7.1-7.4 (4H, m,).

Example 12

cis-4b,5,9b,10-Tetrahydro-8-iso-propylindeno[1,2-b]indole

5,10-Dihydro-8-iso-propylindeno [1,2-b]indole (5.27 g, 21.3 mmol) was stirred at room temperature in glacial acetic acid (100 $cm^3$). Sodium cyanoborohydride (5 g, 3 equivalents) was added portionwise over a period of 30 minutes. The reaction was stirred for a further 30 minutes, and then poured into ice/water (150 $cm^3$). After stirring for 30 minutes, the solution was neutralised with aqueous sodium hydroxide, and the colourless solid thus formed collected by filtration. This solid was washed copiously with water until the residue was free from cyanide ion and then dried in a vacuum oven to give the title compound as a colourless solid. Yield: 3.25 g (61 %). M.p. 104°C [from petrol (60-80°C)]. $^1H$ NMR ($CDCL_3$) δ: 1.19 (6H, d,), 2.80 (1H, septet,), 3.20 (1H, dd,), 3.48 (1H, dd,), 4.07 (1H, br,), 4.15 (1H, ddd,), 5.21 (1H, d,), 6.53 (1H, d,), 6.86 (1H, dd,), 7.03 (1H, s,), 7.1-7.4 (4H, m,).

Example 13

cis-4b,5,9b,10-Tetrahydro-5-methyl-8-iso-propylindeno[1,2-b]indole

A solution of cis-4b,5,9b,10-tetrahydro-8-iso-propylindeno[1,2-b]indole (1.75 g, 7.0 mmol) in THF (10 $cm^3$), was added to a suspension of sodium hydride (200 mg, 1.2 equivalents) in THF (7 $cm^3$), at 0°C. The reaction was stirred for two hours, and iodomethane (0.53 $cm^3$, 1.2 equivalents) was added. The reaction was stirred overnight, and then quenched with a saturated solution of ammonium chloride. The organic phase was separated, and the aqueous phase extracted with diethyl ether. The combined organic phases were dried ($Na_2SO_4$), and solvent removed in vacuo to yield the title compound as a colourless gum which was purified by bulb to bulb distillation. Yield: 1.0 g (54%). B.p. 200°C at 0.4 mmHg. $^1H$ NMR ($CDCl_3$) δ: 1.22 (6H, d,), 2.81 (1H, septet,), 2.94 (3H, s,), 3.09 (1H, dd,), 3.43 (1H, dd,), 4.15 (1H, ddd,), 4.90 (1H, d,), 6.32 (1H, d,), 6.93 (1H, dd,), 7.02 (1H, br,), 7.1-7.5 (4H, m,).

Example 14

cis-4b,5,9b,10-Tetrahydro-2-methoxy-1,3-dimethylindeno[1,2-b]indole

5,10-Dihydro-2-methoxy-1,3-dimethylindeno[1,2-b]indole (1 g, 3.80 mmol) was stirred at room temperature in glacial acetic acid (15 $cm^3$). Sodium cyanoborohydride (0.75 g, 3 equivalents) was added portionwise over 15 minutes, and the reaction stirred for a further two hours. The reaction was poured into ice/water (30 $cm^3$), and stirred for a further 30 minutes. The solution was neutralised with aqueous sodium hydroxide, and extracted into diethyl ether. The organic extracts were copiously washed with water until the washings were free of cyanide ion. The solvent was removed in vacuo to give the title compound. This was purified by column chromatography to give a colourless solid. Yield: 0.8 g (79%). M.p. 117°C from EtOAc/petrol (60-80°C). Elementary analysis: Found: C 81.6, H 7.3, N 5.8, $C_{18}H_{19}NO$ Calc. for C 81.5, H 7.2, N 5.9, $^1H$ NMR ($CDCl_3$) δ: 2.15 (3H, s,), 2.25 (3H, s,), 3.07 (1H, dd,), 3.36 (1H, dd,), 3.64 (3H, s,), 4.1 (1H, br,), 4.19 (1H, ddd,), 5.20 (1H, d,), 6.60 (1H, d,), 6.74 (1H, ddd,), 6.97 (1H, s,), 7.00 (1H, ddd,), 7.17 (1H, d,).

Example 15

cis-4b,5,9b,10-Tetrahydro-4b,6,8,9b-tetramethylindeno[1,2-b]indole

A mixture of 5.1 g (0.03 mol) of 2,4-dimethylphenylhydrazine hydrochloride, 5,4 g (0.03 mol) of 2-methyl-1-indanon, 100 ml of ethanol (99,5%) and 2.5 ml of conc. hydrochloric acid was refluxed for 2 hours. The resulting mixture was filtered, the filtrate was evaporated and the residue partioned between ether and water. The organic phase was washed with aqueous sodium carbonate, dried ($MgSO_4$), filtered and evaporated. The residue was subjected to flash chromatography on 60 Å silica. After elution of non-polar impurities with dichloromethane/isooctane (1/1), a mixture of methanol/ethyl acetate/hexane (1/4/5) eluted 5 g of crude 9b, 10-dihydro6,8,9b-trimethylindeno[1,2-b]indole. Without further purification this material was dissolved in 100 ml of dry tetrahydrofuran. In an argon atmosphere, 50 ml of 1.6 M methyl lithium in ether was added at-65 to -55°C. The resulting mixture was kept at -78°C for 1 hour, and was then poured into a cold aqueous armonium chloride solution. The mixture was extracted with ether and the combined organic phases were evaporated to yield 5 g of a green oil. Chromatography on 60 Å silica using 7.5% ethyl acetate in isooctane gave 1 g of the title compound. $^1H$ NMR ($CDCl_3$)δ; 1.37 (3H,s), 1.48 (3H,s), 2.07 (3H,s), 2.20 (3H, s),3.00-3.35 (2H, AB system, J 15 Hz), 3.9 (1H,bs) 6.60 (1H,s) 6.88 (1H,s), 7.08-7.28 (4H,m).

Example 16

cis-5,5a,6,10b-Tetrahydro-methylindeno[2.1-b]indole

A mixture of 0.6 g (0.00289 mol) of 5,5a,6,10b-tetrahydroindeno[2,1-b]indole, 0.9 g (0.00723 mol) of $K_2CO_3$ and 1.03 g (0.00723 mol) of methyl iodide in 10 ml of acetonitrile was stirred for over the night at room temperature. The resulting mixture was filtered and evaporated. The resulting residue was dissolved in ether and then washed twice with water. Drying ($Na_2SO_4$) and evaporation gave 0.25 g (39%) of the title compound. 1H NMR ($CDCl_3$) δ, 2.78 (3H,s), 3.2 (2H,d), 4.3 (1H,m) 4.66 (1H,d), 6.37 (1H,d), 6.68(1H,t), 7.06 (1H, t), 7.13-7.18 (2H,m),7.22-7.26 (1H,m) 7.3-7.37 (2H,m).

Example 17

cis-4b,5,9b,10-Tetrahydro-8-methoxy 6-methylindeno[1,2-b]indole

To a solution of 5.0 g(0.020 mol) of 5,10-dihydro-8-methoxy-6-methylindeno[1,2-b]indole in 50 ml of tetrahydrofuran was added 8.1 g (0.080 mol) of morpholino borane and dropwise 6.3 ml of conc. hydrochloric acid. The initially exothermic reaction mixture was stirred at room temperature for 72 hour. An additional 6.3 ml of conc. hydrochloric acid was then added and the mixture stirred over night. 25 ml of water was then added and the mixture evaporated. The residue was suspended in 200 ml of water and 5 ml of conc. hydrochloric acid and the mixture was heated on a water bath until most of the solid material had dissolved. The solution was then filtered hot and the filtrate cooled and alkalinized by addition of 10 M sodium hydroxide solution. Filtration and washing with water gave 1.93 g (38.4%) of the title compound $^1$H NMR ($CDCl_3$)δ: 2.08 (3H,s), 3.15-3.25 (1H,dd), 3,42-3.55 (1H,dd), 3.7 (3H,s), 4.1-4.22 (1H,t), 5.22-5.28 (1H, d), 6.42-6.46 (1H,d), 6.6-6.65 (1H,d), 7.14-7,25 (3H,m), 7.3-7.4 (1H,m).

Example 18

cis-4b,5,9b,10-Tetrahydro-8-methoxy-7,9-dimethylindeno[1.2-b]indole

To an solution of 0.8 g (0.3 mmol) of 5,10-dihydro-8-methoxy-7,9-dimethylindeno[1,2-b]indole and 1,21 g of morpholino borane in 4 ml of dioxane was added dropwise 1 ml of conc. hydrochloric acid. The mixture was refluxed for 30 minutes, then cooled whereupon 3 ml of 6 M hydrochloric acid was added. The resulting mixture was then refluxed for 15 minutes. After cooling the solution was alkalinized with aqueous sodium hydroxide and extracted three times with ether. Drying ($MgSO_4$) and evaporation gave a crude product, which was crystallized by dissolving in ethyl acetate and addition of light petroleum at -20°C. Filtration gave 0.73 g (92%) of the title compound.

Example 19

cis-4b,5,9b,10-Tetrahydro-6-isopropylindeno[1.2-b]indole

To a solution of 4.95 g (0.020 mol) of 5.10-dihydro-6-iso-propylindeno[1,2-b]indole and 8.08 g (0.080 mo) of morpholino borane in 25 ml of dioxane was added dropwise 7 ml of conc. hydrochloric acid. The mixture was refluxed for 30 minutes, cooled to room temperature whereupon 20 ml of 6 M hydrochloric acid was added. The mixture was refluxed for 15. After cooling the mixture was alkalinzed by addition of aqueous sodium hydroxide and extracted three times with ether. Drying ($MgSO_4$) and evaporation gave a crude product which was purified by column chromatography on silica gel using methylene chloride/light petroleum (20/80) as eluent. Thus 3.53 g (71%) of the title compound was obtained. $^1$H NMR($CDCl_3$) δ: 1.19 (3H,d), 1.29 (3H, d), 2.84 (1h,dq), 3,26 (1H,dd), 3.56 (1H,dd), 4,24 (1H,td), 5.31 (1H,d), 6.79 (1H,dd), 6.97 (1H, d), 7.05-7.09 (1H,m), 7,28-7,21 (3H,m), 7.36-7,40 (1H,m).

Example 20

cis-4b,5,9b,10-Tetrahydro-4b-methylindeno[1,2-b]inole

A flame-dried flask was charged under an inert atmosphere with 4b,5,9b-10-tetrahydroindeno[1,2-b] indole (1.04 g, 5.02 mmol) and freshly distilled tetrahydrofuran (30 $cm^3$). The solution was cooled to -78°C, and a solution of n-butyllithium (3.45 $cm^3$ of 1.6M solution in hexanes, 1.1 eq) added dropwise. The pale yellow solution was allowed to warm to room temperature, and dry carbon dioxide gas bubbled through, until the solution was colourless. The solvent and excess carbon dioxide were carefully removed at reduced pressure of a vacuum pump, and an atmosphere of dry nitrogen re-introduced. The colourless solid was redissolved in dry tetrahydrofuran (30 $cm^3$), cooled to 78°C, and a further 1.1 equivalents of n-butyllithium added. The reaction was stirred at -78°C for 1 1/2 hours, and then quenched with iodometh-

ane (0.35 $cm^3$,1.2eq). After allowing the reaction to warm to room temperature, the solvents were removed as before, and 2M HCl solution (20 $cm^3$) added. After gas evolution had ceased (ca 20 minutes), the solution was neutralised with solid sodium carbonate. The organic material was extracted into ethyl acetate, and the extract was washed with brine, and dried ($Na_2SO_4$). After removal of solvent the product was purified by flash chromatography[$R_F$=0.4(10%-EtOAc/60-80°C petrol)] eluting with 5% ethyl acetate/60-80°C petrol) as a colourless oil which solidified at -20°C to give a pink solid. Yield 0.76 g, 69% M.p 52°C; $^1$H NMR ($CDCl_3$) δ: 7.3-7.1 (5H,m); 6.96(1H,ddd); 6.70(1H,ddd); 6.53(1H,d,J=7.7Hz), 4.2 (1H,br),3.37 (1H,dm,J=8.2Hz), 3.48 (1H,dd,J=16.3, 7.2Hz), 3.14 (1H,dd,J=16.3,2.0Hz), 1.61 (3H,s).

Example 21

cis-4b,5,9b,10-Tetrahydro-4b-methyl-8-isopropylindeno-[1,2-b]indole

Under an intert atmosphere, a flame-dried flash was charged with 4b,5,9b,10-tetrahydro-8-isopropylindeno[1,2-b]indole (1.49 g, 5.98 mmol) and dry tetrahydrofuran (THF, 20 $cm^3$). The solution was cooled to -78°C, and a solution of n-butyllithium in hexanes (4.0 $cm^3$ of 1.6M solution) added dropwise. The reaction was warmed to room temperature, and dry $CO_2$ gas bubbled through until the colour of the anion had dispersed. The solvent and excess $CO_2$ were carefully removed at a vacuum pump, the resulting solid re-dissolved in dry THF (20 $cm^3$) and cooled to -78°C. A further equivalent of n-butyllithium was added, and the reaction stirred at -20°C for 30 minutes. Iodomethane (0.4 $cm^3$, 1 eq) was added at -78°C, and the reaction warmed to room temperature and stirred for 3 hours. The solvents wre removed at the water pump, and 2N HCl solution (20 $cm^3$) added. After 20 minutes, the solution was basified with solid sodium carbonate, and extracted into ethyl acetate. The organic extract was washed with brine, dired ($Na_2SO_4$) and concentrated in vacuo. THe product was purified by flash chromatography as an unstable pale yellow oil. Yield: 1.01 g, 64%. $^1$H NMR ($CDCl_3$) δ: 7.30-7.15 (4H, m), 7.00 (1H, s), 6.85 (1H, d, J = 7.9Hz), 6.49 (1H, d, J = 7.9Hz), 4.0 (1H, br), 3.73 (1H, d, J = 8.1Hz), 3.49 (1H, dd, J = 16.1, 8.2Hz), 3.16 (1H, d, J = 16.1Hz), 2.79 (1H, septet, J = 6.8Hz), 1.61 (3H, s), 1.19 (6H, d, J = 6.8Hz).

Example 22

cis-4b,5,9b,10-Tetrahydro-2-hydroxy-1,3-dimethylindeno[1,2-b]indole.

Under anhydrous conditions, 4b,5,9b,10-tetrahydro-2-methoxy-1,3-dimethylindeno[1,2-b]indole (76 mg, 0.29 mmol) was dissolved in dry dichloromethane (DCM, 1 $cm^3$) into which a small amount of ethanol had been added. The solution was cooled to -78°C, and a solution of boron tribromide in DCM (0.5 $cm^3$ of 1M solution) added. The reaction was slowly warmed to room temperature, reaction occurring at 0°C. After 30 minutes at 10°C, water (1 $cm^3$) was added cautiously, and the reaction stirred for 15 minutes. The mostly solid material was exhaustively extracted between DCM and a saturated solution of sodium bicarbonate. The DCM layer was dried ($Na_2SO_4$), and filtered through a pad of flash silica to yield a colourless solid. Yield: 73 mg, 100% .M.p. 178-180° (dec). $^1$H NMR ($CDCl_3$) δ: 7.16 (1H, d, J = 7.3Hz), 6.99 (1H, ddm), 6.94 (1H, s), 6.73 (1H, ddm, J = 7.3, 1.1Hz), 6.60 (1H, d, J = 7.7Hz), 5.20 (1H, d, J = 8.4Hz), 4.4 (2H, br), 4.15 (1H, ddm), 3.38 (1H, dd, J = 16.1, 8.3Hz), 3.09 (1H, dd, J = 16.2Hz), 2.21 (3H, s), 2.12 (3H, s).

Example 23

cis-5-Acetyl-4b,5,9b,10-tetrahydro-2-methoxy-1,3-dimethylindeno[1,2-b]indole.

4b,5,9b,10-Tetrahydro-2-methoxy-1,3-dimethylindeno[1,2-b]indole (140 mg, 0.53 mmol) was stirred in acetic anhydride (2 $cm^3$) for 5 minutes. Water (5 $cm^3$) was then added, and stirring continued for 30 minutes. The solution was neutralised with solid sodium bicarbonate, and extracted into dichloromethane. The organic material was dried ($MgSO_4$), concentrated and excess dichloromethane azeotroped on a rotary evaporator using 60-80° petrol to yield a colourless solid. Yield: 170 mg, 100% .M.p. 201°C. $^1$H NMR ($CDCl_3$) δ: (mixture of E/Z isomers). 8.10 ($^1/_2$H, d, J = 7.9Hz), 8/4-7.0 ($4^1/_2$H, m), 6.24 and 5.75 (1H, d, J = 8.2 and 7.7Hz), 4.23 and 4.13 (1H, ddd, J = 7.5 and 8.3Hz), 3.64 (3H, s), 3.4 (1H, m), 3.13 (1H, 2 x dd, J = 15.5Hz), 2.59 and 2.50 (3H, s), 2.53 and 2.22 (3H, s), 2.16 and 2.14 (3H, s).

Example 24

cis-5-Acetyl-4b,5,9b,10-tetrahydro-2-hydroxy-1,3-dimethyl-indeno-[1,2-b]indole.

Under anhydrous conditions a solution of 5-acetyl-4b,5,9b, 10-tetrahydro-2-methoxy-3,3-dimethylindeno[1,2-b]indole (109 mg, 0.35 mmol) in dichloromethane (1 $cm^3$) was cooled to -78°C, and a solution of boron tribromide in dichloromethane (0.7 cm3 of 1M solution) added. The reaction was allowed to warm to room temperature, and stirred

for 90 minutes, where-upon water (5 $cm^3$) was cautiously added. After stirring for a further 10 minutes, the mixture was diluted, and extracted with dichloromethane. The combined dichloromethane extracts were dried ($Na_2SO_4$) and filtered through a pad of "flash" silica eluting further with 30% EtOAc/60-80° petrol. Removal of solvent yielded a colourless solid. Yield: 96 mg, 94%. M.p. 205°C (dec). $^1H$ NMR (CDCl $_3$)δ: [mixture of E/Z isomers] 8.06 ($^1/_2$H, d, J = 7.1Hz), 8.05-7.00 (4$^1/_2$H, m), 4.23 and 5.75 (1H, d, J = 8.1 and 7.5Hz), 4.6 (1H, br), 4.22-4.08 (1H,2 x ddd), 3.45-3.26 (1H, m), 3.25-3.05 (1H, 2 x dd), 2.63 and 2.52 (3H, s), 2.20 and 2.17 (3H, s), 2.13 and 2.11 (3H, s).

Example 25

cis 4b,5,9b,10-Tetrahydro-2-methoxy-1,3-dimethyl-8-isopropylindeno[1,2-b]indole.

5,10-Dihydro-2-methoxy-1,3-dimethyl-8-isopropylindeno[1,2-b]indole (1.68 g, 5.5 mmol) was dissolved in trifluoroacetic acid (10 $cm^3$) and stirred vigourously. Triethylsilane (1 $cm^3$, 1.1 eq) was added, and stirring continued for 4 hours. The reaction was poured into water, stirred for 15 minutes, and neutralised with 2M NaOH. All organic material was extracted into ethyl acetate, washed with water and dried ($Na_2SO_4$).After removal of solvent, the solid material was recrystallised from 60-80° petroleum ether as white needles. Yield: 1.18 g, 70% .M.p. 106°C.
$^1H$ NMR ($CDCl_3$) δ: 7.05 (1H, s), 6.97 (1H, s), 6.88 (1H, dd, J = 8.6Hz), 6.55 (1H, d, J = 8.1Hz), 5.19 (1H, d, J = 8.4Hz), 4.18 (1H, ddd), 3.8 (1H, br), 3.64 (3H, s), 3.36 (1H, dd, J = 16.5, 8.3Hz), 3.07 (1H, d(br) J = 16.5Hz), 2.81 (1H, septet, J = 7.0Hz), 2.25 (3H, s), 2.15 (3H, s), 1.20 (6H, d, J = 7.0Hz).

Example 26

cis-4b,5,9b,10-Tetrahydro-2-hydroxy-1,3-dimethyl-8-isopropylindeno[1,2-b]indole.

A solution of 4b,5,9b,10-tetrahydro-2-methoxy-1,3-dimethyl-8-isopropylindeno[1,2-b]inodle (97 mg, 0.32 mmol) in dichloromethane (DCM, 1 $cm^3$) to which some ethanol vapour had been added, was cooled to -78°C. Boron tribromide (0.4 $cm^3$ of 1M solution in DCM) was added, and the reaction warmed to room temperature. Water (1 $cm^3$) was cautiously added, and the mixture partitioned between DCM and sodium bicarbonate solution. The organic layer was dried ($Na_2SO_4$) and filtered through a pad of flash silica, eluting with DCM. Removal of solvent yielded a white solid. Yield: 88 mg, 94% M.p. 165°C (dec.). $^1H$ NMR ($CDCl_3$) δ: 7.04 (1H, s) 6.95 (1H, s), 6.87 (1H, dd), 6.56 (1H, d, J = 8.1Hz), 5.20 (1H, d, J = 8.6Hz), 4.l$_6$ (1H, ddm), 4.6-4.0 (2H, br), 3.36 (1H, dd), 3.06 (1H, dd), 2.81 (1H, septet, J = 7.0Hz), 2.20 (3H, s), 2.12 (3H, s), 1.19 (6H, d, J = 7.9Hz).

Example 27

cis-4b,5,9b,10-Tetrahydro-2,8-dimethoxy-1,3-dimethylindeno[1,2-b]indole.

5,10 Dihydro-2,8-dimethoxy-1,3-dimethylindeno[1,2-b]indole (1.04 g, 3.55 mmol) was dissolved in trifluoroacetic acid (5 $cm^3$) and triethylsilane (0.6 $cm^3$) added with vigourous stirring. Stirring was continued for 4 hours, and the reaction worked up incomplete due to the appearance by t.l.c. of impurities. The solution was poured into water, stirred, and brought to pH 7.0 with solid sodium bicarbonate. The organic material was extracted into DCM, which was washed with sodium bicarbonate solution, and filtered through phase separating filter paper. The product and starting material were separated by column chromatography eluting with 20% EtOAc/60-80° petrol to yield starting indole (0.06 g, 6%) and a colourless solid. Yield: 0.57 g, 58% .M.p. 149-50°C (from EtOAc/petrol). $^1H$ NMR ($CDCl_3$) δ: 6.99 (1H, s), 6.79 (1H, m), 6.57 (2H, m), 5.18 (1H, d, J = 8.5Hz), 4.18 (1H, dd(br)), 3.74 (3H, s), 3.65 (3H, s), 3.35 (1H, dd, $^3J$ = 8.4Hz), 3.06 (1H, d(d)), 2.25 (3H, s), 2. 15 (3H, s).

Example 28

cis-5-Acetyl-4b,5,9b,10-Tetrahydro-4b,9b-dimethylindeno[1,2-b]indole.

4b,5,9b,10-tetrahydro-4b,9b-dimethylindeno[1,2-b]indole, (0.2 g, 0.85 mmol) was dissolved in acetic anhydride (1 $cm^3$) and the solution stirred at room temperature for 16 hours. The reaction mixture was poured into saturated sodium bicarbonate solution and stirred for a further $^1/_2$ hour. The mixture was then extracted with ether (3 x 15 $cm^3$) and the combined organic extracts collected washed with brine, dried ($Na_2SO_4$) and evaporated to yield the title compound as an oil. This was purified by flash chromatorgraphy on silica gel eluting with 5-7% ethyl acetate/60-80° petrol, to yield a colourless solid. Yield: 0.15 g, 64% .M.p. 81°C. $^1H$ NMR ($CDCl_3$) δ: 7.82 (1H, s), 6.99-7.29 (7H, m), 3.26 (1H, d, J = 15.9Hz), 2.97 (1H, d, J = 15.9Hz), 2.42 (3H, s), 1.77 (3H, s), 1.25 (3H, s).

Example 29

cis 4b,5,9b,10-Tetrahydro-4b,8,9b-trimethylindeno[1,2-b]indole

To a solution of 9b,10-dihydro-8,9b-dimethylindeno[1,2-b]indole, (5.05 g, 0.022 mol) in dry tetrahydrofuran (100 $cm^3$) at -78°C, under nitrogen, in a flame dried flask, was added dropwise methyllithium, (1.4M solution in diethyl ether, 23.2 $cm^3$, 0.032 mol). The mixture was stirred at -78°C for 2 hours and then at -15°C for a further 1 hour. Saturated ammonium chloride solution (3 $cm^3$) was then added and the mixture allowed to warm to room temperature. The reaction mixture was portioned between ether and saturated ammonium chloride solution and the layers separated. The aqueous phase was extracted with diethyl ether (2 x 25 $cm^3$) and the combined organic extracts were washed with brine, dried ($Na_2SO_4$) and evaporated to afford the title compound as an off white solid. This was purified by flash chromatography on silica gel eluting with 3-10% ethyl acetate/60-80° petrol to yield a colourless solid. Yield: 2.73 g, 51% .M.p. 208-212°C. $^1H$ NMR ($CDCl_3$) δ: 7.1-7.3 (4H, m), 6.94 (1H, m), 6.77 (1H, d, J = 7.8Hz), 6.46 (1H, d, J = 7.8Hz), 3.35 (1H, d, J = 15.9Hz), 3.06 (1H, d, J = 15.9Hz), 2.22 (3H, s), 1.46 (3H, s), 1.34 (3H, s).

Example 30

cis-4b,5,9b,10-Tetrahydro-4b,9b-dimethyl-8-isopropylindeno[1,2-b]indole.

To a solution of methyllithium (1.4M in ether, 14.5 $cm^3$, 12.4 mmol) in dry tetrahydrofuran (20 $cm^3$), at -78°C, under nitrogen, in a flame dried flask, was added dropwise, over 1 hour, a solution of 9b,10-dihydro-9b-methyl-8-isopropylindeno-[1,2-b]indole (1.62 g, 6.20 mmol) in dry tetrahydrofuran (30 $cm^3$). After addition, the mixture was stirred for a further $^1/_2$ hour at -78°C. Saturated ammonium chloride solution (2 $cm^3$) was then added and the mixture allowed to warm to room temperature. The reaction mixture was then portioned between diethyl ether (50 $cm^3$) and saturated ammonium chloride solution (25 $cm^3$), and the layers separated. The aqueous phase was extracted with diethyl ether (2 x 10 $cm^3$) and the combined organic extracts were washed brine, dried ($Na_2SO_4$) and evaporated. The crude material thus obtained was purified by flash chromatography on silica gel eluting with 3-10% ethyl acetate/60-80° petrol to yield a pale yellow gum. Yield: 0.77 g, 45%. $^1H$ NMR ($CDCl_3$) δ: 7.1-7.3 (4H, m), 6.98 (1H, d, J = 1.8Hz), 6.82 (1H, dd, J = 1.8Hz and 7.9Hz), 6.47 (1H, d, J = 7.9Hz), 3.36 (1H, d, J = 15.9Hz), 3.06 (1H, d, J = 15.9Hz), 2.78 (1H, sept., J = 6.8Hz), 1.45 (3H, s), 1,34 (3H, s), 1.18 (6H, dd, J = 1.3Hz and 6.9Hz).

Example 31

cis-5-Acetyl-4b,5,9b,10-tetrahydro-4b,8,9b-trimethylindeno[1,2-b]indole.

4b,5,9b,10-Tetrahydro-4b,8,9b-trimethylindeno[1,2-b]indole (0.25 g, 1.00 mmol) was dissolved in acetic anhydride (1 $cm^3$) and stirred at room temperature for 16 hours. The reaction mixture was poured into saturated sodium bicarbonate solution (25 $cm^3$) and stirred for $^1/_2$ hour. The mixture was extracted with diethyl ether (3 x 10 $cm^3$) and the combined organic extracts were washed with brine, dried ($Na_2SO_4$) and evaporated. The crude material was purified by flash chromatography on silica gel eluting with 4% ethyl acetate/60-80° petrol to yield a pale yellow gum. Yield: 0.15 g, 53%. $^1H$ NMR ($CDCl_3$) δ: 7.84 (1H, s), 6.90-7.23 (6H, m), 3.26 (1H, d, J = 15.9Hz), 2.96 (1H, d, J = 15.9Hz), 2.40 (3H, s), 2.31 (3H, s), 1.77 (3H, s), 1.32 (3H, s).

Example 32

cis-5-Acetyl-4b,5,9b,10-tetrahydro-4b,9b-dimethyl-8-isopropylindeno[1,2-b]indole.

4b,5,9b,10-Tetrahydro-4b,9b-dimethyl-8-isopropylindeno[1,2-b]indole (0.24 g, 0.87 mmol) was dissolved in acetic anhydride (1 $cm^3$) and stirred at room temperature for 16 hours. The reaction mixture was poured into saturated sodium bicarbonate solution (25 $cm^3$) and stirred for $^1/_2$ hour. The mixture was extracted with ether (3 x 25 $cm^3$). Combined organic extracts were washed with brine, dried ($Na_2SO_4$) and evaporated. The crude material was purified by flash chromatography on silica gel eluting with 4% ethyl acetate/60-80° petrol to yield a pale yellow gum. Yield: 0.25 g, 89%. $^1H$ NMR ($CDCl_3$) δ: 7.84 ((1H, s), 6.90-7.24 (6h, m), 3.27 (1H, d, J = 15.9Hz), 2.97 (1H, d, J = 15.9Hz), 2.88 (1H, sept., J = 6.8Hz), 2.40 (3H, s), 1.77 (3H, s), 1.33 (3H, s), 1.23 (6H, dd, J = 0.73Hz and 6.8Hz).

Example 33

cis-4b,5,9b,10-tetrahydro-5-ethyl-indeno[1,2-b]indole

5,10-Dihydroindeno[1,2-b]indole (60.0 g, 0.29 M) was vigourously stirred in glacial acetic acid (1000 $cm^3$) and to it was added sodium cyanoborohydride (79 g, 1.25 M) portion-wise over 40 minutes. After 3 hours stirring the reaction mixture was poured onto ice-water (2000 $cm^3$) and the gelatinous solid which formed was separated and stirred with a mixture of ethyl acetate (75 $cm^3$) and water (100 $cm^3$). A colourless solid remained this was found to be unreacted starting material (19.0 g). The original filtrate was extracted with ethyl acetate (2 x 75 $cm^3$) and the combined organic phases dried and evaporated. The residue was then partly dissolved in a mixture of 60-80°C petroleum ether (20 $cm^3$) and ethyl acetate (40 $cm^3$). The residual solid was removed and shown to be impure starting material (2.5 g). The filtrate was extracted with 2M hydrochloric acid (8 x 25 $cm^3$) and the combined acid extracts were washed with ethyl acetate (2 x 15 $cm^3$), prior to basification with 0.89 ammonia. The oil which separated was extracted into ethyl acetate (6 x 25 $cm^3$) and the combined organic layers were then dried and evaporated to give the title compound as a colourless oil. Yield: 34 g, 75%. $^1H$ NMR ($CDCl_3$) δ: 1.20 (3H, t, J = 7.0Hz), 3.05 (1H, dd, J = 16.5 and 5.0Hz), 3.38 (2H, g, J = 7.0Hz), 3.40 (1, dd, J = 16.5 and 9.0Hz), 4.12 (1H, ddd, J = 9.0, 5.0 and 5.0Hz), 5.11 (1H, d, J = 9.0Hz), 6.33 (1H, d, J = 7.0Hz), 6.57 (1H, dt, J = 7.5 and 1.0Hz), 7.02 (1H, t, J = 8.0HHz), 7.08 (1H, d, J = 8.0Hz), 7.14-7.20 (3H, m), 7.34-7.40 (1H, m).

Example 34

cis-4b,5,9b,10-Tetrahydro-2-(N,N-Diethylamino)-indeno[1,2-b]indole.

5,10-Dihydro-2-(N,N-diethylamino)-indeno[1,2-b]indole (180 mg, 0.65 mM) was dissolved in trifluoroacetic acid (3 $cm^3$) containing triethylsilane (0.5 $cm^3$) and the solution was stirred at room temperature for 3 hours. It was then treated with water (15 $cm^3$) and 0.89 ammonia (5 $cm^3$), and stirred for a further 0.5 hour. The mixture was extracted with dichloromethane (3 x 5 $cm^3$), and the combined extracts were filtered through phase separation paper and evaporated to give a pale yellow oil (280 mg). The oil was subjected to column chromatography (silica gel; 7.5% EtAc in 60-80° petrol ether) giving the title compound as colourless needles. Yield: 65 mg, 35% M.p. 116 118°C. This product was recrystallized from diethyl ether - 60-80°C petroleum ether as long colourless needles. M.p. 118°C; $^1H$ NMR ($CDCl_3$) δ: 1.08 (6H, t, J = 7.0Hz), 3.10 (1H, dd, J = 16.0 and 2.0Hz), 3.25 (4H, q, J = 7.0Hz), 3.42 (1H, dd, J = 16.0 and 8.5Hz), 3.55 (1H, br.s), 4.10 (1H, ddd, J = 8.5, 8.5 and 2.0Hz), 5.13 (1H, d, J = 8.5Hz), 6.46 (1H, d, J = 2.0Hz), 6.50-6.60 (2H, m), 6.70 (1H, ddd, J = 7.0, 7.0 and 1.0Hz), 6.95 (1H, ddd, J = 7.0, 7.0 and 1.0Hz), 7.06-7.13 (2H, m).

Example 35

cis-(E)- and (Z)-4b,5,9b,10-Tetrahydro-5-acetyl-8-(N,N-diethylamino)-indeno[1,2-b]indole.

(E)- and (Z)-4b,5,9b,10-Tetrahydro-5-acetyl-8-amino-indeno-[1,2-b]indole (1.0 g) sodium carbonate (1 g) and ethyl iodide (2.0 $cm^3$) were heated together at reflux in a mixture of tetrahydrofuran (80 $cm^3$) and water (15 $cm^3$), with stirring, for 24 hours. More ethyl iodide (0.5 $cm^3$) was then added and the heating continued for a further 3 hours. The solvents evaporated and dichloromethane added to the residue. Solids were removed by filtration and these were then washed thoroughly with diethy ether. Filtrate and washings were combined and reduced in volume to about 15 $cm^3$. On cooling, the title compounds separated out as pale yellow prisms. Yield: 0.75 g, 62% M.p. 176-178°C; $^1H$ NMR ($CDCl_3$) δ: 1.10 (6H, t, J = 7.0Hz), 1.13 (6H, t, J = 7.0Hz), 2.43 (3H, s), 2.54 (3H, s), 3.21 (1H, d, J = 16Hz), 3.29 (10H, m), 4.06 (1H, dd, J=J=8Hz), 4.16 (1H, dd, J=J=7.5Hz), 5.56 (1H, d, J = 2Hz), 5.72 (1H, d, J = 7.5Hz), 6.27 (1H, d, J = 8Hz), 6.47 (2H, ddd, J = 7.5, J = 2Hz), 6.63 (1H, d, J = 2Hz), 6.90 (1H, d, J = 9Hz), 7.15-7.23 (6H, m), 7.41 (1H, m), 7.60 (1H, m), 7.89 (1H, d, J = 9Hz).

Example 36

cis-4b,5,9b,10-Tetrahydro-5-ethyl-8-(N.N-diethylamino)-indeno[1,2-b]indole.

cis-(E)- and (Z)-4b,5,9b,10-Tetrahydro-5-acetyl-8-(N,N-diethylamino)-indeno[1,2-b]indoles (0.32 g, 1 mM) in dry tetrahydrofuran (60 $cm^3$) were treated with lithium aluminium hydride (0.38 g, 10 mM) in portions over a period of 30 minutes. The reaction mixture was then heated at reflux for 3 hours and then excess reagent was destroyed by the addition of 30% sodium ammonium tartrate. The organic solvent was then decanted off and the residue extracted with tetrahydrofuran (3 x 10$cm^3$). Solvent and extracts were combined, dried and evaporated to yield an oil which was absorbed onto silica (1 g) and added to the top off a column of silica (5 g), prior to elution with 10 ethyl acetate in 60-80°C petroleum ether. The colour of the column became dark blue but the title compound was eluted off as a colour-less oil. Yield: 0.2 g, 65%. The compound is unstable in air becoming blue and then dark red. $^1$H NMR ($CDCl_3$) δ: 1.09 (6H, t, J = 7.0Hz), 1.27 (3H, t, J = 7.0Hz), 3.1-3.3 (5H, m), 3.3-3.5 (3H, m), 4.18 (1H, br.s), 5.07 (1H, br.s), 6.40 (1H, d, J = 7.0Hz), 6.57 (1H, d, J = 7.0Hz), 6.74 (1H, s), 7.22 (3H, s), 7.43 (1H, m).

Example 37

cis-4b,5,9b,10-Tetrahydro-8-tert-butylindeno[1,2-b]indole.

A solution of 5,10-dihydro-8-tert-butylindeno[1,2-b]indole (0.57 g, 2.2 mM) in trifluoroacetic acid (5 $cm^3$) was stirred rapidly, and triethylsilane (0.7 $cm^3$, 2 eq.) added in one portion. The reaction was stirred overnight, poured into water (10 $cm^3$) and neutralised by the addition of sodium hydroxide. The product was extracted into diethyl ether (2 x 5 $cm^3$), and the combined extracts were washed with water, dried ($Na_2SO_4$) and evaporated to yield a pink solid. This was washed with cold petroleum ether (60-80°C), and then crystallised from petrol to yield a colourless solid. Yield: 0.47 g, 81% M.p. 103-105°C; $^1$H NMR ($CDCl_3$) δ: 7.4-6.9 (6H, m), 6.58 (1H, d, J = 8Hz), 5.25 (1H, d, J = 8.5Hz), 4.15 (2H, br.m), 3.5 (1H, dd, J = 16.0 and 9Hz), 3.2 (1H, d, J = 16Hz), 1.2 (9H, s).

Example 38

cis-4b,5,9b,10 Tetrahydro-5-methyl-8-tert-butylindeno[1,2--b]indole.

A flame dried flask was charged with 4b,5,9b,10-tetrahydro-8-tert-butyl-indeno[1,2-b]indole (309 mg, 1.17 mM), and tetrahydrofuran (2.5 $cm^3$). The solution was cooled to -78°C, and a solution n-butyllithium (0.75 $cm^3$ of 1.6 M solution in hexanes, 1.1 eq.) added dropwise. The reaction mixture was stirred at -78°C for one hour, and iodomethane (0.1 $cm^3$, 1.3 eq.) was then added. After allowing the reaction to warm slowly to room temperature, a saturated solution of ammonium chloride was introduced, and the organic material extracted into diethyl ether. The organic phase was washed with brine, and dried ($MgSO_4$). Evaporation of the solvent yielded a light brown oil, which solidified on cooling as a beige solid. Yield: 311 mg, 96%. M.p. 74°C; $^1$H NMR ($CDCl_3$) δ: 7.5-7.0 (6H, m), 6.32 (1H, d, J = 8.3Hz), 4.91 (1H, d, J = 8.8Hz), 4.16 (1H, ddd, J = 9.0, 8.8 and 5.3Hz), 3.44 (1H, dd, J = 16.3 and 9.1Hz), 3.10 (1H, dd, J = 16.3 and 5.3Hz), 2.95 (3H, s), 1.29 (9H, s).

Example 39

cis-4b,5,9b,10-Tetrahydro-4b-methyl-8-tert-butylindeno[1,2-b]indole.

A flame-dried flask was purged with nitrogen and charged with 4b,5,9b,10-tetrahydro-8-tert-butylindeno[1,2-b]indole (240 mg, 0.91 mM) and freshly distilled tetrahydrofuran (3 $cm^3$). The solution so formed was cooled to -78°C, and n-butyllithium (0.60 $cm^3$ of 1.6 M solution in hexanes, 1.1 eq.) added dropwise. The pale yellow solution was allowed to warm to room temperature, and dry carbon dioxide gas was bubbled through the solution until it became virtually colourless. The solvent was carefully removed at reduced pressure and an atmosphere of dry nitrogen introduced. The colourless residue was redissolved in dry tetrahydrofuran (3 $cm^3$), and the solution cooled to -78°C, and 1.1 equivalents of n-butyllithium added. The reaction mixture was stirred at -78°C for 2 hours, and then treated with iodomethane (0.06 $cm^3$), 1.1 eq.). After allowing the reaction to warm to room temperature,the solvents were removed and 2M HCl solution (20 $cm^3$) added. When the gas evolution had ceased (ca 20 minutes), the solution was neutralised with solid sodium carbonate. The organic material was extracted in dichloromethane (3 x 5 $cm^3$), and the combined extracts were washed with brine, and dried ($Na_2SO_4$). After removal of solvent, the solid product remaining was purified by flash chromatography [$R_F$ = 0.4 (10% EtOAc/60-80°C petrol)] eluting with 10% ethyl acetate/60-80°C petrol. This afforded a pale yellow oil which solidified at -20°C as a waxy solid. Yield: 0.86 mg, 34%. M.p. 82-84°C. 1H NMR ($CDCl_3$) δ: 7.4-7.0 (6H, m), 6.49 (1H, d, J = 9.0Hz), 4.15 (1H, br.m), 3.71 (1H, br.m), 3.51 (1H, dd, J = 16.0 and 9.0Hz), 3.17 (1H, dd, J = 16.0 and 5Hz), 1.61 (3H, s), 1.27 (9H, s).

Example 40

cis-4b,5,9b,10-Tetrahydro-8-fluoroindeno[1,2-b]indole.

A solution of 5-10-dihydro-8-fluoro-indeno[1,2-b]indole (0.8 g, 3.6 mM) in trifluoroacetic acid (5 $cm^3$) was stirred rapidly, and triethylsilane (0.86 $cm^3$, 1.5 eq.) added in one portion. The reaction was stirred for 4 hours and the excess trifluoroacetic acid removed in vacuo. Water (10 $cm^3$) was added to the solid, and the suspension neutralised by the addition of sodium hydroxide. The product was extracted into diethyl ether, which was washed with water, dried and

evaporated to yield an off white solid. This was crystallised from ethyl acetate/petroleum ether (60-80°C) to yield a colourless solid. Yield: 0.53 g, 81% M.p. 92-94°C. $^1H$ NMR ($CDCl_3$) δ: 7.34 (1H, m), 7.8-7.2 (3H, m), 6.87 (1H, m), 6.69 (1H, m), 6.52 (1H, dd, J = 8.4 and 4.4Hz), 5.27 (1H, d, J = 8.8Hz), 4.16 (1H, ddm, J = 8.8 and 8.3Hz), 4.1 (1H, br.s), 3.51 (1H, dd, J = 16.5 and 8.3Hz), 3.18 (1H, dd, J = 16.5 and 2.0Hz).

Example 41

cis-4b,5,9b,10-Tetrahydro-3,7-dinitroindeno[1,2-b]indole.

A solution of cis-4b,5,9b,10-tetrahydroindeno[1,2-b]indole (1.0 g, 4.8 mM) in concentrated sulphuric acid (20 $cm^3$) under vigorous stirring during 45 minutes then cooled to 0°C and treated with potassium nitrate (0.7 g, 6.9 mM) in small portions over a period of 15 minutes. The cherry red solution was stirred at 0°C for a further 15 minutes and then poured onto ice. The yellow solid which had formed was collected by filtration and washed firstly with water, and then with hot 25% ethanol-water solution (30 $cm^3$). The filtrate from the last washing was allowed to cool whereupon the title compound separated out as deep yellow platelets. Yield: 0.45 g, 31.5% M.p. 174-176°C. $^1H$ NMR ($CDCl_3$) δ: 3.30 (1H, dd, J = 17.5 and 1,0Hz), 3.65 (1H, dd, J = 17.5 and 8.5Hz), 4.20 (1H, br.s), 4.33 (1H, t, J = 8.5Hz), 5.41 (1H, d, J = 8.5Hz), 7.22 (1H, dd, J = 8.0 and 1.0Hz), 7.28 (1H, d, J = 2.0Hz), 7.38 (1H, d, J = 8.5Hz), 7.50 (1H, dd, J = 8.0 and 2.0Hz), 8.17 (1H, dd, J = 8.5 and 2.0Hz), 8.40 (1H, d, J = 2.0Hz).

Example 42

cis-4b,5,9b,10-Tetrahydro-5-acetyl-3,7-dinitroindeno[1,2-b]indole.

cis-4b,5,9b,10-Tetrahydro-3,7-dinitroindeno[1,2-b]indole (0.40 g, 1.35 mM) and acetic anhydride (1.5 $cm^3$) were heated at 90°C for 1 hour, cooled and poured into water (7 $cm^3$) ice and the mixture was stirred for 30 minutes. The colourless solid which deposited was collected and taken up into hot ethanol. The after hot filtration, the title compound separated from the cold filtrate as prisms. Yield: 0.42 g, 92% M.p. 264-266°C.

Example 43

cis-(E) and (Z)-4b,5,9b,10-Tetrahydro-5-acetyl-3,7-diaminoindeno[1,2-b]indole.

cis-4b,5,9b,10-Tetrahydro-5-acetyl-3,7-dinitroindeno[1,2-b]indole (0.4 g, 1.2 mM) was stirred in a solution of glacial acetic acid (30 $cm^3$) and water (5 $cm^3$). To this mixture was added titanium trichloride (3 $cm^3$ of a 30% solution in aqueous 24% hydrochloric acid) over a period of 5 minutes. After a further 2 hours, the reaction mixture became colourless and a further portion (0.5 $cm^3$ of the titanium trichloride reagent was introduced. Unreacted starting material was filtered off (0.13 g) and the filtrate was poured onto crushed ice. The pH of the solution thus formed was adjusted to 6 (15 $cm^3$, 0.89 ammonia), and the product extracted into ethyl acetate (8 x 50 $cm^3$). This extraction was extremely slow because of the formation of emulsions, and was accomplished during 3 days. The extracts were combined and evaporated to give a greenish solid which was triturated with diethyl ether to afford colourless micro prisms. Yield: 0.13 g, 58% M.p. 254-256°C.

Example 44

cis-4b,5,9b,10-Tetrahydro-5-acetyl-3,7-di(-N,N-diethylamino)indeno-[1,2-b]indole

cis-4b,5,9b,10-Tetrahydro-5-acetyl-3,7-diamino-indeno[1,2-b]indole (0.13 g) was dissolved in tetrahydrofuran (18 $cm^3$) containing water (3.5 $cm^3$), sodium carbonate (0.3 g), and ethyl iodide (0.8 $cm^3$) and heated under reflux for 24 hours. A further quantity of ethyl iodide (0.8 $cm^3$) was then added, and the heating continued for 4 hours. The solvents and excess reagent were removed and the residue extracted with diethyl ether (6 x 10 $cm^3$). The combined extracts were evaporated to yield a brown gum (0.33 g), this was purified by column chromatography on silica (4 g), eluting with 20% ethyl acetate in 60-80°C petroleum ether. This gave the title compound as colourless prisms. Yield: 0.023 g. 12.5% M.p. 137-138°C. $^1H$ NMR ($CDCl_3$) δ: 4 x [1.12 (6H, t, J = 7.0Hz)], 2.50 and 2.57 2 x [3H, s)], 3.05 (1H, d, J = 16.5Hz), 3.10 (1H, d, J = 16.0Hz), 3.26 and 3.30 4 x [(4H, q, J = 7.0Hz)], 3.98 (1H, dd, J = 7.5 and 7.5Hz), 4.07 (1H, dd, J = 7.5 and 7.0Hz), 5.70 (1, d, J = 7.5Hz), 6.23 (1H, d, J = 7.0Hz), 6.32-6.40 (2H, m), 6.59 (2H, ddd, J = 8.0, 8.0, 1.5Hz), 6.96 (2H, d, J = 1.0Hz), 6.95-7.10 (5H, m), 7.61 (1H, d, J = 1.5Hz).

Example 45

cis-5,5a,6,10b-Tetrahydro-9-methoxyindeno[2,1-b]indole

5,6-Dihydro-9-methoxyindeno[2,1-b]indole (0.56 g), as a suspension in glacial acetic acid (25 $cm^3$) at 16°C, was treated with sodium cyanoborohydride (1.0 g) in small portions over 6 hours. The resulting solution was stirred for a further 1 hour, and then poured into ice-water (100 $cm^3$). The solution was separated from a small amount of resinous material and the filtrate treated with sodium carbonate (2.5 g) in small portions with vigorous stirring. The colourless solid which separated was collected and crystallised form ethanol as needles. Yield: 0.31 g, 55% M.p. 129-130°C. $^1H$ NMR ($CDCl_3$) δ: 3.06 (1H, dd, J = 16.5 and 1.5Hz), 3.2-3.8 (1H, br.s), 3.31 (1H, dd, J = 16.5 and 6.0Hz), 3.76 (3H, s), 4.71 (1H, d, J = 8.0Hz), 4.80 (1H, ddd, J = 8.0, 6.0 and 2.0Hz), 6.5 (1H, d, J = 8.5Hz), 6.58 (1H, dd, J = 8.5 and 2.5Hz), 6.99 (1H, d, J = 2.5Hz), 7.15-7.24 (3H, m), 7.33-7.36 (1H, m).

Example 46

cis-5,5a,6,10b-Tetrahydro-9-isopropylindeno[2,1-b]indole and cis-5,5a,6,10b-Tetrahydro-6-ethyl-9-isopropylindeno[2,1-b]-indole.

To a suspension of 5,6-dihydro-9-isopropylindeno[2,1-b]indole (2.3 g, 9.3 mmol) in glacial acetic acid (30 $cm^3$) was added sodium cyanoborohydride (2 g) in small portions over 30 minutes. The mixture was stirred for 3 hours, and the solution then obtained was poured into ice/water (50 $cm^3$) and stirred for 1 hour. The clear solution was carefully neutralised with sodium hydroxide causing a white precipitate to form. This was extracted into diethyl ether (3 x 10 $cm^3$), and the combined extracts were washed copiously with water, dried ($Na_2SO_4$) and evaporated. Tlc analysis of the residue indicated two products had formed these were isolated by column chromatography eluting with 10% ethyl acetate/petroleum ether (60-80°C) to yield first a small amount of cis-5,5a,6,10b-tetrahydro-6-ethyl-9-isopropylindeno[2,1-b]indole (0.07, 3%), and then the title product (0.93 g, 40%), both as colourless oils). Further purification of the last products was achieved by distillation. $^1H$ NMR ($CDCl_3$) δ: 7.4-7.1 (5H, m), 6.87 (1H, dd, J = 8.1, 1.8Hz), 6.50 (1H, d, J = 8.1Hz), 4.81 (1H, ddd, J = 8.1, 6.2 and 2.0Hz), 4.73 (1H, d, J = 8.1Hz), 3.32 (1H, dd, J = 16.6 and 6.2), 3.08 (1H, dd, J = 16.6 and 2.0Hz), 2.83 (1H, septet, J = 6.9Hz), 1.23 (6H, d, 6.9Hz).

Example 47

cis-5,5a,6,10b-Tetrahydro-9-fluoroindeno[2,1-b]indole.

5,6-Dihydro-9-fluoroindeno[2,1-b]indole (0.55 g, 2.5 mM) in glacial acetic acid (25 $cm^3$) was stirred and treated with sodium cyanoborohydride (2.1 g, 36.5 mM) in small portions over 10 hours, maintaining the temperature below 18°C. The amount of the reducing agent appears crucial since mixtures form if more is added. The reaction mixture was then added to ice-water (100 $cm^3$) and the yellow oil which was formed was separated from the aqueous phase. The pH of the aqueous phase was then adjusted to 6 by the addition of sodium carbonate (30 g), and the colourless oil which was liberated was extracted into diethyl ether (4 x 20 $cm^3$). The combined extracts were dried and evaporated to yield an oil which was extracted with hot 60-80°C petroleum ether (6 x 10 $cm^3$) and the residue triturated with ethanol (1 $cm^3$). This treatment caused the compound to crystallise as a colourless prisms which recrystallised from ethanol to give the title compound. Yield: 60 mg, 11% M.p. 116-117°C; $^1H$ NMR ($CDCl_3$) δ: 3.06 (1H, dd, J = 16.5 and 1.5Hz), 3.31 (1H, dd, J = 16.5 and 6.0Hz), 3.67 (1H, br.s), 4.70 (1H, d, J = 8.0Hz), 4.82 (1H, ddd, J = 8.0, 6.0 and 1.5Hz), 6.43 (1H, dd, J = 8.5 and 4.0Hz), 6.70 (1H, ddd, J = 8.5, 8.5 and 2.5Hz), 7.07 (1H, dd, J = 8.5 and 2.5Hz), 7.16-7.25 (3H, m), 7.33 (1H, m).

Example 48

cis-9-tert-Butyl-5,5a,6,10b,tetrahydroindeno[2,1-b]indole

9-Tert-butyl-5,6-dihydroindeno[2,1-b]indole (0.16 mg, 0.6 mM) in glacial acetic acid (25 $cm^3$) was stirred and treated with sodium cyanoborohydride (0.7 g, 11 mM) in small portions over 3 hours, maintaining the temperature below 18°C. The reaction mixture was then added to ice-water (80 $cm^3$) and the yellow oil which was formed was separated from the aqueous phase. The pH of the aqueous phase was then adjusted to 6 by the addition of sodium carbonate (25 g), and the colourless oil which was liberated was extracted into diethyl ether (6 x 10 $cm^3$). The combined extracts were dried and evaported to yield an oil which was chromatographed on silica eluting with 5% ethyl acetate in 60-80°C petroleum ether. This gave the title compound as colourless prisms. Yield: 0.11g, 7% M.p. 92°C; $^1H$ NMR ($CDCl_3$) δ: 1.30 (9H, s), 3.05 (1H, d, J = 16.5Hz), 3.28 (1H, dd, J = 16.5 and 6.0Hz), 3.79 (1H, s), 4.70 (1H, d, J = 8.0Hz), 4.75 (1H, ddd,

J = 8.0, 6.0 and 2.0Hz), 6.48 (1H, d, J = 8.0Hz), 7.03 (1H, dd, J = 8.0 and 2.0Hz), 7.14-7.24 (3H, m), 7.34 (1H, m), 7.40 (1H, d, J = 2.0Hz).

Example 49

9b,10-Dihydro-9b-methylindeno[1,2-b]indole

A flame dried flask was charged with a solution of the phenylhydrazone of 2-methyl-1-indanone (1.47 g, 6.22 mmol) in DCM (30 $cm^3$), followed by phosphorus trichloride (3.4 $cm^3$ of 2.0M solution in DCM). The solution was heated to reflux for 2 hours, cooled, and poured into a saturated solution of sodium hydrogen carbonate. After stirring for 1 hour, the organic material was extracted with more DCM. The basic componants were back-extracted into 2M hydrochloric acid. This aqueous solution was made basic, and re-extracted with DCM. Evaporation of the solvent in vacuo, and column chromatography of the residue (20% EtOAc/petrol [60-80°C]), gave a clear gum ($R_f$[10% EtOAc/petrol] 0.1) which could be further purified by bulb to bulb distillation to give the title compound as a gum. Yield: 0.4 g (30%). B.p. 170°C (0.2 mmHg). $^1H$ NMR ($CDCl_3$ δ: 1.39 (3H, s,), 2.84 (1H, d,), 3.11 (1H, d,), 6.4, 8.4 (8H, m,).

Example 50

cis-9b,10-Dihydro-8,9b-dimethylindeno[1,2-b]indole

To a solution of 4-methylphenylhydrazine hydrochloride, (9.73 g, 0.06 mmol) in a absolute ethanol (240 $cm^3$) was added dropwise 2-methyl-1-indanone, (8.14 g, 0.056 mmol), followed by conc. hydrochloric acid (3 $cm^3$). The mixture was boiled for 2 hours, the solvents removed, and the residue portioned between diethyl ether and water, and the layers separated. The aqueous phase was extracted with diethyl ether (3 x 50 $cm^3$). The combined organic phases were washed sequentially with saturated sodium bicarbonate solution and brine, dried ($Na_2SO_4$), and evaporated. The crude material thus obtained was purified by flash chromatography on silica gel, eluting with 7-12% ethyl acetate/60-80° petrol to yield a solid (5.05 g, 39%) $^1H$ NMR ($CDCl_3$) δ: 7.87 (1H, m) 7.51 (1H, d, J = 7.9Hz) 7.39 (3H, m) 7.25 (1H, s) 7.15 (1H, d, J = 8.0Hz) 3.07 (1H, d, J = 14.7Hz) 2.81 (1H, d, J = 14.7HZ) 2.41 (3H, s) 7.37 (3H, s).

Example 51

9b,10-Dihydro-9b-methyl-8-isopropylindeno [1,2-b]indole

To a solution of 4-isopropylphenylhydrazine hydrochloride, (6.50 g, 0.035 mmol) in absolute ethanol (140 $cm^3$) was added dropwise 2-methyl-1-indanone, (4.6 g, 0.032 mmol) followed by conc. hydrochloric acid (2.5 $cm^3$). The mixture was refluxed for 2 hours and the ethanol evaporated. The residue was portioned between diethyl ether (100 $cm^3$) and water (100 $cm^3$) and the layers separated. The aqueous phase was extracted with diethyl ether (2 x 30 $cm^3$) and the organic extracts were washed sequentially with saturated sodium bicarbonate solution and brine, and then dried ($Na_2SO_4$). Removal of the solvent gave the title compound which was purified by flash chromatography on silica gel eluting with 10% ethyl acetate/60-80° petrol, to yield a yellow gum; (1.62 g, 25%); $^1H$ NMR ($CDCl_3$) δ: 7.88 (1H, m), 7.55 (1H, d, J = 8.1Hz) 7.41 (3H, m) 7.30 (1H, d, J = 1.8Hz) 7.23 (1H, dd, J = 1.8Hz and 7.1Hz) 3.10 (1H, d, J = 14.7Hz) 2.98 (1H, septet, J = 7.0Hz) 2.85 (1H, d, J = 14.7Hz) 1.39 (3H, s) 1.30 (6H, d, J = 7.0Hz).

The starting materiels DHII and iso-DHII derivatives are further illustrated by the working examples in the co-pending application.

Example 52

cis-(E)-and(Z)-5-Acetyl-8-amino-4b,5,9b,10-tetrahydroindeno-1,2-b]indole

(E)- and (Z)-5-Acetyl-8-nitro-4b,5,9b,10-tetrahydroindeno-[1,2-b]indole(4.2 g) in glacial acetic acid (250 $cm^{3)}$ and water (25 $cm^3$) were stirred and treated with 30% aqueous titanium trichloride (42 $cm^3$) over a period of 5 min.

After a further 15 min., the reaction mixture was poured on to ice and water (800 $cm^3$) and the pH of the solution adjusted to 4.5 with armonium hydroxide. The product was then extracted as rapidly as possible into dichloromethane (6x75 $cm^3$). The combined extracts were dried and evaporated to give a solid which was triturated with diethyl ether to afford the title compounds as a colourless solid. Yield: 2.9 g, 77%. M.p. 196-198°C; $^1H$ NMR δ:2.42(3H,s),2.55(3H,),3.16(1H,d,J=16Hz),3.22 (1H,d,J=16Hz),3.45(2H,m),3.65(4H, exchanged by $D_2O$), 4.01(1H,dd, J==8Hz),4.13(1H,dd,J==7.5Hz) ,5.72 (1H,d,) , 6.26(1H,d,J=8Hz),6.46(2H,d,J=8.5Hz),6.57(1H,s),6.64(1H,), 6.82 (1H,d,J=8.5Hz),7.16-7.25(6H,m),7.38(1H,d,J=7.5Hz), 7.64(1H,d,J=7.5Hz),7.85(1H,d,J=8.5Hz). The title compounds

can be obtained in similar yield by catalytic hydrogenation of the mixed isomeric nitro compounds over 10% palladium on carbon catalyst using chloroform as the solvent.

Example 53

cis-(E)-and(Z)-5-Acetyl-8-(N-acetylamino)-4b,5,9b,10-tetrahydroindeno[1,2-b]indole

The mixture of cis-(E)- and (Z)-5-Acetyl-8-amino-4b,5,9b,10-tetrahydroindeno[1,2-b]indole from Example 52 were acetylated by conventional methods to give the title compounds. [Found: 74.1;H,5.8;N,9.0$C_{19}H_{18}N_2O_2$ requires: C,74.5;H,5.9;N,9.2%].

Pharmacological Properties

The indenoindoles described in the present invention are hydrophobic and stable structures which form cations, stable cation radicals or radicals upon oxidation. They constitute potent antioxidants as measured by inhibition of $Fe^{2+}$-ascorbate induced lipid peroxidation in vitro, with $IC_{50}$ value as low as 10 nM. The compounds of formulas (IA) and (IB) prevent efficiently oxidation of lipoproteins in human plasma in the presence of rabbit smooth muscle cells or mouse peritoneal macrophages. They also prevent ischemic/reperfusion damage to the isolated perfused rat heart, and protect against carbon tetrachloride-, acetaminophen-, methylmethane sulfonate-, menadione-, t-butyl hydroperoxide-, and N-methyl-$N^1$-nitro-N-nitrosoguanidine-induced liver damage in mice, or in isolated rat hepatocytes.

These properties suggest that the structures of formulas (IA) and (IB) have a potential effect in the protection or treatment of ischemic or reperfusion injury, particularly cerebral and cardiac ischemia/infarct, atherosclerosis, thrombosis, embolism, Parkinson's disease, ageing, Alzheimer's disease, neoplasms and toxicity of anti-neoplastic drugs, immunusuppresive agents and inflammation including allergic/inflammatory conditions like broncial asthma and rheumatoid arthritis. Other potential applications are chemoprevention against chemical toxicity or radiation damage. The indenoindole compounds are not appreciably activated by UV light making them candidates for use in skin care products. Another interesting and important feature of the indenoindole compounds of the present invention is their ability to stabilize membranes.

Pharmacological Tests

The most remarkable feature of the compounds of the invention is their efficacy as free-radical scavengers or antioxidants. An assay system measuring the concentration of the compounds of formulas (IA) and (IB) required to inhibit lipid peroxidation by 50% ($IC_{50}$) was used. The lipid peroxidation assay is described below and the data presented in Table 1. Other assays described below are the red blood cell fragility test used for measuring membrane stabilisation by indenoindoles (Table 2), and protection by indenoindoles against cytotoxicity of N-methyl-$N^1$-nitro-N-nitrosoguanidine (MNNG) in rat hepatocytes (Table 3). MNNG is a highly cytotoxic agent, the mechanism of action of which may involve a radical-mediated membrane destabilization.

1. Ascorbate/$Fe^{2+}$-dependent lipid peroxidation

For the ferrous/ascorbate lipid peroxidation system, 6.25 ml of 0.1 M potassium phosphate buffer ($KP_i$), pH 7.4, was added to 12.5 mg dried soy bean phospholipids. After flushing with argon for 2 min, the suspension was sealed with five layers of Parafilm and sonicated until the suspension was translucent. The final reaction mixture was composed of 200 µg/ml phospholipid, 10 µM $FeNH_4 (SO_4)_2$ or $Fe(NH_4)_2(SO_4)_2$, and 100 µM ascorbic acid in 0.1 M $KP_i$ (pH 7.4), and the antioxidant to be tested in acetone or DMSO. The volume of vehicle never exceeded 1% of the total volume. The reaction was initiated by the addition of ascorbic acid plus iron. The reaction was continued at room temperature in a shaking water bath for 30 min and then stopped by the addition of 10 µM of 0.5 M butylated hydroxytoluene in DMSO. The above procedure and the subsequent determination of 2-thiobarbituric acid-reactive material is described in: Shertzer, H.G. et

al, Biochem. Pharmacol. 37, 333 (1988). Table 1 shows the effects of indenoindoles and α-tocopherol on ascorbate/$Fe^{2+}$-dependent lipid peroxidation.

Table 1

| Compounds | $pIC_{50}$ |
|---|---|
| 2,8-Dimethoxy-1,3,dimethyl-THII | 8.1 |
| 8-Methoxy-6-methyl-THII | 8.0 |
| 2-Hydroxy-1,3-dimethyl-THII | 7.9 |
| 8-Isopropyl-4b,9b-dimethyl | 7.7 |
| 8-Isopropyl-4b-methyl-THII | 7.7 |
| 9-Methoxy-iso-THII | 7.7 |
| 4b,8,9b-Trimethyl-THII | 7.6 |
| 8-Fluoro-THII | 7.5 |
| 4b,6,8,9b-Tetramethyl-THII | 7.4 |
| 9-Isopropyl-iso-THII | 7.4 |
| 6,8-Dimethyl-THII | 7.3 |
| 8-Isopropyl-THII | 7.3 |
| 4b,9b-Dimethyl-THII | 7.2 |
| 2-Methoxy-1,3-dimethyl-THII | 7.2 |
| 2-Diethylamino-THII | 7.2 |
| 8-Methyl-THII | 7.1 |
| 4b-Methyl-THII | 7.1 |
| 8-Diethylamino-5-ethyl-THII | 7.1 |
| 8-Methoxy-5-methyl-THII | 7.0 |
| 9b-Methyl-THII | 7.0 |
| THII | 6.9 |
| iso-THII | 6.9 |
| 10,10-Dimethyl-THII | 6.9 |
| 4b,5,8,9b-Tetramethyl-THII | 6.8 |
| 5,8-Dimethyl-THII | 6.4 |
| 6-Methyl-iso-THII | 6.1 |
| 5-Methyl-THII | 6.1 |
| α-Tocopherol (Vitamin E) | 5.0 |

2. Membrane stabilization in red blood cells

The membrane stabilization effect of indenoindoles was assayed by the red blood cell fragility test. Rats were anesthesized With 65 mg pentobarbital per kg body weight by i.p. injection. Blood samples were removed into a heparinized syringe from the left ventricle and diluted 20-fold with buffer containing 140 mM NaCl, 10 mM sodium citrate and 5 mM glucose (pH 7.4) at 0°C. Diluted blood was kept on ice. A 0.75 ml aliquot of blood was added to a 4 ml cuvette containing 10 µl DMSO or 10 µl of the antioxidant dissolved in DMSO vehicle. After 1 min of gentle swirling, 0.75 ml of 0.9 % NaCl or $H_2O$ were added to the cuvette by forceful pipetting, and the absorbance at 656 nm was recorded with a Beckman DU-70 spectrophotometer. When $H_2O$ was added in the absence of a stabilizing agent, absorbance decreased wtihin 15 sec to 0.8. Addition of NaCl instead of $H_2O$ gave a time-independent absorbance of 2.2. In the presence of increasing concentrations of stabilizing chemicals, the absorbance decrease observed after the addition of water was diminished. The % protection from osmolysis was obtained from the equation [E(2.2- 0.8) - A/2.2-0.8)] x 100% , where

A=2.2 minus the absor bance decrease when water is added in the presence of a known concentration of chemical. The % protection is then plotted against several concentrations of the chemical being treated. The red blood cell fragility protective index value (RBC-PIV) is the linear regression slope of this plot, expressed as the percentage protection against osmolysis per µM protecting agent. Table 2 shows the RBC-PIV values for different indenoindoles and a-tocopherol.

Table 2

| Compound | RBC-PIV (%/µM) |
|---|---|
| 8-Methoxy-THII | 0.21 |
| iso-THII | 0.38 |
| THII | 0.41 |
| 9-Methyl-THII | 0.48 |
| 5-Methyl-THII | 0.64 |
| α-Tocopherol | 0.10 |

3. Protection against cytotoxic effects of MNNG in hepatocytes

The protective effects of indenoindoles on MNNG-induced cytotoxicity was assayed with rat hepatocytes. Hepatocytes were prepared from male Sprague-Dawley rats by collagenase treatment as originally described by Zahlten and Stratman (Zahlten, R.N. and Stratman, F.W., Arch. Biochem. Biophys. 163, 600 (1988)), as modified by Reitman et al (Reitman, F.A., Shertzer, H.G. and Berger, M.L., Biochem. Pharmacol. 37,3183 (1988)). In order to improve viability, cells were centrifuged through 0.508 g/ml Percoll (Pharmacia AB, Uppsala, Sweden) in 137 mM NaCl, 8.1 mM $Na_2HPO_4$ and 1.5 mM $KH_2PO_4$ (pH 7.4). Putative protecting agents were added to the cells as solutions in DMSO, with the final concentration of DMSO never exceeding 5 µl/ml of cell suspension. MNNG was added to a concentration of 0.5 mM as a solution in ethanol, giving a final concentration of ethanol of 1%; ethanol alone was by itself without effect. Viability was determined as the percentage of cells that excluded 0.2 % trypan blue. The protective effects by indenoindoles and α-tocopheryl-acetate on cytotoxicity are shown in Table 3. Values are the concentration of compound required to extend by 1 hour the time needed for MNNG to kill 50 % of the viable cells.

Table 3

| Compound | $IC_{50}$ (µM) |
|---|---|
| iso-THII | 2.0 |
| THII | 2.2 |
| 5-Methyl-THII | 2.2 |
| α-Tocopheryl-acetate | 161 |

## Claims

**Claims for the following Contracting States : AT, BE, CH, DE, DK, FR, GB, IT, LI, LU, NL, SE**

1. A compound of the formula IA or IB

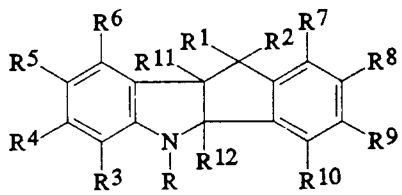 IA or 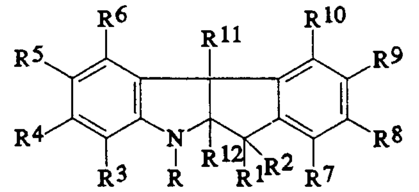 IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group or a $C_1$-$C_6$ alkoxy group, a mono- or di-($C_1$-$C_6$ alkyl) amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the provisos that:

i, when R is methyl in formula IA, then at least one of the radicals $R^1$ to $R^{12}$ is not hydrogen,

ii, when R is hydrogen or acetyl and $R^{11}$ is ethyl in formula IA, then at least one of the radicals $R^1$ to $R^{10}$ or $R^{12}$ is not hydrogen;

and enantiomers and salts thereof.

2. A compound according to claim 1 wherein at least one of $R^3$, $R^5$, $R^7$, $R^9$, $R^{11}$ and $R^{12}$ is a $C_1$-$C_6$ alkyl group, preferably methyl, ethyl or i-propyl.

3. A compound according to claim 1 or 2 wherein at least one of $R^5$ and $R^8$ are a mono- or di-($C_1$-$C_6$ alkyl)amino group, preferably ethyl- or diethylamino.

4. A compound according to claim 1 or 2 wherein at least one of $R^5$ and $R^8$ are hydroxy or a $C_1$-$C_6$ alkoxy group, preferably methoxy.

5. A compound according to any one of the preceding claims wherein R, $R^1$, $R^2$, $R^4$, $R^6$ and $R^{10}$ are hydrogen.

6. A compound according to claim 1 which is
cis-4b,5,9b,10-tetrahydroindeno[1,2-b]indole
cis-4b,5,9b,10-tetrahydro-6,8-dimethylindeno[1,2-b]indole
cis-4b,5,9b,10-tetrahydro-5,8-dimethylindeno[1,2-b]indole
cis-4b,5,9b,10-tetrahydro-8-methylindeno[1,2-b]indole
cis-4b,5,9b,10-tetrahydro-4b,6,8,9b,tetramethylindeno[1,2-b]indole
cis-4b,5,9b,10-tetrahydro-8-isopropylindeno[1,2-b]indole
cis-4b,5,9b,10-tetrahydro-8-methoxy-5-methylindeno[1,2-b]indole
cis-4b,5,9b,10-tetrahydro-8-methoxyindeno[1,2-b]indole
cis-4b,5,9b,10-tetrahydro-10,10-dimethylindeno[1,2-b]indole
cis-4b,5,9b,10-tetrahydro-9b-methylindeno[1,2-b]indole
cis-4b,5,9b,10-tetrahydro-4b,9b-dimethylindeno[1,2-b]indole
cis-4b,5,9b,10-tetrahydro-4b,5,9b,trimethylindeno[1,2-b]indole
cis-4b,5,9b,10-tetrahydro-2-methoxy-1,3-dimethylindeno[1,2-b]indole
cis-4b,5,9b,10-tetrahydro-2-methoxy-1,3-dimethyl-8-isopropylindeno[1,2-b]indole
cis-4b,5,9b,10-tetrahydro-4b-methylindeno[1,2-b]indole
cis-4b,5,9b,10-tetrahydro-2-hydroxy-1,3-dimethyl-8-isopropylindeno[1,2-b]indole

cis-4b,5,9b,10-tetrahydro-2-hydroxy-1,3-dimethylindeno[1,2-b]indole
cis-4b,5,9b,10-tetrahydro-4b,8,9b-trimethylindeno[1,2-b]indole
cis-4b,5,9b,10-tetrahydro-8-isopropyl-4b,9b-dimethylindeno[1,2-b]indole
cis-4b,5,9b,10-tetrahydro-8-isopropyl-4b-methylindeno[1,2-b]indole
cis-4b,5,9b,10-tetrahydro-2,8-dimethoxy-1,3-dimethylindeno[1,2-b]indole
cis-4b,5,9b,10-tetrahydro-4b,5,8,9b-tetramethylindeno[1,2-b]indole
cis-4b,5,9b,10-tetrahydro-8-tert.butylindeno[1,2-b]indole
cis-4b,5,9b,10-tetrahydro-8-methoxy-7,9-dimethylindeno[1,2-b]indole
cis-4b,5,9b,10-tetrahydro-8-methoxy-6-methylindeno[1,2-b]indole
cis-4b,5,9b,10-tetrahydro-8-diethylamino-5-ethylindeno[1,2-b]indole
cis-4b,5,9b,10-tetrahydro-2-diethylaminoindeno[1,2-b]indole
cis-4b,5,9b,10-tetrahydro-8-tert.butyl-4b-methylindeno[1,2-b]indole
cis-4b,5,9b,10-tetrahydro-8-fluoroindeno[1,2-b]indole
cis-5,5a,6,10b-tetrahydroindeno[2,1-b]indole
cis-5,5a,6,10b-tetrahydro-9-methoxyindeno[2,1-b]indole
cis-5,5a,6,10b-tetrahydro-9-isopropylindeno[2,1-b]indole
cis-5,5a,6,10b-tetrahydro-9-methoxy-7-methylindeno[2,1-b]indole
cis-4b,5,9b,10-tetrahydro-4,6-dimethyl-8-methoxyindeno[1,2-b]indole
cis-4b,5,9b,10-tetrahydro-8-methoxy-4b,6,9b-trimethylindeno[1,2-b]indole.

**7.** cis-4b,5,9b,10-Tetrahydro-8-methoxy-6-methylindeno[1,2-b]indole, its enantiomers and salts.

**8.** cis-5,5a,6,10b-Tetrahydro-9-methoxy-7-methylindeno[2,1-b]indole, its enantiomers and salts.

**9.** A compound of formula IA or IB

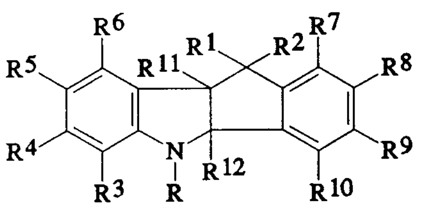

IA or

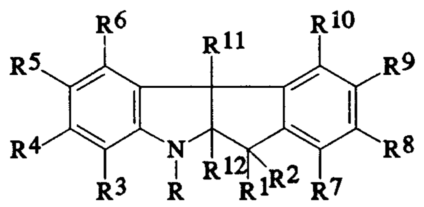

IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$ are hydroxy or a mono- or di-($C_1$-$C_6$ alkyl)amino group,
enantiomers and pharmaceutically acceptable salts thereof; for use in a method of therapy practised on the human or animal body.

**10.** cis-4b,5,9b,10-Tetrahydro-8-methoxy-6-methylindeno[1,2-b]indole, or an enantiomer or pharmaceutically acceptable salt thereof for use in a method of therapy practised on the human or animal body.

**11.** cis-5,5a,6,10b-Tetrahydro-9-methoxy-7-methylindeno[2,1-b]indole, or an enantiomer or pharmaceutically acceptable salt thereof for use in a method of therapy practised on the human or animal body.

**12.** A compound according to any one of claims 9 to 11 for use in the treatment of ischemic or reperfusion injuries, thrombosis, and embolism.

**13.** A compound according to any one of claims 9 to 11 for use in the treatment or prevention of neoplasms.

**14.** A compound according to any one of claims 9 to 11 for use in the treatment of Parkinson's disease, Alzheimer's disease or ageing.

**15.** A compound according to any one of claims 9 to 11 for use in the treatment of atherosclerosis.

**16.** A compound according to any one of claims 9 to 11 for use in the treatment of allergic/inflammatory conditions such as bronchial asthma and rheumatoid arthritis.

**17.** A compound according to any one of claims 9 to 11 for use in the treatment of damage caused by chemicals, radiation, antineoplastic or immunosuppresive agents.

**18.** A compound according to any one of claims 9 to 17 wherein formula IA or IB is as defined in any one of claims 1-6.

**19.** A pharmaceutical composition comprising an active ingredient which is a compound as defined in any one of claims 1-6, an enantiomer or a pharmaceutically acceptable salt thereof together with a pharmaceutically acceptable carrier.

**20.** A pharmaceutical composition comprising an active ingredient which is cis-4b,5,9b,10-tetrahydro-8-methoxy-6-methylindeno[1,2-b]indole, or an enantiomer or a pharmaceutically acceptable salt thereof together with a pharmaceutically acceptable carrier.

**21.** A pharmaceutical composition comprising an active ingredient which is cis-5,5a,6,10b-tetrahydro-9-methoxy-7-methyl-indeno[2,1-b]indole, or an enantiomer or a pharmaceutically acceptable salt thereof together with a pharmaceutically acceptable carrier.

**22.** Use of a compound of formula IA or IB as defined in any one of claims 1 to 9 in the manufacture of a medicament for treatment of ischemic or reperfusion injuries, thrombosis, embolism, atherosclerosis, Parkinson's disease, Alzheimer's disease, ageing, neoplasms, allergic or inflammatory conditions such as bronchial asthma, and rheumatoid arthritis, and damage caused by chemicals, radiation, antineoplastic or immunosuppresive agents.

**23.** A process for preparation of a compound of the formula IA or IB, as defined in any one of claims 1-8, by:

a. reduction of 5,10-dihydroindeno[1,2-b]indole (DHII)

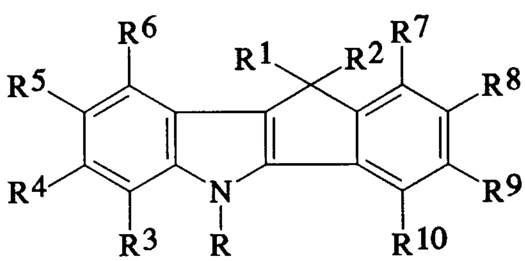

XI

wherein R, $R^1$, $R^2$, $R^3$, $R^4$, $R^5$, $R^6$, $R^7$, $R^8$, $R^9$ and $R^{10}$ are as defined in formula IA, if appropriate preceded by N-alkylation of iso-DHII prior to the reduction or followed by N-alkylation of iso-THII with R-halide or R-sulphate, wherein R is as defined in formula I.

b. Fischer indolisation of a phenylhydrazine of the formula II and 2-substituted-1-indanone of formula III followed by reduction of the indolenines (IV)

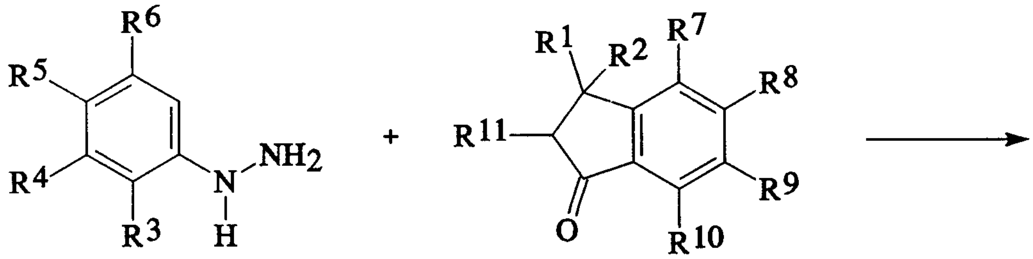

II III

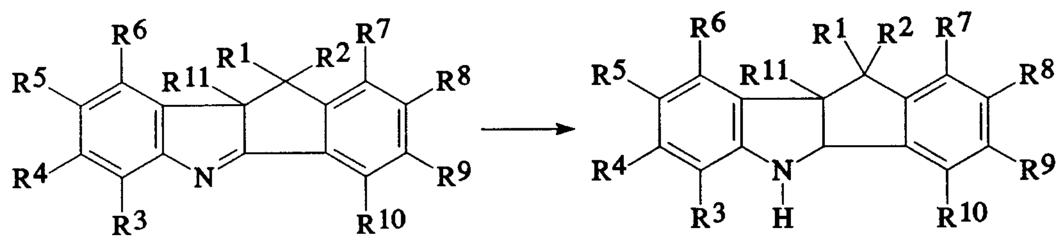

IV V

wherein $R^1$, $R^2$, $R^3$, $R^4$, $R^5$, $R^6$, $R^7$, $R^8$, $R^9$, $R^{10}$ and $R^{11}$ are as defined in formula IA, and if appropriate followed by N-alkylation with a R-halide or R-sulphate, wherein R is as defined in formula IA,

c. reaction of indolenines of formula IV with lithiumalkyls ($R^{12}$-Li)

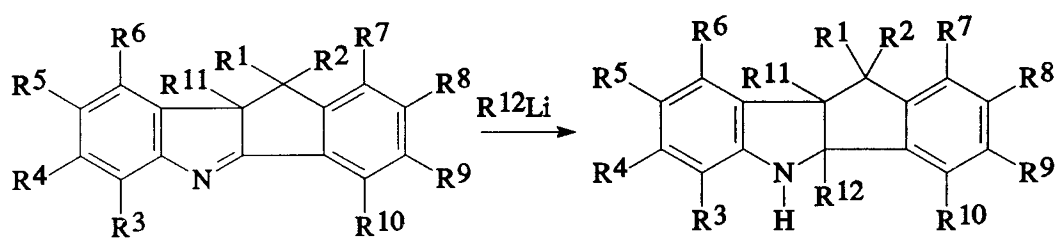


IV VI

wherein $R^1$, $R^2$, $R^3$, $R^4$, $R^5$, $R^6$, $R^7$, $R^8$, $R^9$, $R^{10}$, $R^{11}$ and $R^{12}$ are as defined in formula IA, and if appropriate followed by N-alkylation with a R-halide or R-sulphate, wherein R is as defined in formula IA.

d. reduction of 5,6-dihydroindeno[2,1-b]indole (iso-DHII), if appropriate preceded by N-alkylation of iso-DHII prior to the reduction or followed by N-alkylation of iso-THII with R-halide or R-sulphate, wherein R is as defined in formula I.

e. for 10b-substituted-5,5a,6,10b-tetrahydroindeno [2,1-b]indoles (IX) and analogues, using indan-2-ones (XII) bearing a substituent group at C-3, in Fischer indolisation with phenylhydrazines of the formula (II) followed by reaction of the intermediate VIII with a reducing agent.

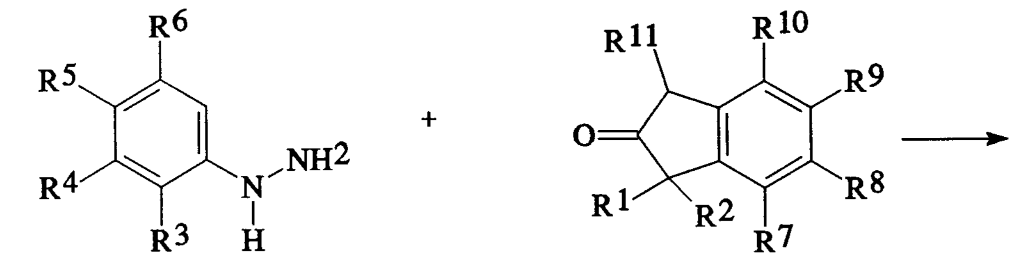

II XII

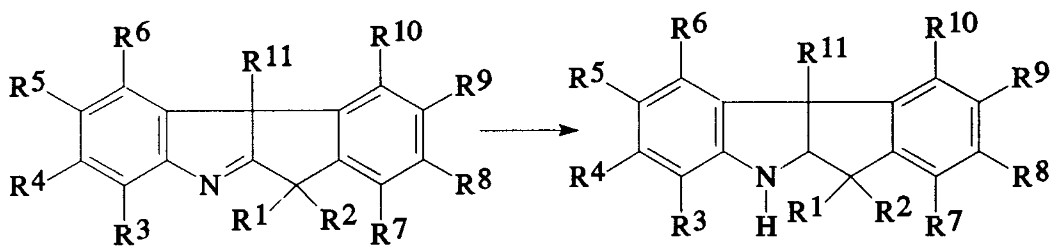

VIII IX

wherein $R^1$, $R^2$, $R^3$, $R^4$, $R^5$, $R^6$, $R^7$, $R^8$, $R^9$, $R^{10}$ and $R^{11}$ are as defined under formula IB, or if appropriate followed by N-alkylation with R-halide or R-sulphate, wherein R is as defined in formula IB.

f. for 5a,10b-Substituted-5,5a,6-10b-tetrahydroindeno[2,1-b]indoles and analogues by reaction of indolenines of formula VIII with lithium alkyls $R^{12}Li$

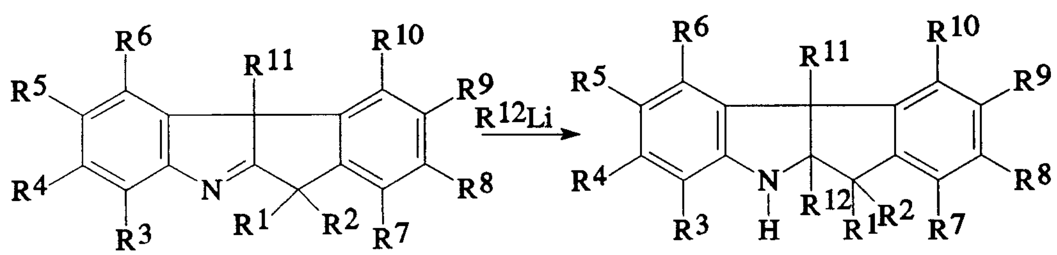


VIII IB

wherein $R^1$, $R^2$, $R^3$, $R^4$, $R^5$, $R^6$, $R^7$, $R^8$, $R^9$, $R^{10}$, $R^{11}$ and $R^{12}$ are as defined under formula IB, or if appropriate followed by N-alkylation with R-halide or R-sulphate, wherein R is as defined in formula IB.

g. for 5-Alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by N-alkylation of corresponding THII or iso-THII with R-halide or R-sulphate.

h. for 5-alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by reduction of corresponding 5-acyl THII or 6-acyl iso-THII.

i. for THII or iso-THII wherein $R^3$ - $R^6$ and/or $R^7$ - $R^{10}$ are defined as a mono- or di-($C_1$-$C_6$ alkyl)amino group and R, $R^1$, $R^2$, $R^{11}$ and $R^{12}$ are as defined in formula I by reduction of corresponding 5-acyl THII or 6-acyl iso-THII nitro components followed by N-alkylation and optionally by acidic hydrolysis.

j. for THII or iso-THII wherein $R^3$ - $R^6$ and/or $R^7$ - $R^{10}$ are defined as hydroxy and R, $R^1$, $R^2$, $R^{11}$ and $R^{12}$ are as defined in formula I by ether dealkylation of corresponding alkoxy substituted THII or iso-THII compounds.

k. for THII or iso-THII wherein $R^{12}$ is a lower alkyl group and R, $R^1$ - $R^{11}$ are as defined in formula I by sequence of metallation of corresponding 4b-unsubstituted analogue followed by alkylation with $R^{12}$-halide or $R^{12}$-sulphate and a final hydrolysis.

**24.** A composition comprising a compound susceptible to oxidative deterioration and a compound of formula IA or IB

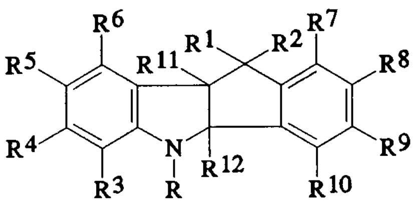

IA or

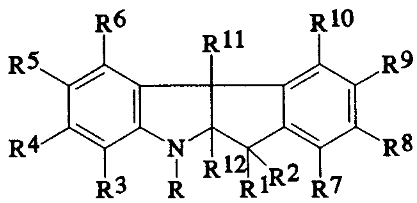

IB

wherein R is hydrogen, a $C_1$-$C_{24}$ alkyl group or $COR^{15}$,
$R^1$, $R^2$, $R^{11}$ and $R^{12}$ are independently selected from hydrogen or a lower alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl) amino group, $NH_2$ or $NR^{13}COR^{14}$.
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl) amino group, $NH_2$ or $NR^{13}COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$ are hydroxy or a mono- or di-($C_1$-$C_6$ alkyl)amino group,
or enantiomers and salts thereof.

**25.** A composition according to claim 24 wherein the compound of formula IA or IB is as defined in any one of claims 1-6.

**26.** A composition comprising a compound susceptible to oxidative deterioration and cis-4b,5,9b,10-tetrahydro-8-methoxy-6-methylindeno[1,2-b]indole, or an enantiomer or salt thereof.

**27.** A composition comprising a compound susceptible to oxidative deterioration and cis-5,5a,6,10b-tetrahydro-9-methoxy-7-methylindeno[2,1-b]indole, or an enantiomer or salt thereof.

**28.** A non-medical method of stabilising a compound susceptible to oxidative deterioration by contacting the susceptible compound with a compound of formula IA or IB as defined in claim 24 or 25.

**29.** A non-medical method of stabilizing a compound susceptible to oxidative deterioration by contacting the susceptible compound with cis-4b,5,9b,10-tetrahydro-8-methoxy-6-methylindeno[1,2-b]indole, or an enantiomer or salt thereof.

**30.** A non-medical method of stabilizing a compound susceptible to oxidative deterioration by contacting the susceptible compound with cis-5,5a,6,10b-tetrahydro-9-methoxy-7-methylindeno[2,1-b]indole, or an enantiomer or salt thereof.

**31.** A non-medical method of preserving a product susceptible to oxidative deterioration by contacting the susceptible product with a compound of formula IA or IB as defined in claim 24 or 25.

**32.** A non-medical method of preserving a product susceptible to oxidative deterioration by contacting the susceptible product with cis-4b,5,9b,10-tetrahydro-8-methoxy-6-methylindeno[1,2-b]indole, or an enantiomer or salt thereof.

**33.** A non-medical method of preserving a product susceptible to oxidative deterioration by contacting the susceptible product with cis-5,5a,6,10b-tetrahydro-9-methoxy-7-methylindeno[2,1-b]indole, or an enantiomer or salt thereof.

**34.** A non-medical use of a compound of formula IA or IB as defined in claim 24 or 25 as an agent for preserving product susceptible to oxidative deterioration.

**35.** A non-medical use of cis-4b,5,9b,10-tetrahydro-8-methoxy-6-methylindeno[1,2-b]indole, or an enantiomer or salt thereof as an agent for preserving product susceptible to oxidative deterioration.

**36.** A non-medical use of cis-5,5a,6,10b-tetrahydro-9-methoxy-7-methylindeno[2,1-b]indole, or an enantiomer or salt thereof as an agent for preserving product susceptible to oxidative deterioration.

**Claims for the following Contracting State : ES**

**1.** A process for the preparation of a compound of the formula IA or IB

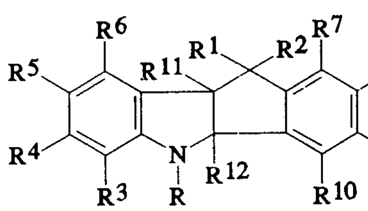 IA or 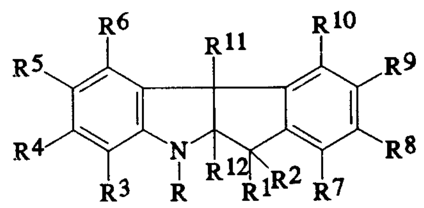 IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group or a $C_1$-$C_6$ alkoxy group, a mono- or di-($C_1$-$C_6$ alkyl) amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the provisos that:

i, when R is methyl in formula IA, then at least one of the radicals $R^1$ to $R^{12}$ is not hydrogen,

ii, when R is hydrogen or acetyl and $R^{11}$ is ethyl in formula IA, then at least one of the radicals $R^1$ to $R^{10}$ or $R^{12}$ is not hydrogen;

or an enantiomer or salt thereof, which process comprises

a. reduction of 5,10-dihydroindeno[1,2-b]indole (DHII)

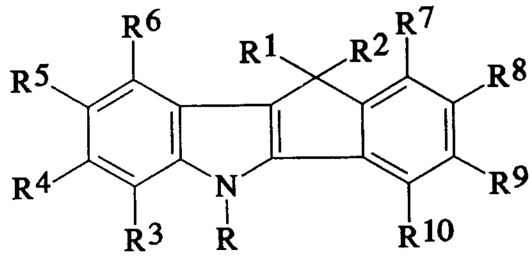

XI

wherein R, $R^1$, $R^2$, $R^3$, $R^4$, $R^5$, $R^6$, $R^7$, $R^8$, $R^9$ and $R^{10}$ are as defined in formula IA, if appropriate preceded by N-alkylation of iso-DHII prior to the reduction or followed by N-alkylation of iso-THII with R-halide or R-sulphate, wherein R is as defined in formula I.

b. Fischer indolisation of a phenylhydrazine of the formula II and 2-substituted-1-indanone of formula III followed by reduction of the indolenines (IV)

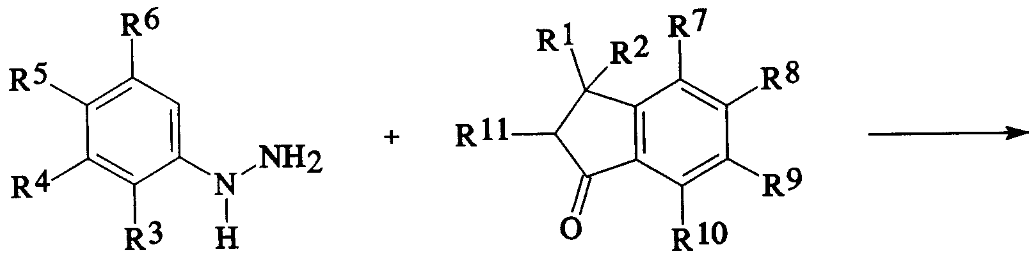

II III

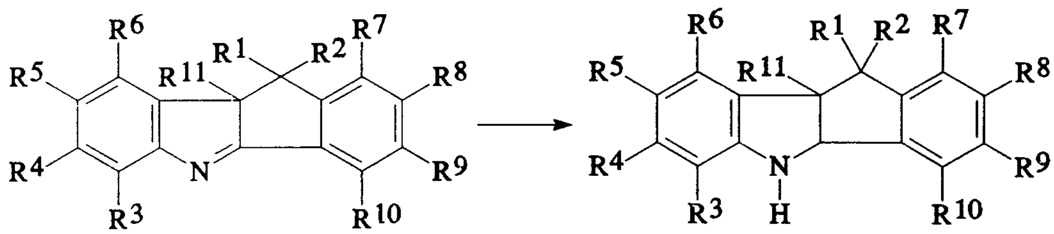

IV V

wherein $R^1$, $R^2$, $R^3$, $R^4$, $R^5$, $R^6$, $R^7$, $R^8$, $R^{9,}$ $R^{10}$ and $R^{11}$ are as defined in formula IA, and if appropriate followed by N-alkylation with a R-halide or R-sulphate, wherein R is as defined in formula IA,

c. reaction of indolenines of formula IV with lithiumalkyls ($R^{12}$-Li)

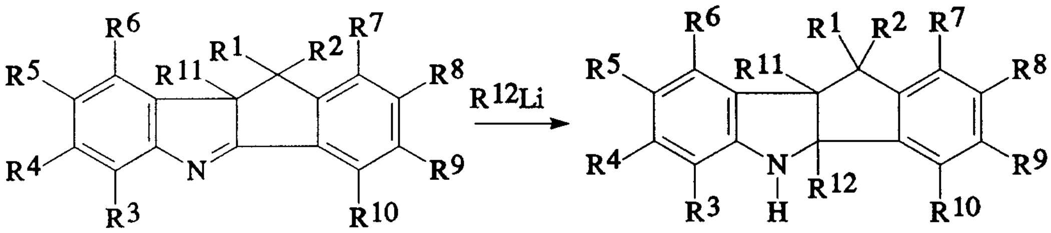


IV VI

wherein $R^1$, $R^2$, $R^3$, $R^4$, $R^5$, $R^6$, $R^7$, $R^8$, $R^9$, $R^{10}$, $R^{11}$ and $R^{12}$ are as defined in formula IA, and if appropriate followed by N-alkylation with a R-halide or R-sulphate, wherein R is as defined in formula IA.

d. reduction of 5,6-dihydroindeno[2,1-b]indole (iso-DHII), if appropriate preceded by N-alkylation of iso-DHII prior to the reduction or followed by N-alkylation of iso-THII with R-halide or R-sulphate, wherein R is as defined in formula I.

e. for 10b-substituted-5,5a,6,10b-tetrahydroindeno [2,1-b]indoles (IX) and analogues, using indan-2-ones (XII) bearing a substituent group at C-3, in Fischer indolisation with phenylhydrazines of the formula (II) followed by reaction of the intermediate VIII with a reducing agent.

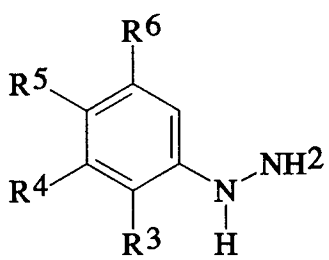

+

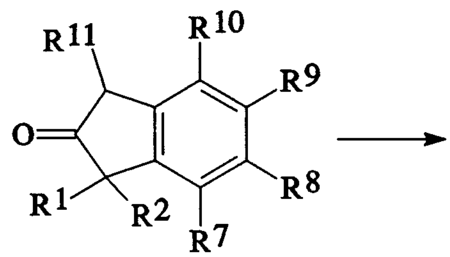

II XII

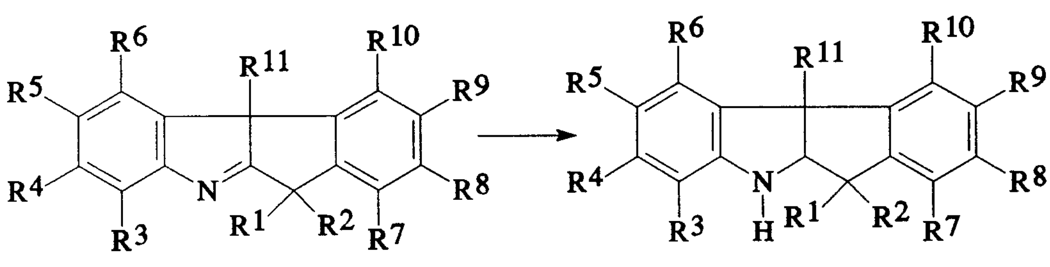

VIII IX

wherein $R^1$, $R^2$, $R^3$, $R^4$, $R^5$, $R^6$, $R^7$, $R^8$, $R^9$, $R^{10}$ and $R^{11}$ are as defined under formula IB, or if appropriate followed by N-alkylation with R-halide or R-sulphate, wherein R is as defined in formula IB.

f. for 5a,10b-Substituted-5,5a,6-10b-tetrahydroindeno[2,1-b]indoles and analogues by reaction of indolenines of formula VIII with lithium alkyls $R^{12}Li$

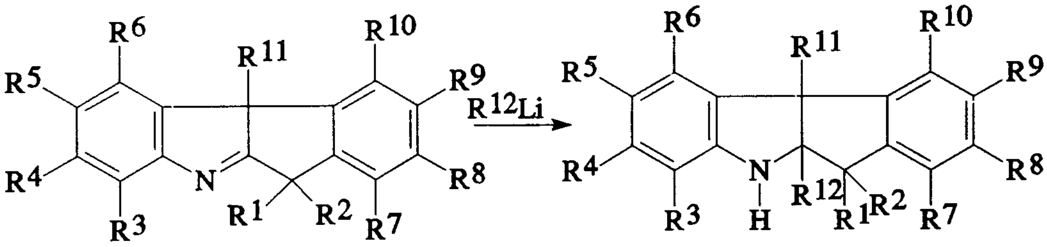


VIII IB

wherein $R^1$, $R^2$, $R^3$, $R^4$, $R^5$, $R^6$, $R^7$, $R^8$, $R^9$, $R^{10}$, $R^{11}$ and $R^{12}$ are as defined under formula IB, or if appropriate followed by N-alkylation with R-halide or R-sulphate, wherein R is as defined in formula IB.

g. for 5-Alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by N-alkylation of corresponding THII or iso-THII with R-halide or R-sulphate.

h. for 5-alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by reduction of corresponding 5-acyl THII or 6-acyl iso-THII.

i. for THII or iso-THII wherein $R^3$ - $R^6$ and/or $R^7$ - $R^{10}$ are defined as a mono- or di-($C_1$-$C_6$ alkyl)amino group and R, $R^1$, $R^2$, $R^{11}$ and $R^{12}$ are as defined in formula I by reduction of corresponding 5-acyl THII or 6-acyl iso-THII nitro components followed by N-alkylation and optionally by acidic hydrolysis.

j. for THII or iso-THII wherein $R^3$ - $R^6$ and/or $R^7$ - $R^{10}$ are defined as hydroxy and R, $R^1$, $R^2$, $R^{11}$ and $R^{12}$ are as defined in formula I by ether dealkylation of corresponding alkoxy substituted THII or iso-THII compounds.

k. for THII or iso-THII wherein $R^{12}$ is a lower alkyl group and R, $R^1$ - $R^{11}$ are as defined in formula I by sequence of metallation of corresponding 4b-unsubstituted analogue followed by alkylation with $R^{12}$-halide or $R^{12}$-sulphate and a final hydrolysis.

2. A process according to claim 1 wherein at least one of $R^3$, $R^5$, $R^7$, $R^9$, $R^{11}$ and $R^{12}$ is a $C_1$-$C_6$ alkyl group, preferably methyl, ethyl or i-propyl.

3. A process according to claim 1 or 2 wherein at least one of $R^5$ and $R^8$ are a mono- or di-($C_1$-$C_6$ alkyl)amino group, preferably ethyl- or diethylamino.

4. A process according to claim 1 or 2 wherein at least one of $R^5$ and $R^8$ are hydroxy or a $C_1$-$C_6$ alkoxy group, preferably methoxy.

5. A process according to any one of the preceding claims wherein R, $R^1$, $R^2$, $R^4$, $R^6$ and $R^{10}$ are hydrogen.

6. A process according to claim 1 in which there is obtained
cis-4b,5,9b,10-tetrahydroindeno[1,2-b]indole
cis-4b,5,9b,10-tetrahydro-6,8-dimethylindeno[1,2-b]indole
cis-4b,5,9b,10-tetrahydro-5,8-dimethylindeno[1,2-b]indole
cis-4b,5,9b,10-tetrahydro-8-methylindeno[1,2-b]indole
cis-4b,5,9b,10-tetrahydro-4b,6,8,9b,tetramethylindeno[1,2-b]indole
cis-4b,5,9b,10-tetrahydro-8-isopropylindeno[1,2-b]indole
cis-4b,5,9b,10-tetrahydro-8-methoxy-5-methylindeno[1,2-b]indole
cis-4b,5,9b,10-tetrahydro-8-methoxyindeno[1,2-b]indole
cis-4b,5,9b,10-tetrahydro-10,10-dimethylindeno[1,2-b]indole
cis-4b,5,9b,10-tetrahydro-9b-methylindeno[1,2-b]indole

cis-4b,5,9b,10-tetrahydro-4b,9b-dimethylindeno[1,2-b]indole
cis-4b,5,9b,10-tetrahydro-4b,5,9b,trimethylindeno[1,2-b]indole
cis-4b,5,9b,10-tetrahydro-2-methoxy-1,3-dimethylindeno[1,2-b]indole
cis-4b,5,9b,10-tetrahydro-2-methoxy-1,3-dimethyl-8-isopropylindeno[1,2-b]indole
cis-4b,5,9b,10-tetrahydro-4b-methylindeno[1,2-b]indole
cis-4b,5,9b,10-tetrahydro-2-hydroxy-1,3-dimethyl-8-isopropylindeno[1,2-b]indole
cis-4b,5,9b,10-tetrahydro-2-hydroxy-1,3-dimethylindeno[1,2-b]indole
cis-4b,5,9b,10-tetrahydro-4b,8,9b-trimethylindeno[1,2-b]indole
cis-4b,5,9b,10-tetrahydro-8-isopropyl-4b,9b-dimethylindeno[1,2-b]indole
cis-4b,5,9b,10-tetrahydro-8-isopropyl-4b-methylindeno[1,2-b]indole
cis-4b,5,9b,10-tetrahydro-2,8-dimethoxy-1,3-dimethylindeno[1,2-b]indole
cis-4b,5,9b,10-tetrahydro-4b,5,8,9b-tetramethylindeno[1,2-b]indole
cis-4b,5,9b,10-tetrahydro-8-tert.butylindeno[1,2-b]indole
cis-4b,5,9b,10-tetrahydro-8-methoxy-7,9-dimethylindeno[1,2-b]indole
cis-4b,5,9b,10-tetrahydro-8-methoxy-6-methylindeno[1,2-b]indole
cis-4b,5,9b,10-tetrahydro-8-diethylamino-5-ethylindeno[1,2-b]indole
cis-4b,5,9b,10-tetrahydro-2-diethylaminoindeno[1,2-b]indole
cis-4b,5,9b,10-tetrahydro-8-tert.butyl-4b-methylindeno[1,2-b]indole
cis-4b,5,9b,10-tetrahydro-8-fluoroindeno[1,2-b]indole
cis-5,5a,6,10b-tetrahydroindeno[2,1-b]indole
cis-5,5a,6,10b-tetrahydro-9-methoxyindeno[2,1-b]indole
cis-5,5a,6,10b-tetrahydro-9-isopropylindeno[2,1-b]indole
cis-5,5a,6,10b-tetrahydro-9-methoxy-7-methylindeno[2,1-b]indole
cis-4b,5,9b,10-tetrahydro-4,6-dimethyl-8-methoxyindeno[1,2-b]indole
cis-4b,5,9b,10-tetrahydro-8-methoxy-4b,6,9b-trimethylindeno[1,2-b]indole.

**7.** A process according to claim 1 in which there is obtained cis-4b,5,9b,10-tetrahydro-8-methoxy-6-methylindeno[1,2-b]indole, or an enantiomer or salt thereof.

**8.** A process according to claim 1 in which there is obtained cis-5,5a,6,10b-tetrahydro-9-methoxy-7-methylindeno[2,1-b]indole, or an enantiomer or salt thereof.

**9.** A process for the preparation of a compound of formula IA or IB

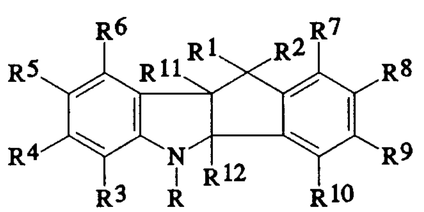

IA or

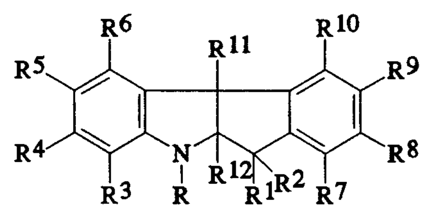

IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$ are hydroxy or a mono- or di-($C_1$-$C_6$ alkyl)amino group,
or an enantiomer or pharmaceutically acceptable salt thereof; for use in a method of therapy practised on the human or animal body, which process comprises

a. reduction of 5,10-dihydroindeno[1,2-b]indole (DHII)

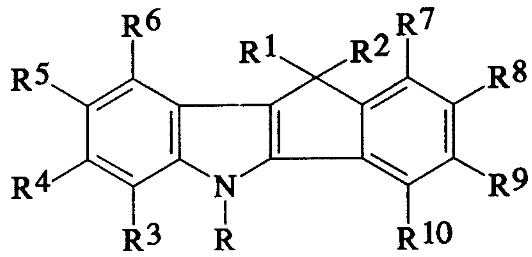

XI

wherein R, $R^1$, $R^2$, $R^3$, $R^4$, $R^5$, $R^6$, $R^7$, $R^8$, $R^9$ and $R^{10}$ are as defined in formula IA, if appropriate preceded by N-alkylation of iso-DHII prior to the reduction or followed by N-alkylation of iso-THII with R-halide or R-sulphate, wherein R is as defined in formula I.

b. Fischer indolisation of a phenylhydrazine of the formula II and 2-substituted-1-indanone of formula III followed by reduction of the indolenines (IV)

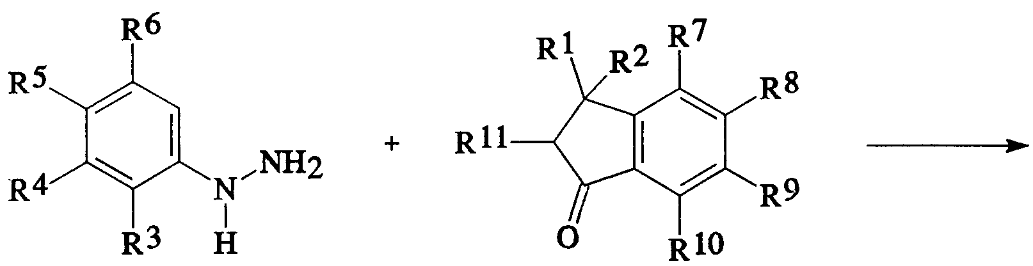

II

III

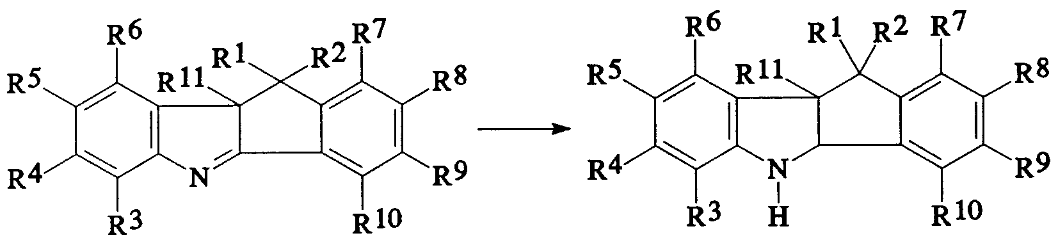

IV

V

wherein $R^1$, $R^2$, $R^3$, $R^4$, $R^5$, $R^6$, $R^7$, $R^8$, $R^{9,}$ $R^{10}$ and $R^{11}$ are as defined in formula IA, and if appropriate followed by N-alkylation with a R-halide or R-sulphate, wherein R is as defined in formula IA,

c. reaction of indolenines of formula IV with lithiumalkyls ($R^{12}$-Li)

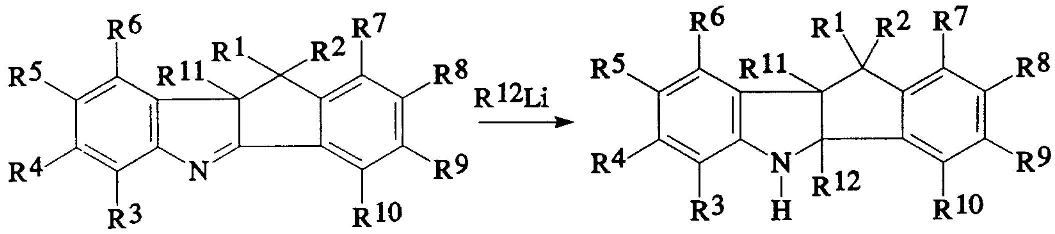


IV VI

wherein $R^1$, $R^2$, $R^3$, $R^4$, $R^5$, $R^6$, $R^7$, $R^8$, $R^9$, $R^{10}$, $R^{11}$ and $R^{12}$ are as defined in formula IA, and if appropriate followed by N-alkylation with a R-halide or R-sulphate, wherein R is as defined in formula IA.

d. reduction of 5,6-dihydroindeno[2,1-b]indole (iso-DHII), if appropriate preceded by N-alkylation of iso-DHII prior to the reduction or followed by N-alkylation of iso-THII with R-halide or R-sulphate, wherein R is as defined in formula I.

e. for 10b-substituted-5,5a,6,10b-tetrahydroindeno [2,1-b]indoles (IX) and analogues, using indan-2-ones (XII) bearing a substituent group at C-3, in Fischer indolisation with phenylhydrazines of the formula (II) followed by reaction of the intermediate VIII with a reducing agent.

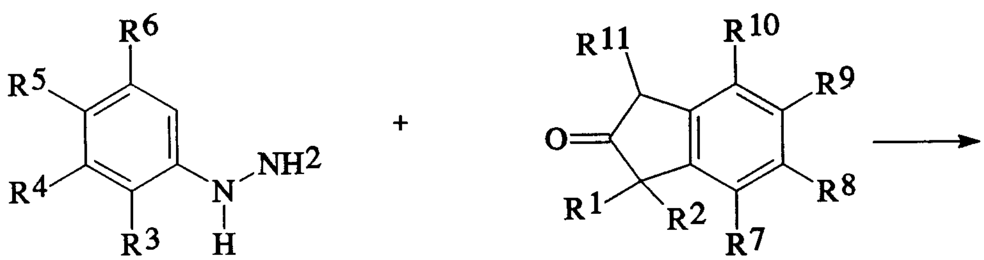

II XII

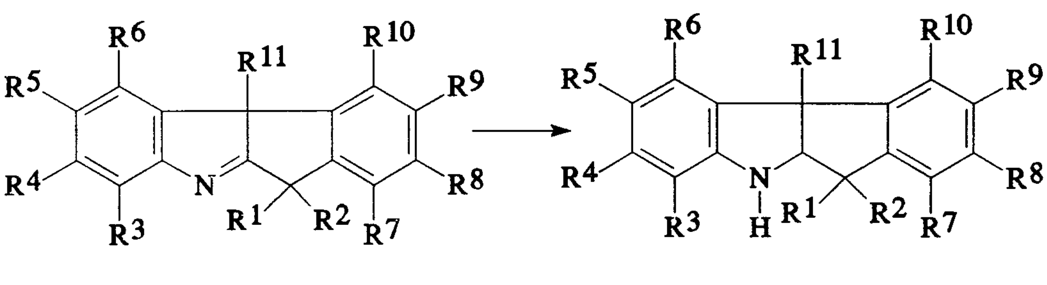

VIII IX

wherein $R^1$, $R^2$, $R^3$, $R^4$, $R^5$, $R^6$, $R^7$, $R^8$, $R^9$, $R^{10}$ and $R^{11}$ are as defined under formula IB, or if appropriate followed by N-alkylation with R-halide or R-sulphate, wherein R is as defined in formula IB.

f. for 5a,10b-Substituted-5,5a,6-10b-tetrahydroindeno[2,1-b]indoles and analogues by reaction of indolenines of formula VIII with lithium alkyls $R^{12}Li$

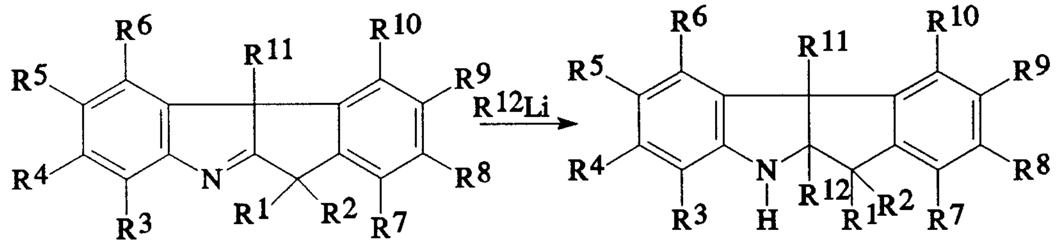


VIII IB

wherein $R^1$, $R^2$, $R^3$, $R^4$,$R^5$, $R^6$, $R^7$, $R^8$, $R^9$, $R^{10}$, $R^{11}$ and $R^{12}$ are as defined under formula IB, or if appropriate followed by N-alkylation with R-halide or R-sulphate, wherein R is as defined in formula IB.

g. for 5-Alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by N-alkylation of corresponding THII or iso-THII with R-halide or R-sulphate.

h. for 5-alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by reduction of corresponding 5-acyl THII or 6-acyl iso-THII.

i. for THII or iso-THII wherein $R^3$ - $R^6$ and/or $R^7$ - $R^{10}$ are defined as a mono- or di-($C_1$-$C_6$ alkyl)amino group and R, $R^1$, $R^2$, $R^{11}$ and $R^{12}$ are as defined in formula I by reduction of corresponding 5-acyl THII or 6-acyl iso-THII nitro components followed by N-alkylation and optionally by acidic hydrolysis.

j. for THII or iso-THII wherein $R^3$ - $R^6$ and/or $R^7$ - $R^{10}$ are defined as hydroxy and R, $R^1$, $R^2$, $R^{11}$ and $R^{12}$ are as defined in formula I by ether dealkylation of corresponding alkoxy substituted THII or iso-THII compounds.

k. for THII or iso-THII wherein $R^{12}$ is a lower alkyl group and R, $R^1$ - $R^{11}$ are as defined in formula I by sequence of metallation of corresponding 4b-unsubstituted analogue followed by alkylation with $R^{12}$-halide or $R^{12}$-sulphate and a final hydrolysis.

**10.** A process according to claim 9 in which there is obtained cis-4b,5,9b,10-tetrahydro-8-methoxy-6-methylindeno[1,2-b]indole, or an enantiomer or pharmaceutically acceptable salt thereof for use in a method of therapy practised on the human or animal body.

**11.** A process according to claim 9 in which there is obtained cis-5,5a,6,10b-tetrahydro-9-methoxy-7-methylindeno[2,1-b]indole, or an enantiomer or pharmaceutically acceptable salt thereof for use in a method of therapy practised on the human or animal body.

**12.** A process according to any one of claims 9 to 11 in which the product is for use in the treatment of ischemic or reperfusion injuries, thrombosis, and embolism.

**13.** A process according to any one of claims 9 to 11 in which the product is for use in the treatment or prevention of neoplasms.

**14.** A process according to any one of claims 9 to 11 in which the product is for use in the treatment of Parkinson's disease, Alzheimer's disease or ageing.

**15.** A process according to any one of claims 9 to 11 in which the product is for use in the treatment of atherosclerosis.

**16.** A process according to any one of claims 9 to 11 in which the product is for use in the treatment of allergic/inflammatory conditions such as bronchial asthma and rheumatoid arthritis.

17. A process according to any one of claims 9 to 11 in which the product is for use in the treatment of damage caused by chemicals, radiation, antineoplastic or immunosuppresive agents.

18. A process according to any one of claims 9 to 17 wherein formula IA or IB is as defined in any one of claims 1-6.

19. A process for preparing a pharmaceutical composition which process comprises mixing an active ingredient which is a compound as defined in any one of claims 1-6, an enantiomer or a pharmaceutically acceptable salt thereof with a pharmaceutically acceptable carrier.

20. A process for preparing a pharmaceutical composition which process comprises mixing an active ingredient which is cis-4b,5,9b,10-tetrahydro-8-methoxy-6-methylindeno[1,2-b]indole, or an enantiomer or a pharmaceutically acceptable salt thereof with a pharmaceutically acceptable carrier.

21. A process for preparing a pharmaceutical composition which process comprises mixing an active ingredient which is cis-5,5a,6,10b-tetrahydro-9-methoxy-7-methyl-indeno[2,1-b]indole, or an enantiomer or a pharmaceutically acceptable salt thereof with a pharmaceutically acceptable carrier.

22. Use of a compound of formula IA or IB as defined in any one of claims 1 to 9 in the manufacture of a medicament for treatment of ischemic or reperfusion injuries, thrombosis, embolism, atherosclerosis, Parkinson's disease, Alzheimer's disease, ageing, neoplasms, allergic or inflammatory conditions such as bronchial asthma, and rheumatoid arthritis, and damage caused by chemicals, radiation, antineoplastic or immunosuppresive agents.

23. A non-medical method of stabilizing a compound susceptible to oxidative deterioration by contacting the compound with a compound of formula IA or IB

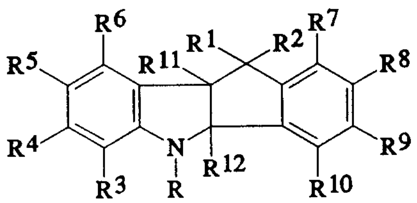 IA or 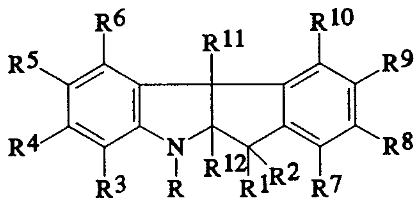 IB

wherein R is hydrogen, a $C_1$-$C_{24}$ alkyl group or $COR^{15}$,
$R^1$, $R^2$, $R^{11}$ and $R^{12}$ are independently selected from hydrogen or a lower alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl) amino group, $NH_2$ or $NR^{13}COR^{14}$.
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl)amino group, $NH_2$ or $NR^{13}$ $COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$ are hydroxy or a mono- or di-($C_1$-$C_6$ alkyl)amino group,
or an enantiomer or salt thereof.

24. A method according to claim 23 wherein the compound of formula IA or IB is as defined in any one of claims 1-6.

25. A non-medical method of stabilizing a compound susceptible to oxidative deterioration by contacting the susceptible compound with cis-4b,5,9b,10-tetrahydro-8-methoxy-6-methylindeno[1,2-b]indole, or an enantiomer or salt thereof.

26. A non-medical method of stabilizing a compound susceptible to oxidative deterioration by contacting the susceptible compound with cis-5,5a,6,10b-tetrahydro-9-methoxy-7-methylindeno[2,1-b]indole, or an enantiomer or salt thereof.

27. A non-medical method of preserving a product susceptible to oxidative deterioration by contacting the susceptible product with a compound of formula IA or IB as defined in claim 23 or 24.

28. A non-medical method of preserving a product susceptible to oxidative deterioration by contacting the susceptible product with cis-4b,5,9b,10-tetrahydro-8-methoxy-6-methylindeno[1,2-b]indole, or an enantiomer or salt thereof.

**29.** A non-medical method of preserving a product susceptible to oxidative deterioration by contacting the susceptible product with cis-5,5a,6,10b-tetrahydro-9-methoxy-7-methylindeno[2,1-b]indole, or an enantiomer or salt thereof.

**30.** A non-medical use of a compound of formula IA or IB as defined in claim 23 or 24 as an agent for preserving product susceptible to oxidative deterioration.

**31.** A non-medical use of cis-4b,5,9b,10-tetrahydro-8-methoxy-6-methylindeno[1,2-b]indole, or an enantiomer or salt thereof as an agent for preserving product susceptible to oxidative deterioration.

**32.** A non-medical use of cis-5,5a,6,10b-tetrahydro-9-methoxy-7-methylindeno[2,1-b]indole, or an enantiomer or salt thereof as an agent for preserving product susceptible to oxidative deterioration.

**Claims for the following Contracting State : GR**

**1.** A process for the preparation of a compound of the formula IA or IB

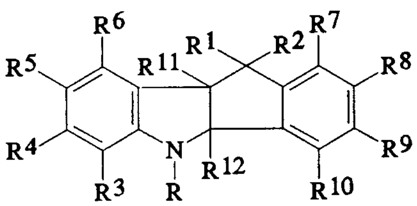

IA or

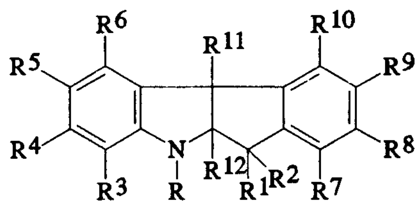

IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group or a $C_1$-$C_6$ alkoxy group, a mono- or di-($C_1$-$C_6$ alkyl) amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the provisos that:

i, when R is methyl in formula IA, then at least one of the radicals $R^1$ to $R^{12}$ is not hydrogen,

ii, when R is hydrogen or acetyl and $R^{11}$ is ethyl in formula IA, then at least one of the radicals $R^1$ to $R^{10}$ or $R^{12}$ is not hydrogen;

or an enantiomer or salt thereof, which process comprises

a. reduction of 5,10-dihydroindeno[1,2-b]indole (DHII)

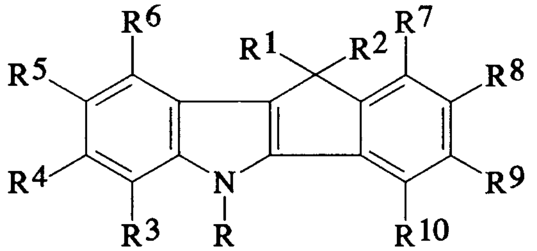

XI

wherein R, $R^1$, $R^2$, $R^3$, $R^4$, $R^5$, $R^6$, $R^7$, $R^8$, $R^9$ and $R^{10}$ are as defined in formula IA, if appropriate preceded by

N-alkylation of iso-DHII prior to the reduction or followed by N-alkylation of iso-THII with R-halide or R-sulphate, wherein R is as defined in formula I.

b. Fischer indolisation of a phenylhydrazine of the formula II and 2-substituted-1-indanone of formula III followed by reduction of the indolenines (IV)

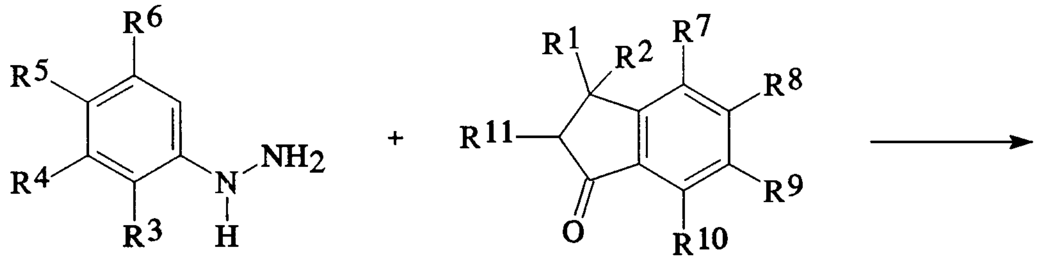

II III

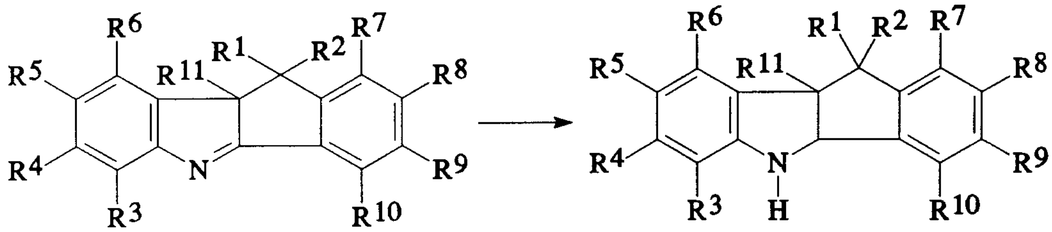

IV V

wherein $R^1$, $R^2$, $R^3$, $R^4$, $R^5$, $R^6$, $R^7$, $R^8$, $R^9$, $R^{10}$ and $R^{11}$ are as defined in formula IA, and if appropriate followed by N-alkylation with a R-halide or R-sulphate, wherein R is as defined in formula IA,

c. reaction of indolenines of formula IV with lithiumalkyls ($R^{12}$-Li)

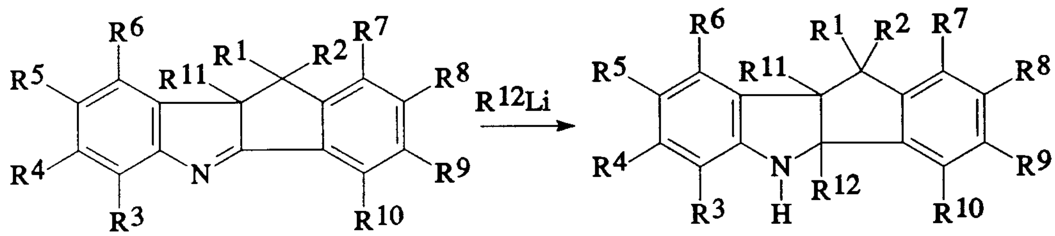


IV VI

wherein $R^1$, $R^2$, $R^3$, $R^4$, $R^5$, $R^6$, $R^7$, $R^8$, $R^9$, $R^{10}$, $R^{11}$ and $R^{12}$ are as defined in formula IA, and if appropriate followed by N-alkylation with a R-halide or R-sulphate, wherein R is as defined in formula IA.

d. reduction of 5,6-dihydroindeno[2,1-b]indole (iso-DHII), if appropriate preceded by N-alkylation of iso-DHII prior to the reduction or followed by N-alkylation of iso-THII with R-halide or R-sulphate, wherein R is as defined in formula I.

e. for 10b-substituted-5,5a,6,10b-tetrahydroindeno [2,1-b]indoles (IX) and analogues, using indan-2-ones (XII) bearing a substituent group at C-3, in Fischer indolisation with phenylhydrazines of the formula (II) followed by reaction of the intermediate VIII with a reducing agent.

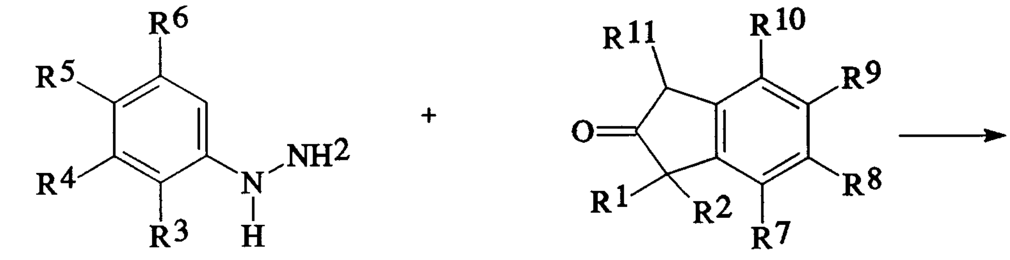

II XII

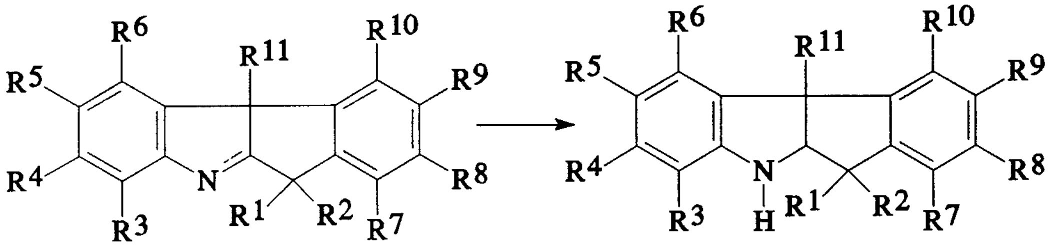

VIII IX

wherein $R^1$, $R^2$, $R^3$, $R^4$, $R^5$, $R^6$, $R^7$, $R^8$, $R^9$, $R^{10}$ and $R^{11}$ are as defined under formula IB, or if appropriate followed by N-alkylation with R-halide or R-sulphate, wherein R is as defined in formula IB.

f. for 5a,10b-Substituted-5,5a,6-10b-tetrahydroindeno[2,1-b]indoles and analogues by reaction of indolenines of formula VIII with lithium alkyls $R^{12}Li$

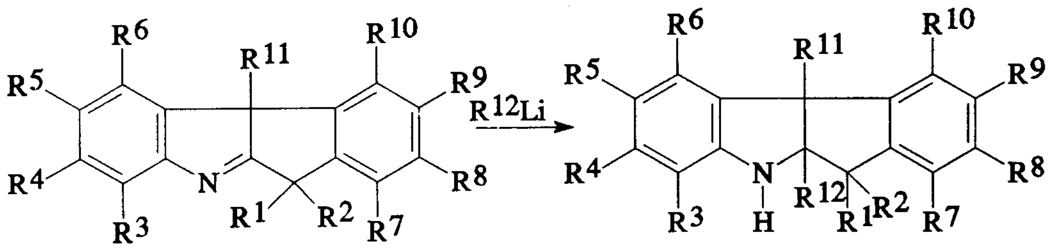


VIII IB

wherein $R^1$, $R^2$, $R^3$, $R^4$, $R^5$, $R^6$, $R^7$, $R^8$, $R^9$, $R^{10}$, $R^{11}$ and $R^{12}$ are as defined under formula IB, or if appropriate followed by N-alkylation with R-halide or R-sulphate, wherein R is as defined in formula IB.

g. for 5-Alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by N-alkylation of corresponding THII or iso-THII with R-halide or R-sulphate.

h. for 5-alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by reduction of corresponding 5-acyl THII or 6-acyl iso-THII.

i. for THII or iso-THII wherein $R^3$ - $R^6$ and/or $R^7$ - $R^{10}$ are defined as a mono- or di-($C_1$-$C_6$ alkyl)amino group and R, $R^1$, $R^2$, $R^{11}$ and $R^{12}$ are as defined in formula I by reduction of corresponding 5-acyl THII or 6-acyl iso-THII nitro components followed by N-alkylation and optionally by acidic hydrolysis.

j. for THII or iso-THII wherein $R^3$ - $R^6$ and/or $R^7$ - $R^{10}$ are defined as hydroxy and R, $R^1$, $R^2$, $R^{11}$ and $R^{12}$ are as defined in formula I by ether dealkylation of corresponding alkoxy substituted THII or iso-THII compounds.

k. for THII or iso-THII wherein $R^{12}$ is a lower alkyl group and R, $R^1$ - $R^{11}$ are as defined in formula I by sequence of metallation of corresponding 4b-unsubstituted analogue followed by alkylation with $R^{12}$-halide or $R^{12}$-sulphate and a final hydrolysis.

**2.** A process according to claim 1 wherein at least one of $R^3$, $R^5$, $R^7$, $R^9$, $R^{11}$ and $R^{12}$ is a $C_1$-$C_6$ alkyl group, preferably methyl, ethyl or i-propyl.

**3.** A process according to claim 1 or 2 wherein at least one of $R^5$ and $R^8$ are a mono- or di-($C_1$-$C_6$ alkyl)amino group, preferably ethyl- or diethylamino.

**4.** A process according to claim 1 or 2 wherein at least one of $R^5$ and $R^8$ are hydroxy or a $C_1$-$C_6$ alkoxy group, preferably methoxy.

**5.** A process according to any one of the preceding claims wherein R, $R^1$, $R^2$, $R^4$, $R^6$ and $R^{10}$ are hydrogen.

**6.** A process according to claim 1 in which there is obtained
cis-4b,5,9b,10-tetrahydroindeno[1,2-b]indole
cis-4b,5,9b,10-tetrahydro-6,8-dimethylindeno[1,2-b]indole
cis-4b,5,9b,10-tetrahydro-5,8-dimethylindeno[1,2-b]indole
cis-4b,5,9b,10-tetrahydro-8-methylindeno[1,2-b]indole
cis-4b,5,9b,10-tetrahydro-4b,6,8,9b,tetramethylindeno[1,2-b]indole
cis-4b,5,9b,10-tetrahydro-8-isopropylindeno[1,2-b]indole
cis-4b,5,9b,10-tetrahydro-8-methoxy-5-methylindeno[1,2-b]indole
cis-4b,5,9b,10-tetrahydro-8-methoxyindeno[1,2-b]indole
cis-4b,5,9b,10-tetrahydro-10,10-dimethylindeno[1,2-b]indole
cis-4b,5,9b,10-tetrahydro-9b-methylindeno[1,2-b]indole
cis-4b,5,9b,10-tetrahydro-4b,9b-dimethylindeno[1,2-b]indole
cis-4b,5,9b,10-tetrahydro-4b,5,9b,trimethylindeno[1,2-b]indole
cis-4b,5,9b,10-tetrahydro-2-methoxy-1,3-dimethylindeno[1,2-b]indole
cis-4b,5,9b,10-tetrahydro-2-methoxy-1,3-dimethyl-8-isopropylindeno[1,2-b]indole
cis-4b,5,9b,10-tetrahydro-4b-methylindeno[1,2-b]indole
cis-4b,5,9b,10-tetrahydro-2-hydroxy-1,3-dimethyl-8-isopropylindeno[1,2-b]indole
cis-4b,5,9b,10-tetrahydro-2-hydroxy-1,3-dimethylindeno[1,2-b]indole
cis-4b,5,9b,10-tetrahydro-4b,8,9b-trimethylindeno[1,2-b]indole
cis-4b,5,9b,10-tetrahydro-8-isopropyl-4b,9b-dimethylindeno[1,2-b]indole
cis-4b,5,9b,10-tetrahydro-8-isopropyl-4b-methylindeno[1,2-b]indole
cis-4b,5,9b,10-tetrahydro-2,8-dimethoxy-1,3-dimethylindeno[1,2-b]indole
cis-4b,5,9b,10-tetrahydro-4b,5,8,9b-tetrmethylindeno[1,2-b]indole
cis-4b,5,9b,10-tetrahydro-8-tert.butylindeno[1,2-b]indole
cis-4b,5,9b,10-tetrahydro-8-methoxy-7,9-dimethylindeno[1,2-b]indole
cis-4b,5,9b,10-tetrahydro-8-methoxy-6-methylindeno[1,2-b]indole
cis-4b,5,9b,10-tetrahydro-8-diethylamino-5-ethylindeno[1,2-b]indole
cis-4b,5,9b,10-tetrahydro-2-diethylaminoindeno-[1,2-b]indole
cis-4b,5,9b,10-tetrahydro-8-tert.butyl-4b-methylindeno[1,2-b]indole
cis-4b,5,9b,10-tetrahydro-8-fluoroindeno[1,2-b]indole
cis-5,5a,6,10b-tetrahydroindeno[2,1-b]indole

cis-5,5a,6,10b-tetrahydro-9-methoxyindeno[2,1-b]indole
cis-5,5a,6,10b-tetrahydro-9-isopropylindeno[2,1-b]indole
cis-5,5a,6,10b-tetrahydro-9-methoxy-7-methylindeno[2,1-b]indole
cis-4b,5,9b,10-tetrahydro-4,6-dimethyl-8-methoxyindeno[1,2-b]indole
cis-4b,5,9b,10-tetrahydro-8-methoxy-4b,6,9b-trimethylindeno[1,2-b]indole.

7. A process according to claim 1 in which there is obtained cis-4b,5,9b,10-tetrahydro-8-methoxy-6-methylindeno[1,2-b]indole, or an enantiomer or salt thereof.

8. A process according to claim 1 in which there is obtained cis-5,5a,6,10b-tetrahydro-9-methoxy-7-methylindeno[2,1-b]indole, or an enantiomer or salt thereof.

9. A process for the preparation of a compound of formula IA or IB

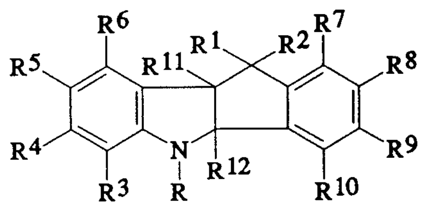

IA or

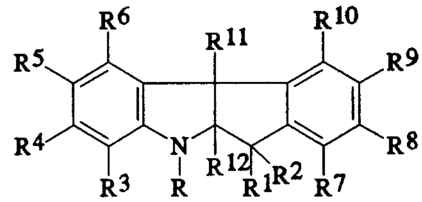

IB

wherein R is hydrogen, a $C_1$-$C_{24}$ alkyl group or $COR^{15}$,
$R^1$, $R^2$, $R^{11}$ and $R^{12}$ are independently selected from hydrogen or a $C_1$-$C_6$ alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl)amino group, $NH_2$ or $NR^{13}COR^{14}$,
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl)amino group, $NH_2$ or $NR^{13}COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$
are hydroxy or a mono- or di-($C_1$-$C_6$ alkyl)amino group, or an enantiomer or pharmaceutically acceptable salt thereof; for use in a method of therapy practised on the human or animal body, which process comprises

a. reduction of 5,10-dihydroindeno[1,2-b]indole (DHII)

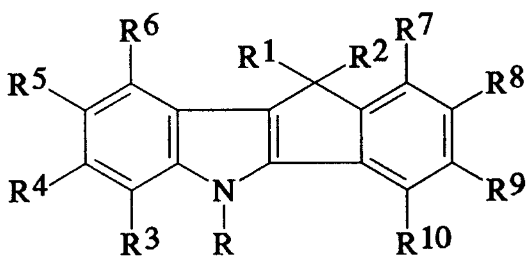

XI

wherein R, $R^1$, $R^2$, $R^3$, $R^4$, $R^5$, $R^6$, $R^7$, $R^8$, $R^9$ and $R^{10}$ are as defined in formula IA, if appropriate preceded by N-alkylation of iso-DHII prior to the reduction or followed by N-alkylation of iso-THII with R-halide or R-sulphate, wherein R is as defined in formula I.

b. Fischer indolisation of a phenylhydrazine of the formula II and 2-substituted-1-indanone of formula III followed by reduction of the indolenines (IV)

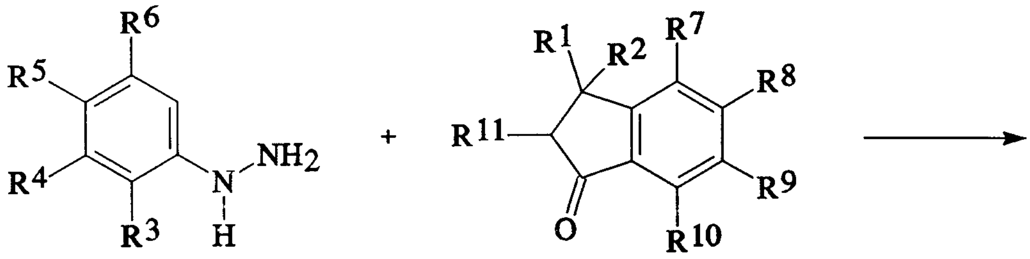

II

III

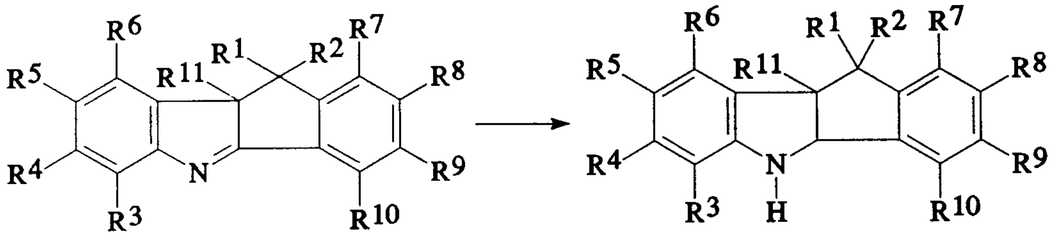

IV

V

wherein $R^1$, $R^2$, $R^3$, $R^4$, $R^5$, $R^6$, $R^7$, $R^8$, $R^9$, $R^{10}$ and $R^{11}$ are as defined in formula IA, and if appropriate followed by N-alkylation with a R-halide or R-sulphate, wherein R is as defined in formula IA,

c. reaction of indolenines of formula IV with lithiumalkyls ($R^{12}$-Li)

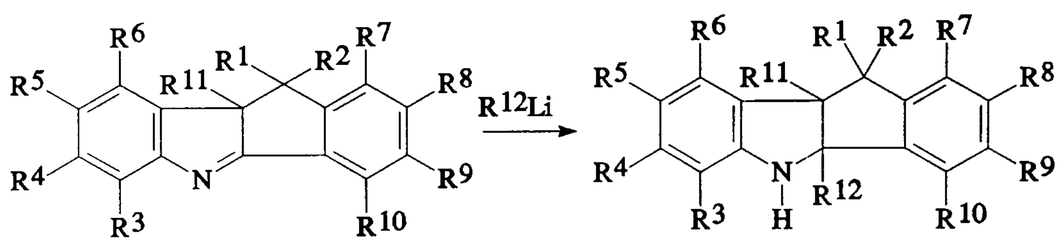


IV

VI

wherein $R^1$, $R^2$, $R^3$, $R^4$, $R^5$, $R^6$, $R^7$, $R^8$, $R^9$, $R^{10}$, $R^{11}$ and $R^{12}$ are as defined in formula IA, and if appropriate followed by N-alkylation with a R-halide or R-sulphate, wherein R is as defined in formula IA.

d. reduction of 5,6-dihydroindeno[2,1-b]indole (iso-DHII), if appropriate preceded by N-alkylation of iso-DHII prior to the reduction or followed by N-alkylation of iso-THII with R-halide or R-sulphate, wherein R is as defined in formula I.

e. for 10b-substituted-5,5a,6,10b-tetrahydroindeno [2,1-b]indoles (IX) and analogues, using indan-2-ones (XII) bearing a substituent group at C-3, in Fischer indolisation with phenylhydrazines of the formula (II) followed by reaction of the intermediate VIII with a reducing agent.

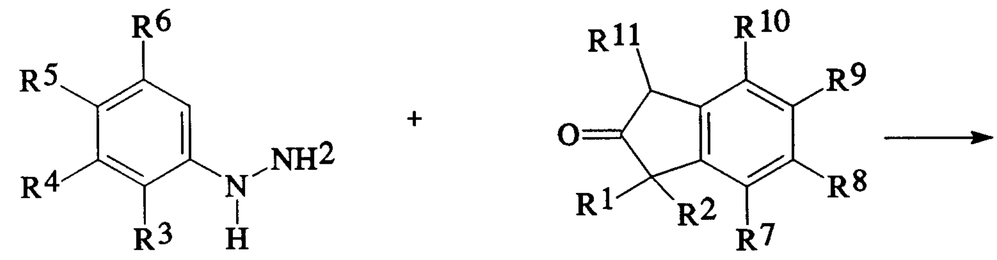

II XII

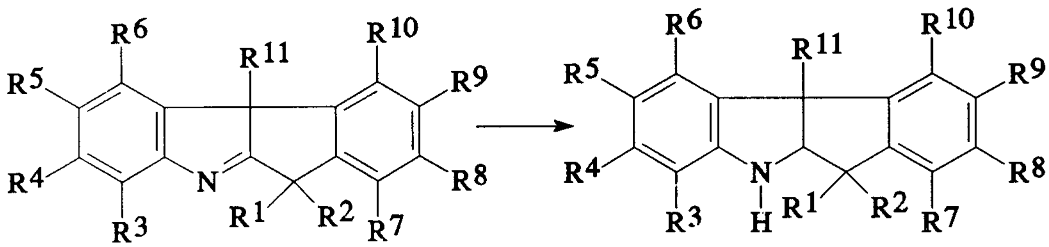

VIII IX

wherein $R^1$, $R^2$, $R^3$, $R^4$, $R^5$, $R^6$, $R^7$, $R^8$, $R^9$, $R^{10}$ and $R^{11}$ are as defined under formula IB, or if appropriate followed by N-alkylation with R-halide or R-sulphate, wherein R is as defined in formula IB.

f. for 5a,10b-Substituted-5,5a,6-10b-tetrahydroindeno[2,1-b]indoles and analogues by reaction of indolenines of formula VIII with lithium alkyls $R^{12}Li$

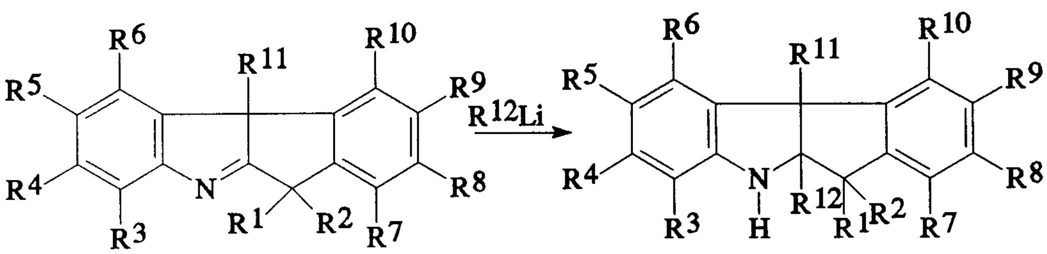


VIII IB

wherein $R^1$, $R^2$, $R^3$, $R^4$, $R^5$, $R^6$, $R^7$, $R^8$, $R^9$, $R^{10}$, $R^{11}$ and $R^{12}$ are as defined under formula IB, or if appropriate followed by N-alkylation with R-halide or R-sulphate, wherein R is as defined in formula IB.

g. for 5-Alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by N-alkylation of corresponding THII or iso-THII with R-halide or R-sulphate.

h. for 5-alkyl THII or 6-alkyl iso-THII wherein R, $R^1$, $R^2$, $R^3$, $R^4$, $R^5$, $R^6$, $R^7$, $R^8$, $R^9$, $R^{10}$ are as defined in formula I by reduction of corresponding 5-acyl THII or 6-acyl iso-THII.

i. for THII or iso-THII wherein $R^3$ - $R^6$ and/or $R^7$ - $R^{10}$ are defined as a mono- or di-($C_1$-$C_6$ alkyl)amino group and R, $R^1$, $R^2$, $R^{11}$ and $R^{12}$ are as defined in formula I by reduction of corresponding 5-acyl THII or 6-acyl iso-THII nitro components followed by N-alkylation and optionally by acidic hydrolysis.

j. for THII or iso-THII wherein $R^3$ - $R^6$ and/or $R^7$ - $R^{10}$ are defined as hydroxy and R, $R^1$, $R^2$, $R^{11}$ and $R^{12}$ are as defined in formula I by ether dealkylation of corresponding alkoxy substituted THII or iso-THII compounds.

k. for THII or iso-THII wherein $R^{12}$ is a lower alkyl group and R, $R^1$ - $R^{11}$ are as defined in formula I by sequence of metallation of corresponding 4b-unsubstituted analogue followed by alkylation with $R^{12}$-halide or $R^{12}$-sulphate and a final hydrolysis.

**10.** A process according to claim 9 in which there is obtained cis-4b,5,9b,10-tetrahydro-8-methoxy-6-methylindeno[1,2-b]indole, or an enantiomer or pharmaceutically acceptable salt thereof for use in a method of therapy practised on the human or animal body.

**11.** A process according to claim 9 in which there is obtained cis-5,5a,6,10b-tetrahydro-9-methoxy-7-methylindeno[2,1-b]indole, or an enantiomer or pharmaceutically acceptable salt thereof for use in a method of therapy practised on the human or animal body.

**12.** A process according to any one of claims 9 to 11 in which the product is for use in the treatment of ischemic or reperfusion injuries, thrombosis, and embolism.

**13.** A process according to any one of claims 9 to 11 in which the product is for use in the treatment or prevention of neoplasms.

**14.** A process according to any one of claims 9 to 11 in which the product is for use in the treatment of Parkinson's disease, Alzheimer's disease or ageing.

**15.** A process according to any one of claims 9 to 11 in which the product is for use in the treatment of atherosclerosis.

**16.** A process according to any one of claims 9 to 11 in which the product is for use in the treatment of allergic/inflammatory conditions such as bronchial asthma and rheumatoid arthritis.

**17.** A process according to any one of claims 9 to 11 in which the product is for use in the treatment of damage caused by chemicals, radiation, antineoplastic or immunosuppresive agents.

**18.** A process according to any one of claims 9 to 17 wherein formula IA or IB is as defined in any one of claims 1-6.

**19.** A process for preparing a pharmaceutical composition which process comprises mixing an active ingredient which is a compound as defined in any one of claims 1-6, an enantiomer or a pharmaceutically acceptable salt thereof with a pharmaceutically acceptable carrier.

**20.** A process for preparing a pharmaceutical composition which process comprises mixing an active ingredient which is cis-4b,5,9b,10-tetrahydro-8-methoxy-6-methylindeno[1,2-b]indole, or an enantiomer or a pharmaceutically acceptable salt thereof with a pharmaceutically acceptable carrier.

**21.** A process for preparing a pharmaceutical composition which process comprises mixing an active ingredient which is cis-5,5a,6,10b-tetrahydro-9-methoxy-7-methyl-indeno[2,1-b]indole, or an enantiomer or a pharmaceutically acceptable salt thereof with a pharmaceutically acceptable carrier.

**22.** Use of a compound of formula IA or IB as defined in any one of claims 1 to 9 in the manufacture of a medicament for treatment of ischemic or reperfusion injuries, thrombosis, embolism, atherosclerosis, Parkinson's disease, Alzheimer's disease, ageing, neoplasms, allergic or inflammatory conditions such as bronchial asthma, and rheumatoid arthritis, and damage caused by chemicals, radiation, antineoplastic or immunosuppresive agents.

**23.** A composition comprising a compound susceptible to oxidative deterioration and a compound of formula IA or IB

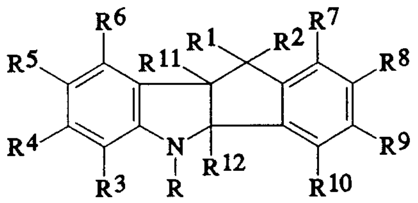 IA or 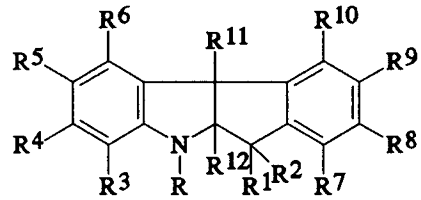 IB

wherein R is hydrogen, a $C_1$-$C_{24}$ alkyl group or $COR^{15}$,
$R^1$, $R^2$, $R^{11}$ and $R^{12}$ are independently selected from hydrogen or a lower alkyl group,
$R^3$, $R^4$, $R^5$ and $R^6$ are independently selected from hydrogen, hydroxy, halogen, a $C_1$-$C_6$ alkyl group, a $C_1$-$C_6$ alkoxy group, a mono- or di-($C_1$-$C_6$ alkyl) amino group, $NH_2$ or $NR^{13}COR^{14}$.
$R^7$, $R^8$, $R^9$ and $R^{10}$ are independently selected from hydrogen, hydroxy, a $C_1$-$C_6$ alkyl group, a $C_1$-$C_6$ alkoxy group or a mono- or di-($C_1$-$C_6$ alkyl)amino group, $NH_2$ or $NR^{13}$ $COR^{14}$,
$R^{13}$, $R^{14}$ and $R^{15}$ are independently selected from hydrogen or a $C_1$-$C_6$ alkyl group,
with the proviso that when R is $COR^{15}$ then at least one of $R^3$ to $R^{10}$ are hydroxy or a mono- or di-($C_1$-$C_6$ alkyl)amino group,
or enantiomers and salts thereof.

**24.** A composition according to claim 23 wherein the compound of formula IA or IB is as defined in any one of claims 1-6.

**25.** A composition comprising a compound susceptible to oxidative deterioration and cis-4b,5,9b,10-tetrahydro-8-methoxy-6-methylindeno[1,2-b]indole, or an enantiomer or salt thereof.

**26.** A composition comprising a compound susceptible to oxidative deterioration and cis-5,5a,6,10b-tetrahydro-9-methoxy-7-methylindeno[2,1-b]indole, or an enantiomer or salt thereof.

**27.** A non-medical method of stabilising a compound susceptible to oxidative deterioration by contacting the susceptible compound with a compound of formula IA or IB as defined in claim 23 or 24.

**28.** A non-medical method of stabilizing a compound susceptible to oxidative deterioration by contacting the susceptible compound with cis-4b,5,9b,10-tetrahydro-8-methoxy-6-methylindeno[1,2-b]indole, or an enantiomer or salt thereof.

**29.** A non-medical method of stabilizing a compound susceptible to oxidative deterioration by contacting the susceptible compound with cis-5,5a,6,10b-tetrahydro-9-methoxy-7-methylindeno[2,1-b]indole, or an enantiomer or salt thereof.

**30.** A non-medical method of preserving a product susceptible to oxidative deterioration by contacting the susceptible product with a compound of formula IA or IB as defined in claim 23 or 24.

**31.** A non-medical method of preserving a product susceptible to oxidative deterioration by contacting the susceptible product with cis-4b,5,9b,10-tetrahydro-8-methoxy-6-methylindeno[1,2-b]indole, or an enantiomer or salt thereof.

**32.** A non-medical method of preserving a product susceptible to oxidative deterioration by contacting the susceptible product with cis-5,5a,6,10b-tetrahydro-9-methoxy-7-methylindeno[2,1-b]indole, or an enantiomer or salt thereof.

**33.** A non-medical use of a compound of formula IA or IB as defined in claim 23 or 24 as an agent for preserving product susceptible to oxidative deterioration.

**34.** A non-medical use of cis-4b,5,9b,10-tetrahydro-8-methoxy-6-methylindeno[1,2-b]indole, or an enantiomer or salt thereof as an agent for preserving product susceptible to oxidative deterioration.

**35.** A non-medical use of cis-5,5a,6,10b-tetrahydro-9-methoxy-7-methylindeno[2,1-b]indole, or an enantiomer or salt thereof as an agent for preserving product susceptible to oxidative deterioration.

## Patentansprüche

### Patentansprüche für folgende Vertragsstaaten : AT, BE, CH, DE, DK, FR, GB, IT, LI, LU, NL, SE

**1.** Verbindung der Formel IA oder IB

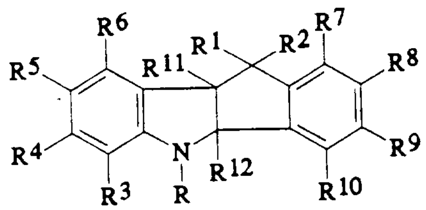 IA oder 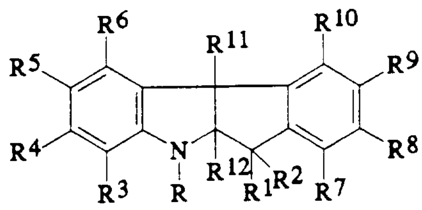 IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe oder einer $C_1$-$C_6$-Alkoxygruppe, einer Mono- oder Di-($C_1$-$C_6$-alkyl)-aminogruppe, $NH_2$ oder $NR^{13}COR^{14}$`
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter den Bedingungen, daß dann,

i, wenn R in Formel IA Methyl ist, mindestens eines der Radikale $R^1$ bis $R^{12}$ kein Wasserstoff ist,

ii, wenn R Wasserstoff oder Acetyl und $R^{11}$ Ethyl in Formel IA ist, mindestens eines der Radikale $R^1$ bis $R^{10}$ oder $R^{12}$ kein Wasserstoff ist;

und Enantiomere und Salze davon.

**2.** Verbindung nach Anspruch 1, worin mindestens eines von $R^3$, $R^5$, $R^7$, $R^9$, $R^{11}$ und $R^{12}$ eine $C_1$-$C_6$-Alkylgruppe, bevorzugt Methyl, Ethyl oder i-Propyl, ist.

**3.** Verbindung nach Anspruch 1 oder 2, worin mindestens eines von $R^5$ und $R^8$ eine Mono- oder Di-($C_1$-$C_6$-alkyl)-aminogruppe, bevorzugt Ethyl- oder Diethylamino, ist.

**4.** Verbindung nach Anspruch 1 oder 2, worin mindestens eines von $R^5$ und $R^8$ eine Hydroxyl- oder eine $C_1$-$C_6$-Alkoxygruppe, bevorzugt Methoxy, ist.

**5.** Verbindung nach einem der vorangegangenen Ansprüche, worin R, $R^1$, $R^2$, $R^4$, $R^6$ und $R^{10}$ Wasserstoff sind.

**6.** Verbindung nach Anspruch 1, welche ist:
cis-4b,5,9b,10-Tetrahydroindeno-[1,2-b]-indol,
cis-4b,5,9b,10-Tetrahydro-6,8-dimethylindeno-[1,2-b]-indol,
cis-4b,5,9b,10-Tetrahydro-5,8-dimethylindeno-[1,2-b]-indol,
cis-4b,5,9b,10-Tetrahydro-8-methylindeno-[1,2-b]-indol,
cis-4b,5,9b,10-Tetrahydro-4b,6,8,9b,tetramethylindeno-[1,2-b]-indol,
cis-4b,5,9b,10-Tetrahydro-8-isopropylindeno-[1,2-b]-indol,
cis-4b,5,9b,10-Tetrahydro-8-methoxy-5-methylindeno-[1,2-b]-indol,
cis-4b,5,9b,10-Tetrahydro-8-methoxyindeno-[1,2-b]-indol,
cis-4b,5,9b,10-Tetrahydro-10,10-dimethylindeno-[1,2-b]-indol,
cis-4b,5,9b,10-Tetrahydro-9b-methylindeno-[1,2-b]-indol,
cis-4b,5,9b,10-Tetrahydro-4b,9b-dimethylindeno-[1,2-b]-indol,
cis-4b,5,9b,10-Tetrahydro-4b,5,9b,Trimethylindeno-[1,2-b]-indol,
cis-4b,5,9b,10-Tetrahydro-2-methoxy-1,3-dimethylindeno-[1,2-b]-indol,
cis-4b,5,9b,10-Ietrahydro-2-methoxy-1,3-dimethyl-8-isopropylindeno-[1,2-b]-indol,

cis-4b,5,9b,10-Tetrahydro-4b-methylindeno-[1,2-b]-indol,
cis-4b,5,9b,10-Tetrahydro-2-hydroxy-1,3-dimethyl-8-isopropylindeno-[1,2-b]-indol,
cis-4b,5,9b,10-Tetrahydro-2-hydroxy-1,3-dimethylindeno-[1,2-b]-indol,
cis-4b,5,9b,10-Tetrahydro-4b,8,9b-trimethylindeno-[1,2-b]-indol,
cis-4b,5,9b,10-Tetrahydro-8-isopropyl-4b,9b-dimethylindeno-[1,2-b]-indol,
cis-4b,5,9b,10-Tetrahydro-8-isopropyl-4b-methylindeno-[1,2-b]-indol,
cis-4b,5,9b,10-Tetrahydro-2,8-dimethoxy-1,3-dimethylindeno-[1,2-b]-indol,
cis-4b,5,9b,10-Tetrahydro-4b,5,8,9b-tetramethylindeno-[1,2-b]-indol,
cis-4b,5,9b,10-Tetrahydro-8-tert.-Butylindeno-[1,2-b]-indol,
cis-4b,5,9b,10-Tetrahydro-8-methoxy-7,9-dimethylindeno-[1,2-b]-indol,
cis-4b,5,9b,10-Tetrahydro-8-methoxy-6-methylindeno-[1,2-b]-indol,
cis-4b,5,9b,10-Tetrahydro-8-diethylamino-5-ethylindeno-[1,2-b]-indol,
cis-4b,5,9b,10-Tetrahydro-2-diethylaminoindeno-[1,2-b]-indol,
cis-4b,5,9b,10-Tetrahydro-8-tert.-Butyl-4b-methylindeno-[1,2-b]-indol,
cis-4b,5,9b,10-Tetrahydro-8-fluoroindeno-[1,2-b]-indol,
cis-5,5a,6,10b-Tetrahydroindeno-[2,1-b]-indol,
cis-5,5a,6,10b-Tetrahydro-9-methoxyindeno-[2,1-b]-indol,
cis-5,5a,6,10b-Tetrahydro-9-isopropylindeno-[2,1-b]-indol,
cis-5,5a,6,10b-Tetrahydro-9-methoxy-7-methylindeno-[2,1-b]-indol,
cis-4b,5,9b,10-Tetrahydro-4,6-dimethyl-8-methoxyindeno-[1,2-b]-indol,
cis-4b,5,9b,10-Tetrahydro-8-methoxy-4b,6,9b-trimethylindeno-[1,2-b]-indol.

**7.** cis-4b,5,9b,10-Tetrahydro-8-methoxy-6-methylindeno-[1,2-b]-indol, seine Enantiomere und Salze.

**8.** cis-5,5a,6,10b-Tetrahydro-9-methoxy-7-methylindeno-[2,1-b]-indol, seine Enantiomere und Salze.

**9.** Verbindung der Formel IA oder IB

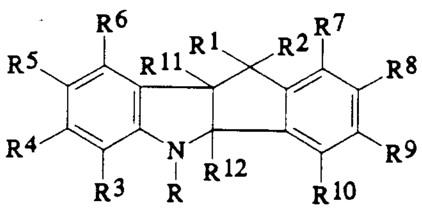 IA oder 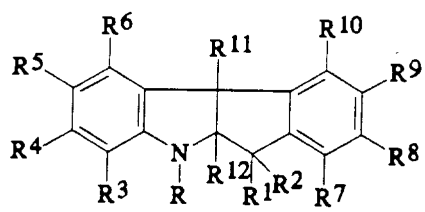 IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ Hydroxyl oder eine Mono- oder Di-($C_1$-$C_6$-alkyl)-aminogruppe ist,
Enantiomere und pharmazeutisch geeignete Salze davon; zur Verwendung bei einer Therapiemethode, die am menschlichen oder tierischen Körper Anwendung findet.

**10.** cis-4b,5,9b,10-Tetrahydro-8-methoxy-6-methylindeno-[1,2-b]-indol oder ein Enantiomer oder pharmazeutisch geeignetes Salz davon, zur Verwendung bei einer Therapiemethode, die am menschlichen oder tierischen Körper Anwendung findet.

**11.** cis-5,5a,6,10b-Tetrahydro-9-methoxy-7-methylindeno-[2,1-b]-indol oder ein Enantiomer oder pharmazeutisch geeignetes Salz davon, zur Verwendung bei einer Therapiemethode, die am menschlichen oder tierischen Körper Anwendung findet.

**12.** Verbindung nach einem der Ansprüche 9 bis 11 zur Verwendung bei der Behandlung von ischämischen oder Reperfusions-Störungen, Thrombose und Embolie.

**13.** Verbindung nach einem der Ansprüche 9 bis 11 zur Verwendung bei der Behandlung oder Verhütung von Neoplasmen.

**14.** Verbindung nach einem der Ansprüche 9 bis 11 zur Verwendung bei der Behandlung von Parkinson-Krankheit, Alzheimer-Krankheit oder Alterserscheinungen.

**15.** Verbindung nach einem der Ansprüche 9 bis 11 zur Verwendung bei der Behandlung von Arteriosklerose.

**16.** Verbindung nach einem der Ansprüche 9 bis 11 zur Verwendung bei der Behandlung von allergischen/entzündlichen Zuständen, wie z.B. Bronchialasthma und rheumatische Arthritis.

**17.** Verbindung nach einem der Ansprüche 9 bis 11 zur Verwendung bei der Behandlung von durch Chemikalien, Bestrahlung, antineoplastische oder immunsuppresive Mittel hervorgerufenen Schädigungen.

**18.** Verbindung nach einem der Ansprüche 9 bis 17, worin die Formel IA oder IB wie in einem der Ansprüche 1-6 definiert ist.

**19.** Pharmazeutische Zusammensetzung, umfassend einen Wirkstoff, welcher eine Verbindung wie in einem der Ansprüche 1-6 definiert, ein Enantiomer oder ein pharmazeutisch geeignetes Salz davon ist, zusammen mit einem pharmazeutisch geeigneten Träger.

**20.** Pharmazeutische Zusammensetzung, umfassend einen Wirkstoff, welcher cis-4b,5,9b,10-Tetrahydro-8-methoxy-6-methylindeno-[1,2-b]-indol oder ein Enantiomer oder pharmazeutisch geeignetes Salz davon ist, zusammen mit einem pharmazeutisch geeigneten Träger.

**21.** Pharmazeutische Zusammensetzung, umfassend einen Wirkstoff, welcher cis-5,5a,6,10b-Tetrahydro-9-methoxy-7-methylindeno-[2,1-b]-indol oder ein Enantiomer oder pharmazeutisch geeignetes Salz davon ist, zusammen mit einem pharmazeutisch geeigneten Träger.

**22.** Verwendung einer Verbindung der Formel IA oder IB, wie in einem der Ansprüche 1 bis 9 definiert, bei der Herstellung eines Medikaments zur Behandlung von ischämischen oder Reperfusions-Störungen, Thrombose, Embolie, Arteriosklerose, Parkinson-Krankheit, Alzheimer-Krankheit, Alterserscheinungen, Neoplasmen, allergischen oder entzündlichen Zuständen, wie z.B. Bronchialasthma und rheumatische Arthritis, und durch Chemikalien, Bestrahlung, antineoplastische oder immunsuppresive Mitteln hervorgerufenen Schädigungen.

**23.** Verfahren zur Herstellung einer Verbindung der Formel IA oder IB, wie in einem der Ansprüche 1-8 definiert, durch:

a. Reduktion von 5,10-Dihydroindeno-[1,2-b]-indol (DHII)

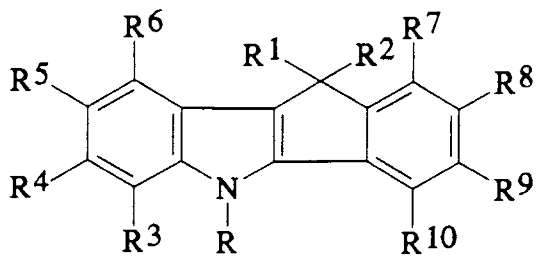

XI

worin R, $R^1$, $R^2$, $R^3$, $R^4$, $R^5$, $R^6$, $R^7$, $R^8$, $R^9$ und $R^{10}$ wie in Formel IA definiert sind, wobei, wenn geeignet eine

N-Alkylierung des iso-DHII der Reduktion vorausgeht oder eine N-Alkylierung des iso-THII mit R-halid oder R-sulfat folgt, worin R wie in Formel I definiert ist.

b. Fischer-Indolisation eines Phenylhydrazins der Formel II und eines 2-substituierten-1-Indanons der Formel III, gefolgt von einer Reduktion der Indolenine (IV)

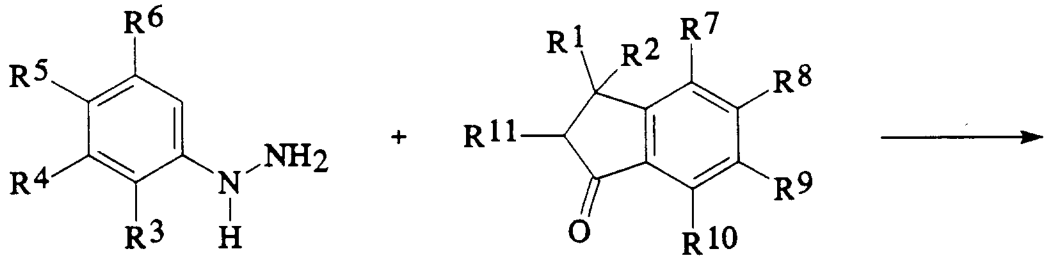

II III

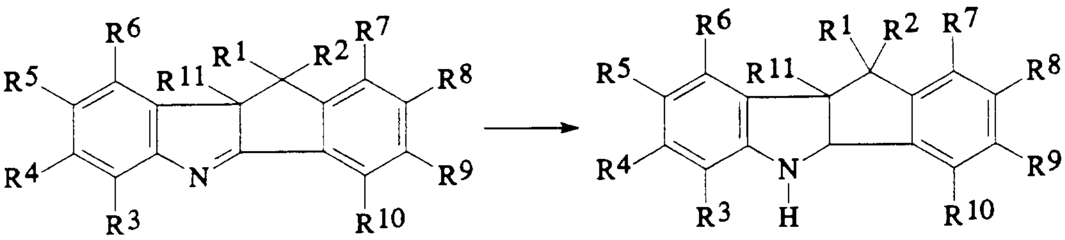

IV V

worin $R^1$, $R^2$, $R^3$, $R^4$, $R^5$, $R^6$, $R^7$, $R^8$, $R^9$, $R^{10}$ und $R^{11}$ wie in Formel IA definiert sind und wobei, wenn geeignet, eine N-Alkylierung mit einem R-halid oder R-sulfat folgt, worin R wie in Formel IA definiert ist.

c. Reaktion von Indoleninen der Formel IV mit Lithiumalkylen ($R^{12}$-Li)

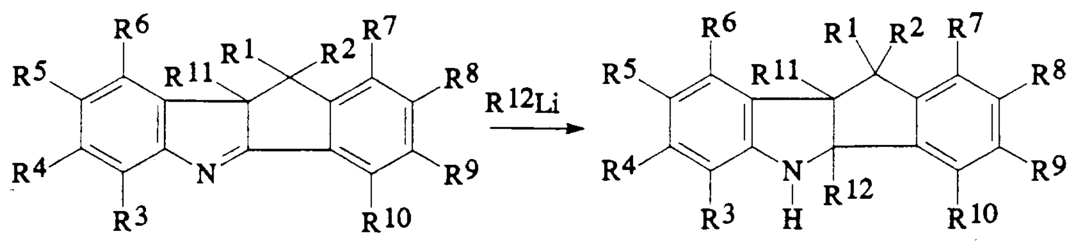


IV VI

worin $R^1$, $R^2$, $R^3$, $R^4$, $R^5$, $R^6$, $R^7$, $R^8$, $R^9$, $R^{10}$, $R^{11}$ und $R^{12}$ wie in Formel IA definiert sind und wobei, wenn geeignet, eine N-Alkylierung mit einem R-halid oder R-sulfat folgt, worin R wie in Formel IA definiert ist.

d. Reduktion von 5,6-Dihydroindeno-[2,1-b]-indol (iso-DHII), wobei, wenn geeignet, eine N-Alkylierung des iso-DHII der Reduktion vorausgeht oder eine N-Alkylierung des iso-THII mit R-halid oder R-sulfat folgt, worin R wie in Formel I definiert ist.

e. zum Erhalt von 10b-substituierten-5,5a,6,10b-Tetrahydroindeno-[2,1-b]-indolen (IX) und Analogen, unter Verwendung von Indan-2-onen (XII), die eine Substitutionsgruppe bei C-3 aufweisen, bei Fischer-Indolisation mit Phenylhydrazinen der Formel (II), gefolgt von einer Reaktion der Zwischenstufe VIII mit einem Reduktionsmittel.

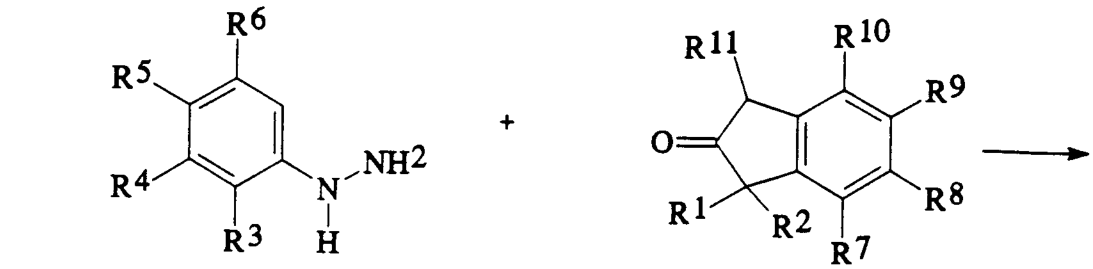

II XII

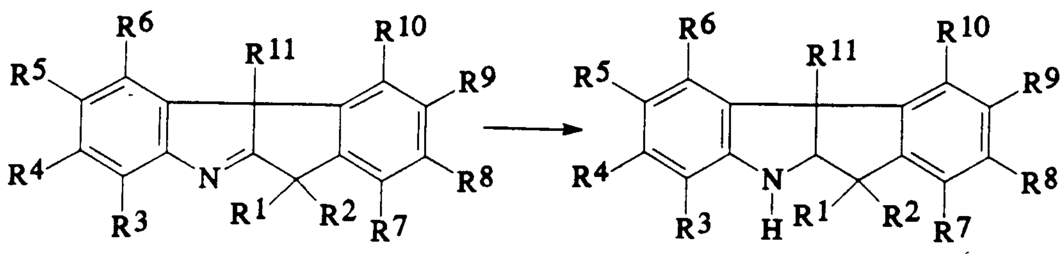

VIII IX

worin $R^1$, $R^2$, $R^3$, $R^4$, $R^5$, $R^6$, $R^7$, $R^8$, $R^9$, $R^{10}$ und $R^{11}$ wie in Formel IB definiert sind oder wobei, wenn geeignet, eine N-Alkylierung mit einem R-halid oder R-sulfat folgt, worin R wie in Formel IB definiert ist.

f. zum Erhalt von 5a,10b-substituierten-5,5a,6,10b-Tetrahydroindeno-[2,1-b]-indolen und Analogen durch Reaktion von Indoleninen der Formel VIII mit Lithiumalkylen $R^{12}Li$

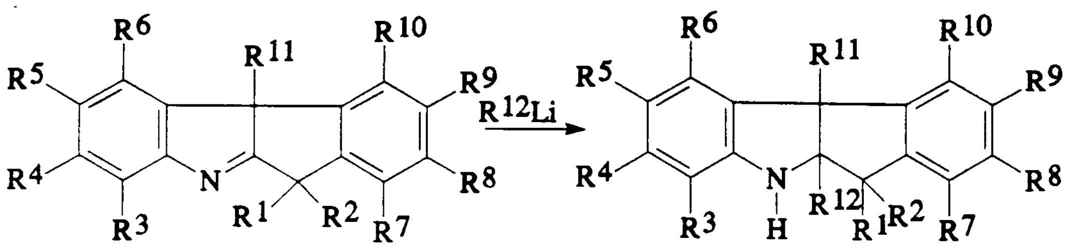


VIII IB

worin $R^1$, $R^2$, $R^3$, $R^4$, $R^5$, $R^6$, $R^7$, $R^8$, $R^9$, $R^{10}$, $R^{11}$ und $R^{12}$ wie in Formel IB definiert sind oder wobei, wenn geeignet, eine N-Alkylierung mit einem R-halid oder R-sulfat folgt, worin R wie in Formel IB definiert ist.

g. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch N-Alkylierung des THII bzw. iso-THII mit R-halid oder R-sulfat.

h. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch Reduktion des 5-Acyl-THII bzw. 6-Acyl-iso-THII.

i. zum Erhalt von THII oder iso-THII, worin $R^3$ - $R^6$ und/oder $R^7$ - $R^{10}$ als Mono- oder Di($C_1$-$C_6$-alkyl)-aminogruppe definiert sind und R, $R^1$, $R^2$, $R^{11}$ und $R^{12}$ wie in Formel I definiert sind, durch Reduktion der Nitrokomponenten von 5-Acyl-THII bzw. 6-Acyl-iso-THII, gefolgt von N-Alkylierung und wahlweise von Säurehydrolyse.

j. zum Erhalt von THII oder iso-THII, worin $R^3$ - $R^6$ und/oder $R^7$ - $R^{10}$ als Hydroxyl definiert sind und R, $R^1$, $R^2$, $R^{11}$ und $R^{12}$ wie in Formel I definiert sind, durch Etherdesalkylierung der Alkoxy-substituierten THII- bzw. iso-THII-Verbindungen.

k. zum Erhalt von THII oder iso-THII, worin $R^{12}$ eine niedere Alkylgruppe ist und R, $R^1$ - $R^{11}$ wie in Formel I definiert sind, durch eine Metallierungsabfolge am entsprechenden 4b-unsubstituierten Analog, gefolgt von Alkylierung mit $R^{12}$-halid oder $R^{12}$-sulfat und einer abschließenden Hydrolyse.

**24.** Zusammensetzung, umfassend eine Verbindung, die empfindlich gegenüber oxidativem Verfall ist, und eine Verbindung der Formel IA oder IB

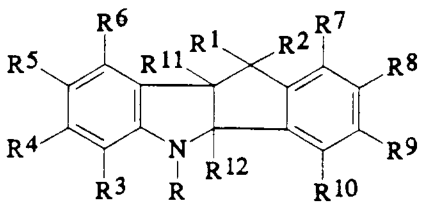
IA oder
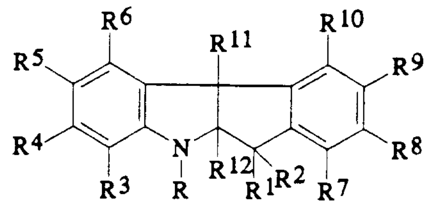
IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer niederen Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ ein Hydroxyl oder eine Mono- oder Di-($C_1$-$C_6$-Alkyl)-aminogruppe ist,
und Enantiomere und Salze davon.

**25.** Zusammensetzung nach Anspruch 24, worin die Verbindung der Formel IA oder IB wie in jedem der Ansprüche 1-6 definiert ist.

**26.** Zusammensetzung, umfassend eine Verbindung, die empfindlich gegenüber oxidativem Verfall ist, und cis-4b,5,9b,10-Tetrahydro-8-methoxy-6-methylindeno-[1,2-b]-indol oder ein Enantiomer oder Salz davon.

**27.** Zusammensetzung, umfassend eine Verbindung, die empfindlich gegenüber oxidativem Verfall ist, und cis-5,5a,6,10b-Tetrahydro-9-methoxy-7-methylindeno-[2,1-b]-indol oder ein Enantiomer oder Salz davon.

**28.** Nicht-medizinisches Verfahren zur Stabilisierung einer Verbindung, die empflindlich gegenüber oxidativem Verfall ist, indem die empfindliche Verbindung mit einer Verbindung der Formel IA oder IB, wie in Anspruch 24 oder 25 definiert, zusammengebracht wird.

**29.** Nicht-medizinisches Verfahren zur Stabilisierung einer Verbindung, die empfindlich gegenüber oxidativem Verfall ist, indem die empfindliche Verbindung mit cis-4b,5,9b,10-Tetrahydro-8-methoxy-6-methylindeno-[1,2-b]-indol oder einem Enantiomer oder Salz davon zusammengebracht wird.

**30.** Nicht-medizinisches Verfahren zur Stabilisierung einer Verbindung, die empfindlich gegenüber oxidativem Verfall ist, indem die empfindliche Verbindung mit cis-5,5a,6,10b-Tetrahydro-9-methoxy-7-methylindeno-[2,1-b]-indol oder einem Enantiomer oder Salz davon zusammengebracht wird.

**31.** Verfahren zur Haltbarmachung eines Produkts, das empfindlich gegenüber oxidativem Verfall ist, indem das empfindliche Produkt mit einer Verbindung der Formel IA oder IB, wie in Anspruch 24 oder 25 definiert, zusammengebracht wird.

**32.** Verfahren zur Haltbarmachung eines Produkts, das empfindlich gegenüber oxidativem Verfall ist, indem das empfindliche Produkt mit cis-4b,5,9b,10-Tetrahydro-8-methoxy-6-methylindeno-[1,2-b]-indol oder einem Enantiomer oder Salz davon zusammengebracht wird.

**33.** Verfahren zur Haltbarmachung eines Produkts, das empfindlich gegenüber oxidativem Verfall ist, indem das empfindliche Produkt mit cis-5,5a,6,10b-Tetrahydro-9-methoxy-7-methylindeno-[2,1-b]-indol oder einem Enantiomer oder Salz davon zusammengebracht wird.

**34.** Verwendung einer Verbindung der Formel IA oder IB, wie in Anspruch 24 oder 25 definiert, als ein Mittel zur Haltbarmachung eines Produkts, das empfindlich gegenüber oxidativem Verfall ist.

**35.** Verwendung von cis-4b,5,9b,10-Tetrahydro-8-methoxy-6-methylindeno-[1,2-b]-indol oder einem Enantiomer oder Salz davon, als ein Mittel zur Haltbarmachung eines Produkts, das empfindlich gegenüber oxidativem Verfall ist.

**36.** Verwendung von cis-5,5a,6,10b-Tetrahydro-9-methoxy-7-methylindeno-[2,1-b]-indol oder einem Enantiomer oder Salz davon, als ein Mittel zur Haltbarmachung eines Produkts, das empfindlich gegenüber oxidativem Verfall ist.

**Patentansprüche für folgenden Vertragsstaat : ES**

**1.** Verfahren zur Herstellung einer Verbindung der Formel IA oder IB

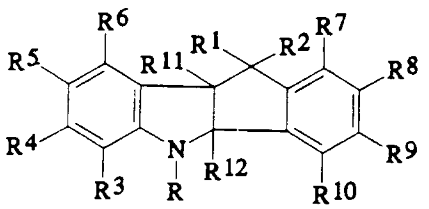
IA oder
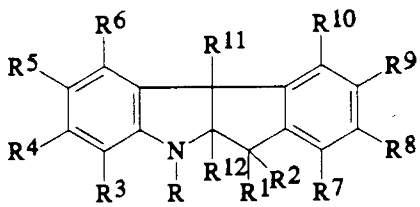
IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe oder einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter den Bedingungen, daß dann,

i, wenn R in Formel IA Methyl ist, mindestens eines der Radikale $R^1$ bis $R^{12}$ kein Wasserstoff ist,

ii, wenn R Wasserstoff oder Acetyl und $R^{11}$ Ethyl in Formel IA ist, mindestens eines der Radikale $R^1$ bis $R^{10}$ oder $R^{12}$ kein Wasserstoff ist;

oder ein Enantiomer oder Salz davon, welches Verfahren umfaßt

a. Reduktion von 5,10-Dihydroindeno-[1,2-b]-indol (DHII)

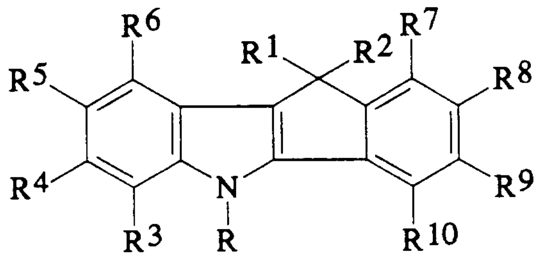

XI

worin R, $R^1$, $R^2$, $R^3$, $R^4$, $R^5$, $R^6$, $R^7$, $R^8$, $R^9$ und $R^{10}$ wie in Formel IA definiert sind, wobei, wenn geeignet eine N-Alkylierung des iso-DHII der Reduktion vorausgeht oder eine N-Alkylierung des iso-THII mit R-halid oder R-sulfat folgt, worin R wie in Formel I definiert ist.

b. Fischer-Indolisation eines Phenylhydrazins der Formel II und eines 2-substituierten-1-Indanons der Formel III, gefolgt von einer Reduktion der Indolenine (IV)

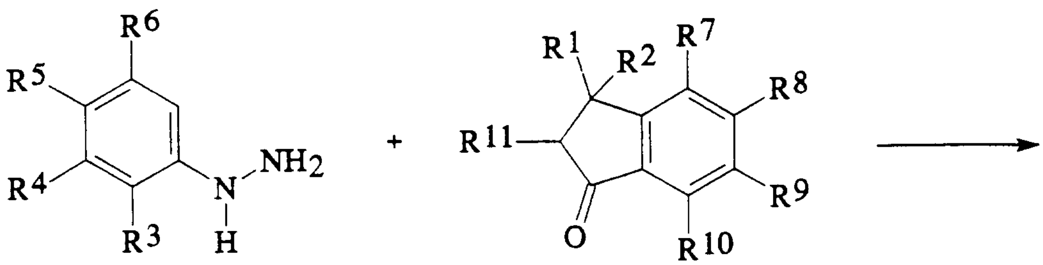

II

III

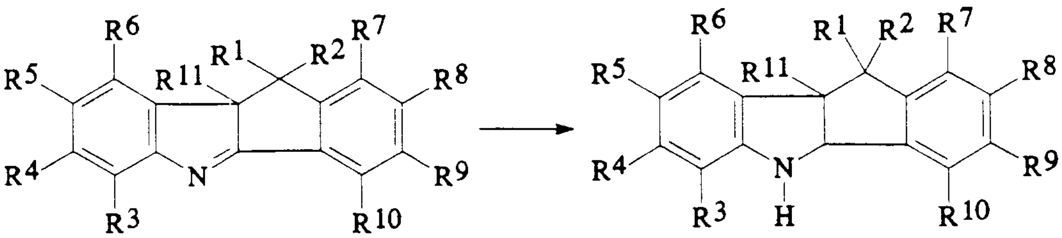

IV

V

worin $R^1$, $R^2$, $R^3$, $R^4$, $R^5$, $R^6$, $R^7$, $R^8$, $R^9$, $R^{10}$ und $R^{11}$ wie in Formel IA definiert sind und wobei, wenn geeignet, eine N-Alkylierung mit einem R-halid oder R-sulfat folgt, worin R wie in Formel IA definiert ist.

c. Reaktion von Indoleninen der Formel IV mit Lithiumalkylen ($R^{12}$-Li)

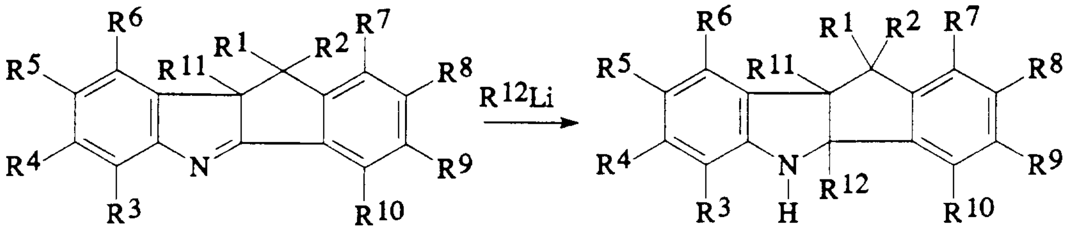


IV VI

worin $R^1$, $R^2$, $R^3$, $R^4$, $R^5$, $R^6$, $R^7$, $R^8$, $R^9$, $R^{10}$, $R^{11}$ und $R^{12}$ wie in Formel IA definiert sind und wobei, wenn geeignet, eine N-Alkylierung mit einem R-halid oder R-sulfat folgt, worin R wie in Formel IA definiert ist.

d. Reduktion von 5,6-Dihydroindeno-[2,1-b]-indol (iso-DHII), wobei, wenn geeignet, eine N-Alkylierung des iso-DHII der Reduktion vorausgeht oder eine N-Alkylierung des iso-THII mit R-halid oder R-sulfat folgt, worin R wie in Formel I definiert ist.

e. zum Erhalt von 10b-substituierten-5,5a,6,10b-Tetrahydroindeno-[2,1-b]-indolen (IX) und Analogen, unter Verwendung von Indan-2-onen (XII), die eine Substitutionsgruppe bei C-3 aufweisen, bei Fischer-Indolisation mit Phenylhydrazinen der Formel (II), gefolgt von einer Reaktion der Zwischenstufe VIII mit einem Reduktionsmittel.

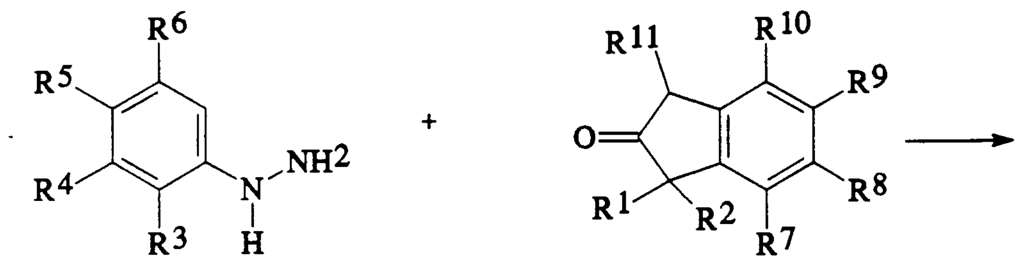

II XII

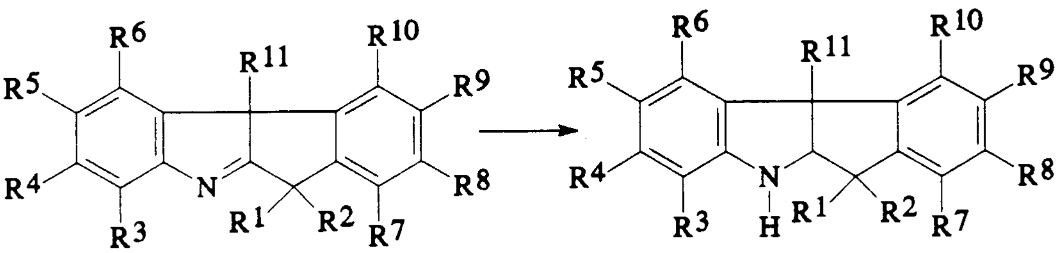

VIII IX

worin $R^1$, $R^2$, $R^3$, $R^4$, $R^5$, $R^6$, $R^7$, $R^8$, $R^9$, $R^{10}$ und $R^{11}$ wie in Formel IB definiert sind oder wobei, wenn geeignet, eine N-Alkylierung mit R-halid oder R-sulfat folgt, worin R wie in Formel IB definiert ist.

f. zum Erhalt von 5a,10b-substituierten-5,5a,6,10b-Tetrahydroindeno-[2,1-b]-indolen und Analogen durch Reaktion von Indoleninen der Formel VIII mit Lithiumalkylen $R^{12}Li$

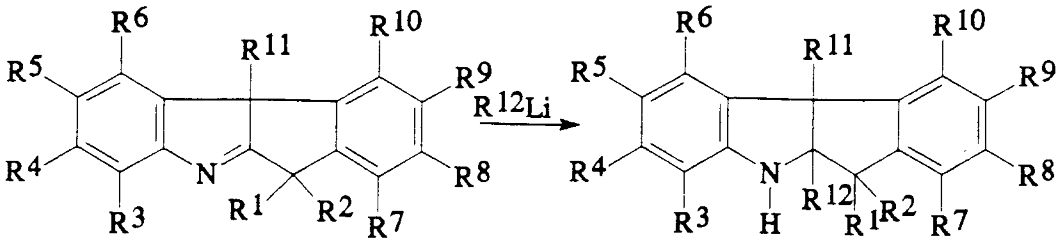

VIII

IB

worin $R^1$, $R^2$, $R^3$, $R^4$, $R^5$, $R^6$, $R^7$, $R^8$, $R^9$, $R^{10}$, $R^{11}$ und $R^{12}$ wie in Formel IB definiert sind oder wobei, wenn geeignet, eine N-Alkylierung mit R-halid oder R-sulfat folgt, worin R wie in Formel IB definiert ist.

g. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch N-Alkylierung des THII bzw. iso-THII mit R-halid oder R-sulfat.

h. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch Reduktion des 5-Acyl-THII bzw. 6-Acyl-iso-THII.

i. zum Erhalt von THII oder iso-THII, worin $R^3$ - $R^6$ und/oder $R^7$ - $R^{10}$ als eine Mono- oder Di($C_1$-$C_6$-alkyl)-aminogruppe definiert und R, $R^1$, $R^2$, $R^{11}$ und $R^{12}$ wie in Formel I definiert sind, durch Reduktion der Nitrokomponenten von 5-Acyl-THII bzw. 6-Acyl-iso-THII, gefolgt von N-Alkylierung und wahlweise von Säurehydrolyse.

j. zum Erhalt von THII oder iso-THII, worin $R^3$ - $R^6$ und/oder $R^7$ - $R^{10}$ als Hydroxyl definiert sind und R, $R^1$, $R^2$, $R^{11}$ und $R^{12}$ wie in Formel I definiert sind, durch Etherdesalkylierung der Alkoxy-substituierten THII- bzw. iso-THII-Verbindungen.

k. zum Erhalt von THII oder iso-THII, worin $R^{12}$ eine niedere Alkylgruppe ist und R, $R^1$ - $R^{11}$ wie in Formel I definiert sind, durch eine Metallierungsabfolge am entsprechenden 4b-unsubstituierten Analog, gefolgt von Alkylierung mit $R^{12}$-halid oder $R^{12}$-sulfat und einer abschließenden Hydrolyse.

2. Verfahren nach Anspruch 1, worin mindestens eines von $R^3$, $R^5$, $R^7$, $R^9$, $R^{11}$ und $R^{12}$ eine $C_1$-$C_6$-Alkylgruppe, bevorzugt Methyl, Ethyl oder i-Propyl, ist.

3. Verfahren nach Anspruch 1 oder 2, worin mindestens eines von $R^5$ und $R^8$ eine Mono- oder Di-($C_1$-$C_6$-alkyl)-aminogruppe, bevorzugt Ethyl- oder Diethylamino, ist.

4. Verfahren nach Anspruch 1 oder 2, worin mindestens eines von $R^5$ und $R^8$ eine Hydroxyl- oder eine $C_1$-$C_6$-Alkoxygruppe, bevorzugt Methoxy, ist.

5. Verfahren nach einem der vorangegangenen Ansprüche, worin R, $R^1$, $R^2$, $R^4$, $R^6$ und $R^{10}$ Wasserstoff sind.

6. Verfahren nach Anspruch 1, bei welchem erhalten wird:
cis-4b,5,9b,10-Tetrahydroindeno-[1,2-b]-indol,
cis-4b,5,9b,10-Tetrahydro-6,8-dimethylindeno-[1,2-b]-indol,
cis-4b,5,9b,10-Tetrahydro-5,8-dimethylindeno-[1,2-b]-indol,
cis-4b,5,9b,10-Tetrahydro-8-methylindeno-[1,2-b]-indol,
cis-4b,5,9b,10-Tetrahydro-4b,6,8,9b,tetramethylindeno-[1,2-b]-indol,
cis-4b,5,9b,10-Tetrahydro-8-isopropylindeno-[1,2-b]-indol,

cis-4b,5,9b,10-Tetrahydro-8-methoxy-5-methylindeno-[1,2-b]-indol,
cis-4b,5,9b,10-Tetrahydro-8-methoxyindeno-[1,2-b]-indol,
cis-4b,5,9b,10-Tetrahydro-10,10-dimethylindeno-[1,2-b]-indol,
cis-4b,5,9b,10-Tetrahydro-9b-methylindeno-[1,2-b]-indol,
cis-4b,5,9b,10-Tetrahydro-4b,9b-dimethylindeno-[1,2-b]-indol,
cis-4b,5,9b,10-Tetrahydro-4b,5,9b,Trimethylindeno-[1,2-b]-indol,
cis-4b,5,9b,10-Tetrahydro-2-methoxy-1,3-dimethylindeno-[1,2-b]-indol,
cis-4b,5,9b,10-Tetrahydro-2-methoxy-1,3-dimethyl-8-isopropylindeno-[1,2-b]-indol,
cis-4b,5,9b,10-Tetrahydro-4b-methylindeno-[1,2-b]-indol,
cis-4b,5,9b,10-Tetrahydro-2-hydroxy-1,3-dimethyl-8-isopropylindeno-[1,2-b]-indol,
cis-4b,5,9b,10-Tetrahydro-2-hydroxy-1,3-dimethylindeno-[1,2-b]-indol,
cis-4b,5,9b,10-Tetrahydro-4b,8,9b-trimethylindeno-[1,2-b]-indol,
cis-4b,5,9b,10-Tetrahydro-8-isopropyl-4b,9b-dimethylindeno-[1,2-b]-indol,
cis-4b,5,9b,10-Tetrahydro-8-isopropyl-4b-methylindeno-[1,2-b]-indol,
cis-4b,5,9b,10-Tetrahydro-2,8-dimethoxy-1,3-dimethylindeno-[1,2-b]-indol,
cis-4b,5,9b,10-Tetrahydro-4b,5,8,9b-tetramethylindeno-[1,2-b]-indol,
cis-4b,5,9b,10-Tetrahydro-8-tert.-Butylindeno-[1,2-b]-indol,
cis-4b,5,9b,10-Tetrahydro-8-methoxy-7,9-dimethylindeno-[1,2-b]-indol,
cis-4b,5,9b,10-Tetrahydro-8-methoxy-6-methylindeno-[1,2-b]-indol,
cis-4b,5,9b,10-Tetrahydro-8-diethylamino-5-ethylindeno-[1,2-b]-indol,
cis-4b,5,9b,10-Tetrahydro-2-diethylaminoindeno-[1,2-b]-indol,
cis-4b,5,9b,10-Tetrahydro-8-tert.-Butyl-4b-methylindeno-[1,2-b]-indol,
cis-4b,5,9b,10-Tetrahydro-8-fluoroindeno-[1,2-b]-indol,
cis-5,5a,6,10b-Tetrahydroindeno-[2,1-b]-indol,
cis-5,5a,6,10b-Tetrahydro-9-methoxyindeno-[2,1-b]-indol,
cis-5,5a,6,10b-Tetrahydro-9-isopropylindeno-[2,1-b]-indol,
cis-5,5a,6,10b-Tetrahydro-9-methoxy-7-methylindeno-[2,1-b]-indol,
cis-4b,5,9b,10-Tetrahydro-4,6-dimethyl-8-methoxyindeno-[1,2-b]-indol,
cis-4b,5,9b,10-Tetrahydro-8-methoxy-4b,6,9b-trimethylindeno-[1,2-b]-indol.

7. Verfahren nach Anspruch 1, bei welchem cis-4b,5,9b,10-Tetrahydro-8-methoxy-6-methylindeno-[1,2-b]-indol oder ein Enantiomer oder Salz davon erhalten wird.

8. Verfahren nach Anspruch 1, bei welchem cis-5,6a,6,10b-Tetrahydro-9-methoxy-7-methylindeno-[2,1-b]-indol oder ein Enantiomer oder Salz davon erhalten wird.

9. Verfahren zur Herstellung einer Verbindung der Formel IA oder IB

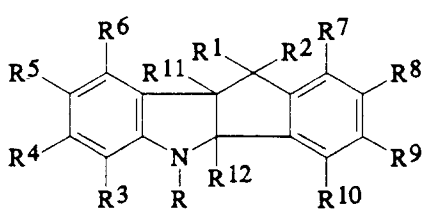

IA oder

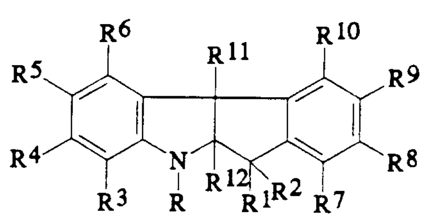

IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ Hydroxyl oder eine Mono- oder Di-($C_1$-$C_6$-alkyl)-aminogruppe ist,
oder ein Enantiomer oder pharmazeutisch geeignetes Salz davon; zur Verwendung bei einer Therapiemethode, die am menschlichen oder tierischen Körper Anwendung findet, welches Verfahren umfaßt

a. Reduktion von 5,10-Dihydroindeno-[1,2-b]-indol (DHII)

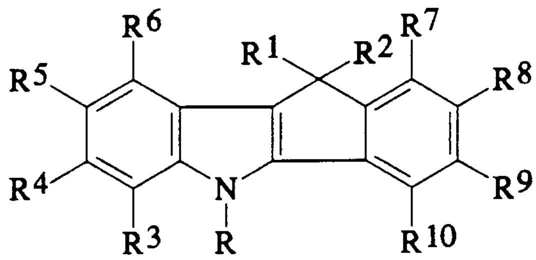

XI

worin R, $R^1$, $R^2$, $R^3$, $R^4$, $R^5$, $R^6$, $R^7$, $R^8$, $R^9$ und $R^{10}$ wie in Formel IA definiert sind, wobei, wenn geeignet eine N-Alkylierung des iso-DHII der Reduktion vorausgeht oder eine N-Alkylierung des iso-THII mit R-halid oder R-sulfat folgt, worin R wie in Formel I definiert ist.

b. Fischer-Indolisation eines Phenylhydrazins der Formel II und eines 2-substituierten-1-Indanons der Formel III, gefolgt von einer Reduktion der Indolenine (IV)

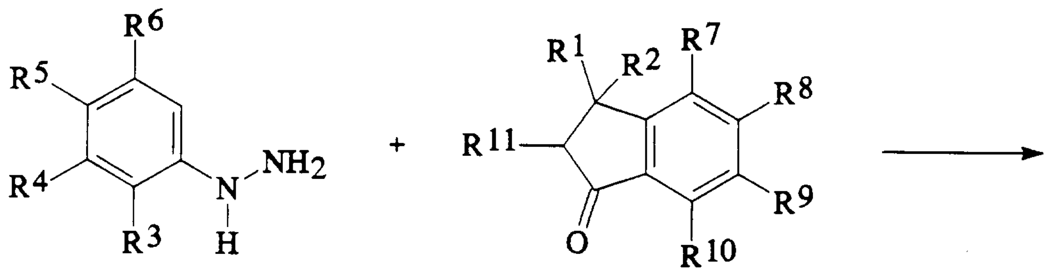

II III

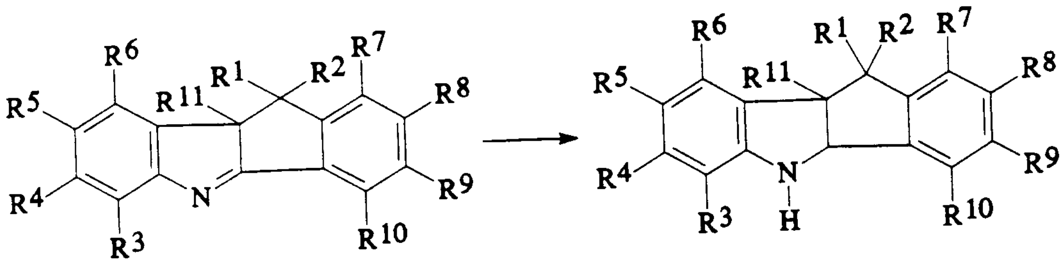

IV V

worin $R^1$, $R^2$, $R^3$, $R^4$, $R^5$, $R^6$, $R^7$, $R^8$, $R^9$, $R^{10}$ und $R^{11}$ wie in Formel IA definiert sind und wobei, wenn geeignet, eine N-Alkylierung mit einem R-halid oder R-sulfat folgt, worin R wie in Formel IA definiert ist.

c. Reaktion von Indoleninen der Formel IV mit Lithiumalkylen ($R^{12}$-Li)

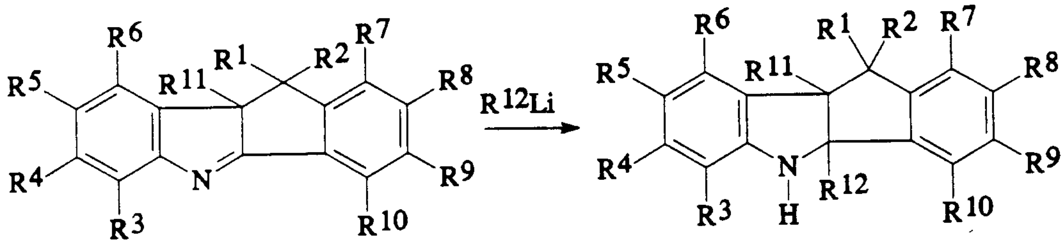


IV VI

worin $R^1$, $R^2$, $R^3$, $R^4$, $R^5$, $R^6$, $R^7$, $R^8$, $R^9$, $R^{10}$, $R^{11}$ und $R^{12}$ wie in Formel IA definiert sind und wobei, wenn geeignet, eine N-Alkylierung mit einem R-halid oder R-sulfat folgt, worin R wie in Formel IA definiert ist.

d. Reduktion von 5,6-Dihydroindeno-[2,1-b]-indol (iso-DHII), wobei, wenn geeignet, eine N-Alkylierung des iso-DHII der Reduktion vorausgeht oder eine N-Alkylierung des iso-THII mit R-halid oder R-sulfat folgt, worin R wie in Formel I definiert ist.

e. zum Erhalt von 10b-substituierten-5,5a,6,10b-Tetrahydroindeno-[2,1-b]-indolen (IX) und Analogen, unter Verwendung von Indan-2-onen (XII), die eine Substitutionsgruppe bei C-3 aufweisen, bei Fischer-Indolisation mit Phenylhydrazinen der Formel (II), gefolgt von einer Reaktion der Zwischenstufe VIII mit einem Reduktionsmittel.

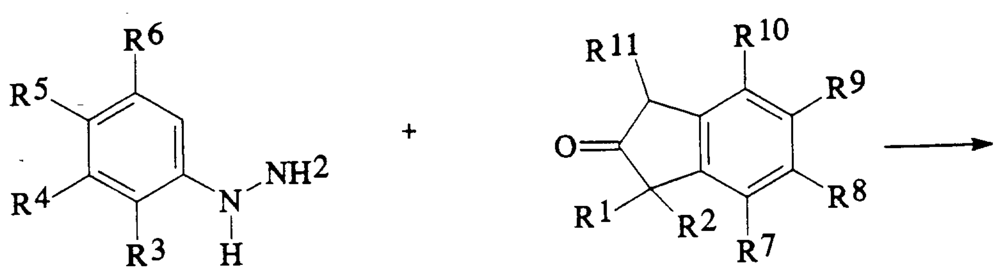

II XII

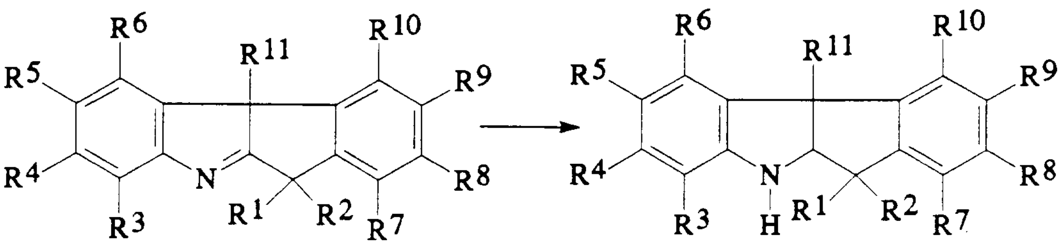

VIII IX

worin $R^1$, $R^2$, $R^3$, $R^4$, $R^5$, $R^6$, $R^7$, $R^8$, $R^9$, $R^{10}$ und $R^{11}$ wie in Formel IB definiert sind oder wobei, wenn geeignet, eine N-Alkylierung mit einem R-halid oder R-sulfat folgt, worin R wie in Formel IB definiert ist.

f. zum Erhalt von 5a,10b-substituierten-5,5a,6,10b-Tetrahydroindeno-[2,1-b]-indolen und Analogen durch Reaktion von Indoleninen der Formel VIII mit Lithiumalkylen $R^{12}Li$

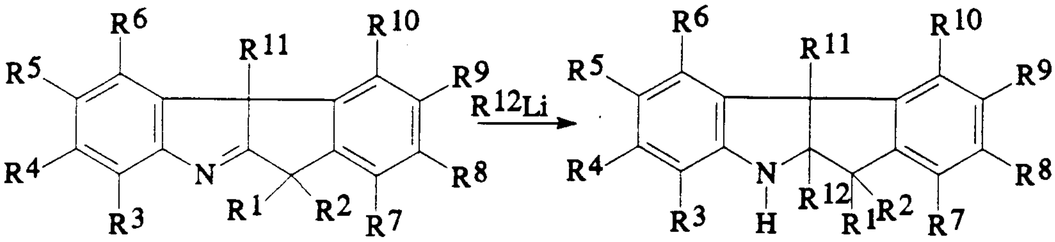


VIII

IB

worin $R^1$, $R^2$, $R^3$, $R^4$, $R^5$, $R^6$, $R^7$, $R^8$, $R^9$, $R^{10}$, $R^{11}$ und $R^{12}$ wie in Formel IB definiert sind oder wobei, wenn geeignet, eine N-Alkylierung mit einem R-halid oder R-sulfat folgt, worin R wie in Formel IB definiert ist.

g. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch N-Alkylierung des THII bzw. iso-THII mit R-halid oder R-sulfat.

h. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch Reduktion des 5-Acyl-THII bzw. 6-Acyl-iso-THII.

i. zum Erhalt von THII oder iso-THII, worin $R^3$ - $R^6$ und/oder $R^7$ - $R^{10}$ als Mono- oder Di($C_1$-$C_6$-alkyl)-aminogruppe definiert sind und R, $R^1$, $R^2$, $R^{11}$ und $R^{12}$ wie in Formel I definiert sind, durch Reduktion der Nitrokomponenten von 5-Acyl-THII bzw. 6-Acyl-iso-THII, gefolgt von N-Alkylierung und wahlweise von Säurehydrolyse.

j. zum Erhalt von THII oder iso-THII, worin $R^3$ - $R^6$ und/oder $R^7$ - $R^{10}$ als Hydroxyl definiert sind und R, $R^1$, $R^2$, $R^{11}$ und $R^{12}$ wie in Formel I definiert sind, durch Etherdesalkylierung der Alkoxy-substituierten THII- bzw. iso-THII-Verbindungen.

k. zum Erhalt von THII oder iso-THII, worin $R^{12}$ eine niedere Alkylgruppe ist und R, $R^1$ - $R^{11}$ wie in Formel I definiert sind, durch eine Metallierungsabfolge am entsprechenden 4b-unsubstituierten Analog, gefolgt von Alkylierung mit $R^{12}$-halid oder $R^{12}$-sulfat und einer abschließenden Hydrolyse.

**10.** Verfahren nach Anspruch 9, bei welchem cis-4b,5,9b,10-Tetrahydro-8-methoxy-6-methylindeno-[1,2-b]-indol oder ein Enantiomer oder pharmazeutisch geeignetes Salz davon erhalten wird, zur Verwendung bei einer Therapiemethode, die am menschlichen oder tierischen Körper Anwendung findet.

**11.** Verfahren nach Anspruch 9, bei welchem cis-5,5a,6,10b-Tetrahydro-9-methoxy-7-methylindeno-[2,1-b]-indol oder ein Enantiomer oder pharmazeutisch geeignetes Salz davon erhalten wird, zur Verwendung bei einer Therapiemethode, die am menschlichen oder tierischen Körper Anwendung findet.

**12.** Verfahren nach einem der Ansprüche 9 bis 11, bei welchem das Produkt zur Verwendung bei der Behandlung von ischämischen oder Reperfusions-Störungen, Thrombose und Embolie dient.

**13.** Verfahren nach einem der Ansprüche 9 bis 11, bei welchem das Produkt zur Verwendung bei der Behandlung oder Verhütung von Neoplasmen dient.

**14.** Verfahren nach einem der Ansprüche 9 bis 11, bei welchem das Produkt zur Verwendung bei der Behandlung von Parkinson-Krankheit, Alzheimer-Krankheit oder Alterserscheinungen dient.

**15.** Verfahren nach einem der Ansprüche 9 bis 11, bei welchem das Produkt zur Verwendung bei der Behandlung von Arteriosklerose dient.

**16.** Verfahren nach einem der Ansprüche 9 bis 11, bei welchem das Produkt zur Verwendung bei der Behandlung von allergischen/entzündlichen Zuständen, wie z.B. Bronchialasthma und rheumatische Arthritis, dient.

**17.** Verfahren nach einem der Ansprüche 9 bis 11, bei welchem das Produkt zur Verwendung bei der Behandlung von durch Chemikalien, Bestrahlung, antineoplastische oder immunsuppresive Mittel hervorgerufenen Schädigungen dient.

**18.** Verfahren nach einem der Ansprüche 9 bis 17, worin Formel IA oder IB wie in einem der Ansprüche 1-6 definiert ist.

**19.** Verfahren zur Herstellung einer pharmazeutischen Zusammensetzung, welches Verfahren das Mischen eines Wirkstoffs, welcher eine Verbindung wie in einem der Ansprüche 1-6 definiert, ein Enantiomer oder ein pharmazeutisch geeignetes Salz davon ist, mit einem pharmazeutisch geeigneten Träger umfaßt.

**20.** Verfahren zur Herstellung einer pharmazeutischen Zusammensetzung, welches Verfahren das Mischen eines Wirkstoffs, welcher cis-4b,5,9b,10-Tetrahydro-8-methoxy-6-methylindeno-[1,2-b]-indol oder ein Enantiomer oder pharmazeutisch geeignetes Salz davon ist, mit einem pharmazeutisch geeigneten Träger umfaßt.

**21.** Verfahren zur Herstellung einer pharmazeutischen Zusammensetzung, welches Verfahren das Mischen eines Wirkstoffs, welcher cis-5,5a,6,10b-Tetrahydro-9-methoxy-7-methylindeno-[2,1-b]-indol oder ein Enantiomer oder pharmazeutisch geeignetes Salz davon ist, mit einem pharmazeutisch geeigneten Träger umfaßt.

**22.** Verwendung einer Verbindung der Formel IA oder IB, wie in einem der Ansprüche 1 bis 9 definiert, bei der Herstellung eines Medikaments zur Behandlung von ischämischen oder Reperfusions-Störungen, Thrombose, Embolie, Arteriosklerose, Parkinson-Krankheit, Alzheimer-Krankheit, Alterserscheinungen, Neoplasmen, allergischen oder entzündlichen Zuständen, wie z.B. Bronchialasthma und rheumatische Arthritis, und durch Chemikalien, Bestrahlung, antineoplastische oder immunsuppresive Mitteln hervorgerufenen Schädigungen.

**23.** Nicht-medizinisches Verfahren zur Stabilisierung einer Verbindung, die empfindlich gegenüber oxidativem Verfall ist, indem die empfindliche Verbindung zusammengebracht wird mit einer Verbindung der Formel IA oder IB

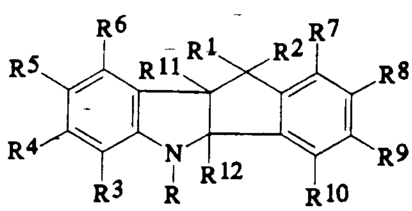

IA oder

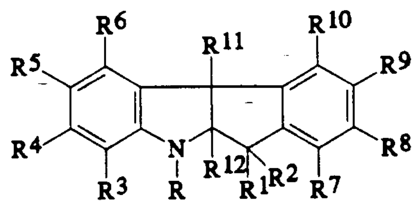

IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer niederen Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ ein Hydroxyl oder eine Mono- oder Di-($C_1$-$C_6$-Alkyl)-aminogruppe ist,
oder ein Enantiomer oder Salz davon.

**24.** Verfahren nach Anspruch 23, worin die Verbindung der Formel IA oder IB wie in jedem der Ansprüche 1-6 definiert ist.

**25.** Nicht-medizinisches Verfahren zur Stabilisierung einer Verbindung, die empfindlich gegenüber oxidativem Verfall ist, indem die empfindliche Verbindung mit cis-4b,5,9b,10-Tetrahydro-8-methoxy-6-methylindeno-[1,2-b]-indol oder einem Enantiomer oder Salz davon zusammengebracht wird.

26. Nicht-medizinisches Verfahren zur Stabilisierung einer Verbindung, die empfindlich gegenüber oxidativem Verfall ist, indem die empfindliche Verbindung mit cis-5,5a,6,10b-Tetrahydro-9-methoxy-7-methylindeno-[2,1-b]-indol oder einem Enantiomer oder Salz davon zusammengebracht wird.

27. Verfahren zur Haltbarmachung eines Produkts, das empfindlich gegenüber oxidativem Verfall ist, indem das empfindliche Produkt mit einer Verbindung der Formel IA oder IB, wie in Anspruch 23 oder 24 definiert, zusammengebracht wird.

28. Verfahren zur Haltbarmachung eines Produkts, das empfindlich gegenüber oxidativem Verfall ist, indem das empfindliche Produkt mit cis-4b,5,9b,10-Tetrahydro-8-methoxy-6-methylindeno-[1,2-b]-indol oder einem Enantiomer oder Salz davon zusammengebracht wird.

29. Verfahren zur Haltbarmachung eines Produkts, das empfindlich gegenüber oxidativem Verfall ist, indem das empfindliche Produkt mit cis-5,5a,6,10b-Tetrahydro-9-methoxy-7-methylindeno-[2,1-b]-indol oder einem Enantiomer oder Salz davon zusammengebracht wird.

30. Verwendung einer Verbindung der Formel IA oder IB, wie in Anspruch 23 oder 24 definiert, als ein Mittel zur Haltbarmachung eines Produkts, das empfindlich gegenüber oxidativem Verfall ist.

31. Verwendung von cis-4b,5,9b,10-Tetrahydro-8-methoxy-6-methylindeno-[1,2-b]-indol oder einem Enantiomer oder Salz davon, als ein Mittel zur Haltbarmachung eines Produkts, das empfindlich gegenüber oxidativem Verfall ist.

32. Verwendung von cis-5,5a,6,10b-Tetrahydro-9-methoxy-7-methylindeno-[2,1-b]-indol oder einem Enantiomer oder Salz davon, als ein Mittel zur Haltbarmachung eines Produkts, das empfindlich gegenüber oxidativem Verfall ist.

**Patentansprüche für folgenden Vertragsstaat : GR**

1. Verfahren zur Herstellung einer Verbindung der Formel IA oder IB

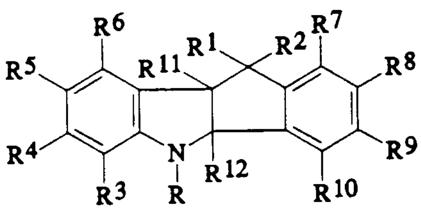 IA oder 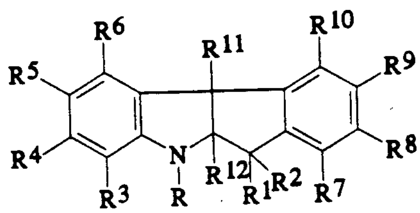 IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe oder einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter den Bedingungen, daß dann,

i, wenn R in Formel IA Methyl ist, mindestens eines der Radikale $R^1$ bis $R^{12}$ kein Wasserstoff ist,

ii, wenn R Wasserstoff oder Acetyl und $R^{11}$ Ethyl in Formel IA ist, mindestens eines der Radikale $R^1$ bis $R^{10}$ oder $R^{12}$ kein Wasserstoff ist;

oder ein Enantiomer oder Salz davon, welches Verfahren umfaßt

a. Reduktion von 5,10-Dihydroindeno-[1,2-b]-indol (DHII)

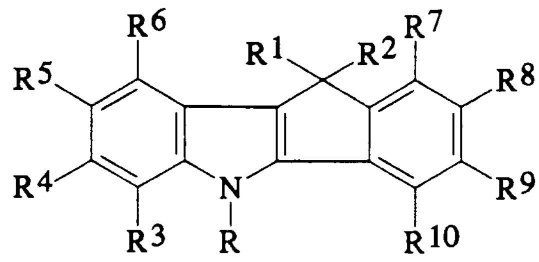

XI

worin R, $R^1$, $R^2$, $R^3$, $R^4$, $R^5$, $R^6$, $R^7$, $R^8$, $R^9$ und $R^{10}$ wie in Formel IA definiert sind, wobei, wenn geeignet eine N-Alkylierung des iso-DHII der Reduktion vorausgeht oder eine N-Alkylierung des iso-THII mit R-halid oder R-sulfat folgt, worin R wie in Formel I definiert ist.

b. Fischer-Indolisation eines Phenylhydrazins der Formel II und eines 2-substituierten-1-Indanons der Formel III, gefolgt von einer Reduktion der Indolenine (IV)

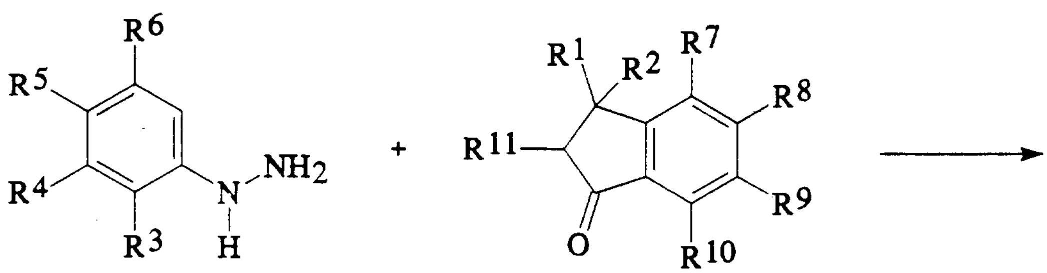

II

III

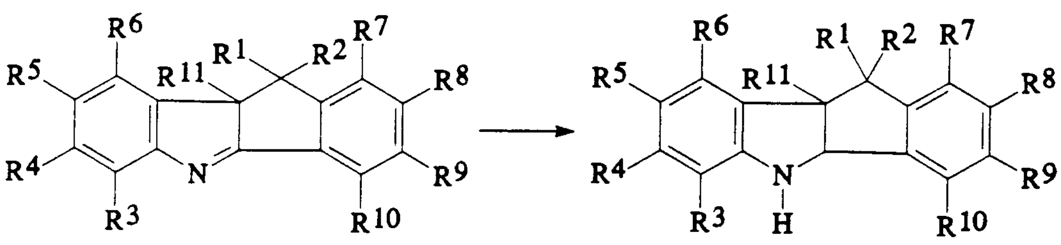

IV

V

worin $R^1$, $R^2$, $R^3$, $R^4$, $R^5$, $R^6$, $R^7$, $R^8$, $R^9$, $R^{10}$ und $R^{11}$ wie in Formel IA definiert sind und wobei, wenn geeignet, eine N-Alkylierung mit einem R-halid oder R-sulfat folgt, worin R wie in Formel IA definiert ist.

c. Reaktion von Indoleninen der Formel IV mit Lithiumalkylen ($R^{12}$-Li)

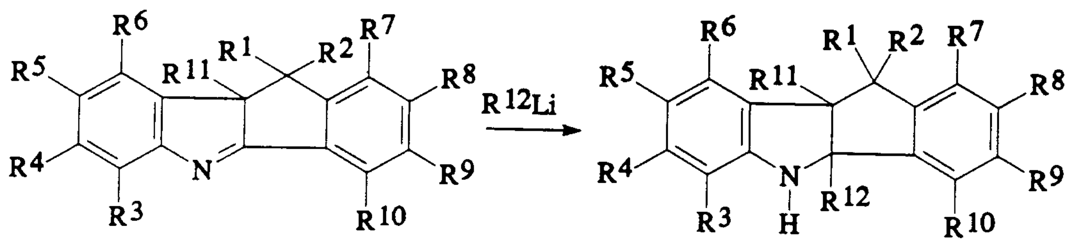


IV VI

worin $R^1$, $R^2$, $R^3$, $R^4$, $R^5$, $R^6$, $R^7$, $R^8$, $R^9$, $R^{10}$, $R^{11}$ und $R^{12}$ wie in Formel IA definiert sind und wobei, wenn geeignet, eine N-Alkylierung mit einem R-halid oder R-sulfat folgt, worin R wie in Formel IA definiert ist.

d. Reduktion von 5,6-Dihydroindeno-[2,1-b]-indol (iso-DHII), wobei, wenn geeignet, eine N-Alkylierung des iso-DHII der Reduktion vorausgeht oder eine N-Alkylierung des iso-THII mit R-halid oder R-sulfat folgt, worin R wie in Formel I definiert ist.

e. zum Erhalt von 10b-substituierten-5,5a,6,10b-Tetrahydroindeno-[2,1-b]-indolen (IX) und Analogen, unter Verwendung von Indan-2-onen (XII), die eine Substitutionsgruppe bei C-3 aufweisen, bei Fischer-Indolisation mit Phenylhydrazinen der Formel (II), gefolgt von einer Reaktion der Zwischenstufe VIII mit einem Reduktionsmittel.

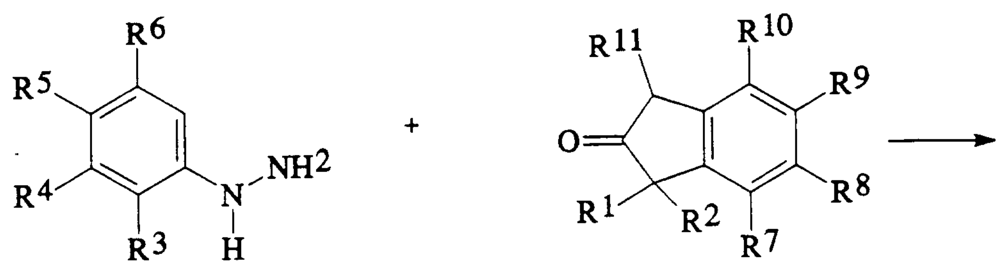

II XII

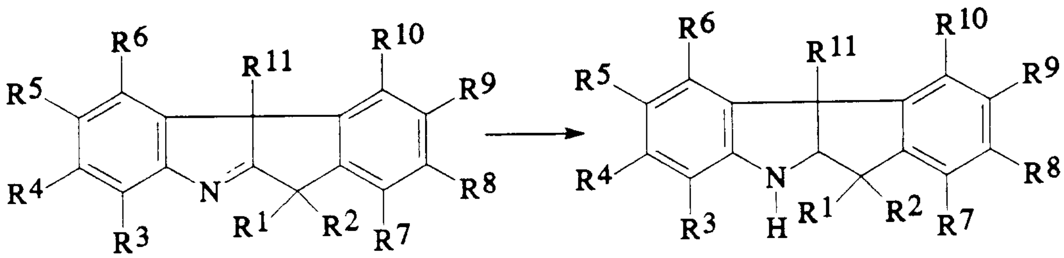

VIII IX

worin $R^1$, $R^2$, $R^3$, $R^4$, $R^5$, $R^6$, $R^7$, $R^8$, $R^9$, $R^{10}$ und $R^{11}$ wie in Formel IB definiert sind oder wobei, wenn geeignet, eine N-Alkylierung mit R-halid oder R-sulfat folgt, worin R wie in Formel IB definiert ist.

f. zum Erhalt von 5a,10b-substituierten-5,5a,6,10b-Tetrahydroindeno-[2,1-b]-indolen und Analogen durch Reaktion von Indoleninen der Formel VIII mit Lithiumalkylen $R^{12}Li$

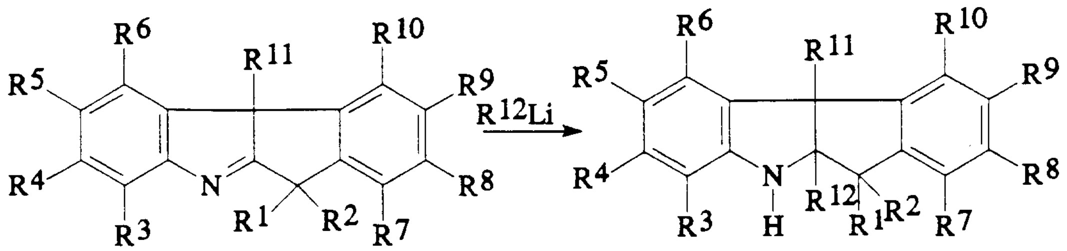


VIII IB

worin $R^1$, $R^2$, $R^3$, $R^4$, $R^5$, $R^6$, $R^7$, $R^8$, $R^9$, $R^{10}$, $R^{11}$ und $R^{12}$ wie in Formel IB definiert sind oder wobei, wenn geeignet, eine N-Alkylierung mit R-halid oder R-sulfat folgt, worin R wie in Formel IB definiert ist.

g. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch N-Alkylierung des THII bzw. iso-THII mit R-halid oder R-sulfat.

h. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch Reduktion des 5-Acyl-THII bzw. 6-Acyl-iso-THII.

i. zum Erhalt von THII oder iso-THII, worin $R^3$ - $R^6$ und/oder $R^7$ - $R^{10}$ als eine Mono- oder Di($C_1$-$C_6$-alkyl)-aminogruppe definiert und R, $R^1$, $R^2$, $R^{11}$ und $R^{12}$ wie in Formel I definiert sind, durch Reduktion der Nitrokomponenten des 5-Acyl-THII bzw. 6-Acyl-iso-THII, gefolgt von N-Alkylierung und wahlweise von Säurehydrolyse.

j. zum Erhalt von THII oder iso-THII, worin $R^3$ - $R^6$ und/oder $R^7$ - $R^{10}$ als Hydroxyl definiert sind und R, $R^1$, $R^2$, $R^{11}$ und $R^{12}$ wie in Formel I definiert sind, durch Etherdesalkylierung der Alkoxy-substituierten THII- bzw. iso-THII-Verbindungen.

k. zum Erhalt von THII oder iso-THII, worin $R^{12}$ eine niedere Alkylgruppe ist und R, $R^1$ - $R^{11}$ wie in Formel I definiert sind, durch eine Metallierungsabfolge am entsprechenden 4b-unsubstituierten Analog, gefolgt von Alkylierung mit $R^{12}$-halid oder $R^{12}$-sulfat und einer abschließenden Hydrolyse.

**2.** Verfahren nach Anspruch 1, worin mindestens eines von $R^3$, $R^5$, $R^7$, $R^9$, $R^{11}$ und $R^{12}$ eine $C_1$-$C_6$-Alkylgruppe, bevorzugt Methyl, Ethyl oder i-Propyl, ist.

**3.** Verfahren nach Anspruch 1 oder 2, worin mindestens eines von $R^5$ und $R^8$ eine Mono- oder Di-($C_1$-$C_6$-alkyl)-aminogruppe, bevorzugt Ethyl- oder Diethylamino, ist.

**4.** Verfahren nach Anspruch 1 oder 2, worin mindestens eines von $R^5$ und $R^8$ eine Hydroxyl- oder eine $C_1$-$C_6$-Alkoxygruppe, bevorzugt Methoxy, ist.

**5.** Verfahren nach einem der vorangegangenen Ansprüche, worin R, $R^1$, $R^2$, $R^4$, $R^6$ und $R^{10}$ Wasserstoff sind.

**6.** Verfahren nach Anspruch 1, bei welchem erhalten wird:
cis-4b,5,9b,10-Tetrahydroindeno-[1,2-b]-indol,
cis-4b,5,9b,10-Tetrahydro-6,8-dimethylindeno-[1,2-b]-indol,
cis-4b,5,9b,10-Tetrahydro-5,8-dimethylindeno-[1,2-b]-indol,
cis-4b,5,9b,10-Tetrahydro-8-methylindeno-[1,2-b]-indol,
cis-4b,5,9b,10-Tetrahydro-4b,6,8,9b,tetramethylindeno-[1,2-b]-indol,
cis-4b,5,9b,10-Tetrahydro-8-isopropylindeno-[1,2-b]-indol,

cis-4b,5,9b,10-Tetrahydro-8-methoxy-5-methylindeno-[1,2-b]-indol,
cis-4b,5,9b,10-Tetrahydro-8-methoxyindeno-[1,2-b]-indol,
cis-4b,5,9b,10-Tetrahydro-10,10-dimethylindeno-[1,2-b]-indol,
cis-4b,5,9b,10-Tetrahydro-9b-methylindeno-[1,2-b]-indol,
cis-4b,5,9b,10-Tetrahydro-4b,9b-dimethylindeno-[1,2-b]-indol,
cis-4b,5,9b,10-Tetrahydro-4b,5,9b,Trimethylindeno-[1,2-b]-indol,
cis-4b,5,9b,10-Tetrahydro-2-methoxy-1,3-dimethylindeno-[1,2-b]-indol,
cis-4b,5,9b,10-Tetrahydro-2-methoxy-1,3-dimethyl-8-isopropylindeno-[1,2-b]-indol,
cis-4b,5,9b,10-Tetrahydro-4b-methylindeno-[1,2-b]-indol,
cis-4b,5,9b,10-Tetrahydro-2-hydroxy-1,3-dimethyl-8-isopropylindeno-[1,2-b]-indol,
cis-4b,5,9b,10-Tetrahydro-2-hydroxy-1,3-dimethylindeno-[1,2-b]-indol,
cis-4b,5,9b,10-Tetrahydro-4b,8,9b-trimethylindeno-[1,2-b]-indol,
cis-4b,5,9b,10-Tetrahydro-8-isopropyl-4b,9b-dimethylindeno-[1,2-b]-indol,
cis-4b,5,9b,10-Tetrahydro-8-isopropyl-4b-methylindeno-[1,2-b]-indol,
cis-4b,5,9b,10-Tetrahydro-2,8-dimethoxy-1,3-dimethylindeno-[1,2-b]-indol,
cis-4b,5,9b,10-Tetrahydro-4b,5,8,9b-tetramethylindeno-[1,2-b]-indol,
cis-4b,5,9b,10-Tetrahydro-8-tert.-Butylindeno-[1,2-b]-indol,
cis-4b,5,9b,10-Tetrahydro-8-methoxy-7,9-dimethylindeno-[1,2-b]-indol,
cis-4b,5,9b,10-Tetrahydro-8-methoxy-6-methylindeno-[1,2-b]-indol,
cis-4b,5,9b,10-Tetrahydro-8-diethylamino-5-ethylindeno-[1,2-b]-indol,
cis-4b,5,9b,10-Tetrahydro-2-diethylaminoindeno-[1,2-b]-indol,
cis-4b,5,9b,10-Tetrahydro-8-tert.-Butyl-4b-methylindeno-[1,2-b]-indol,
cis-4b,5,9b,10-Tetrahydro-8-fluoroindeno-[1,2-b]-indol,
cis-5,5a,6,10b-Tetrahydroindeno-[2,1-b]-indol,
cis-5,5a,6,10b-Tetrahydro-9-methoxyindeno-[2,1-b]-indol,
cis-5,5a,6,10b-Tetrahydro-9-isopropylindeno-[2,1-b]-indol,
cis-5,5a,6,10b-Tetrahydro-9-methoxy-7-methylindeno-[2,1-b]-indol,
cis-4b,5,9b,10-Tetrahydro-4,6-dimethyl-8-methoxyindeno-[1,2-b]-indol,
cis-4b,5,9b,10-Tetrahydro-8-methoxy-4b,6,9b-trimethylindeno-[1,2-b]-indol.

7. Verfahren nach Anspruch 1, bei welchem cis-4b,5,9b,10-Tetrahydro-8-methoxy-6-methylindeno-[1,2-b]-indol oder ein Enantiomer oder Salz davon erhalten wird.

8. Verfahren nach Anspruch 1, bei welchem cis-5,5a,6,10b-Tetrahydro-9-methoxy-7-methylindeno-[2,1-b]-indol oder ein Enantiomer oder Salz davon erhalten wird.

9. Verfahren zur Herstellung einer Verbindung der Formel IA oder IB

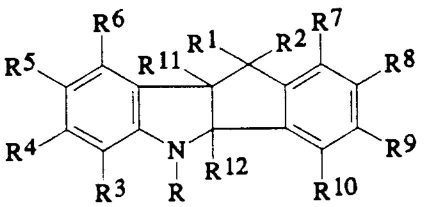 IA oder 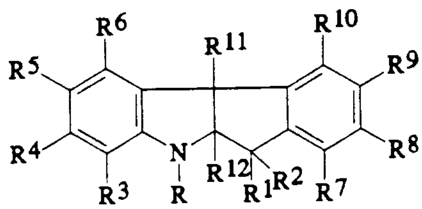 IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer $C_1$-$C_6$-Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ Hydroxyl oder eine Mono- oder Di-($C_1$-$C_6$-alkyl)-aminogruppe ist,

oder ein Enantiomer oder pharmazeutisch geeignetes Salz davon; zur Verwendung bei einer Therapiemethode, die am menschlichen oder tierischen Körper Anwendung findet, welches Verfahren umfaßt

a. Reduktion von 5,10-Dihydroindeno-[1,2-b]-indol (DHII)

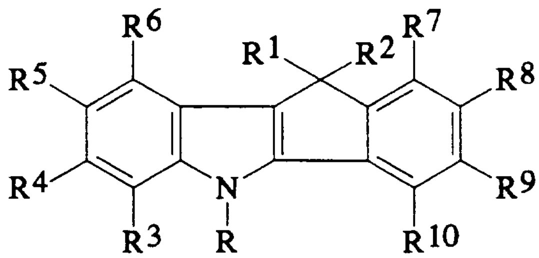

XI

worin R, $R^1$, $R^2$, $R^3$, $R^4$, $R^5$, $R^6$, $R^7$, $R^8$, $R^9$ und $R^{10}$ wie in Formel IA definiert sind, wobei, wenn geeignet eine N-Alkylierung des iso-DHII der Reduktion vorausgeht oder eine N-Alkylierung des iso-THII mit R-halid oder R-sulfat folgt, worin R wie in Formel I definiert ist.

b. Fischer-Indolisation eines Phenylhydrazins der Formel II und eines 2-substituierten-1-Indanons der Formel III, gefolgt von einer Reduktion der Indolenine (IV)

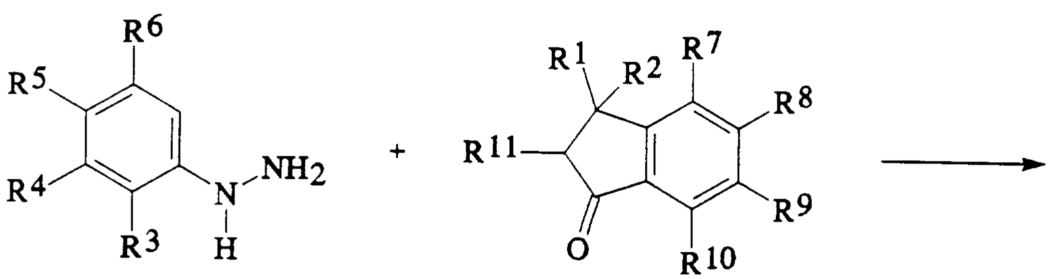

II

III

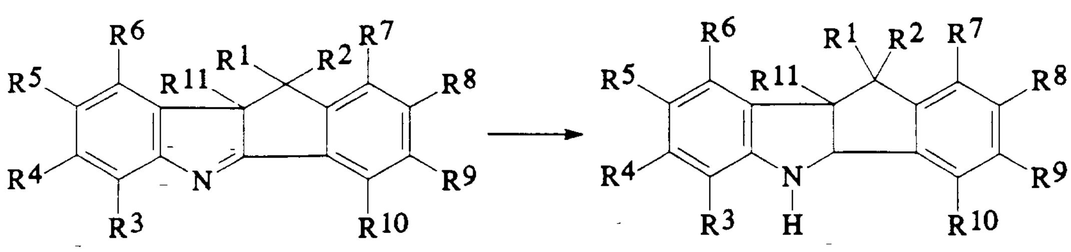

IV

V

worin $R^1$, $R^2$, $R^3$, $R^4$, $R^5$, $R^6$, $R^7$, $R^8$, $R^9$, $R^{10}$ und $R^{11}$ wie in Formel IA definiert sind und wobei, wenn geeignet, eine N-Alkylierung mit einem R-halid oder R-sulfat folgt, worin R wie in Formel IA definiert ist.

c. Reaktion von Indoleninen der Formel IV mit Lithiumalkylen ($R^{12}$-Li)

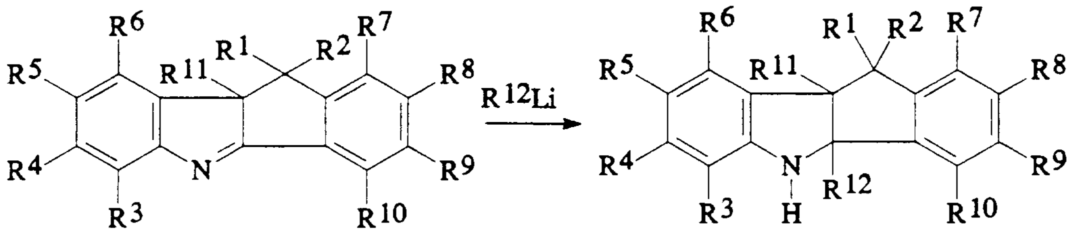


IV VI

worin $R^1$, $R^2$, $R^3$, $R^4$, $R^5$, $R^6$, $R^7$, $R^8$, $R^9$, $R^{10}$, $R^{11}$ und $R^{12}$ wie in Formel IA definiert sind und wobei, wenn geeignet, eine N-Alkylierung mit einem R-halid oder R-sulfat folgt, worin R wie in Formel IA definiert ist.

d. Reduktion von 5,6-Dihydroindeno-[2,1-b]-indol (iso-DHII), wobei, wenn geeignet, eine N-Alkylierung des iso-DHII der Reduktion vorausgeht oder eine N-Alkylierung des iso-THII mit R-halid oder R-sulfat folgt, worin R wie in Formel I definiert ist.

e. zum Erhalt von 10b-substituierten-5,5a,6,10b-Tetrahydroindeno-[2,1-b]-indolen (IX) und Analogen, unter Verwendung von Indan-2-onen (XII), die eine Substitutionsgruppe bei C-3 aufweisen, bei Fischer-Indolisation mit Phenylhydrazinen der Formel (II), gefolgt von einer Reaktion der Zwischenstufe VIII mit einem Reduktionsmittel.

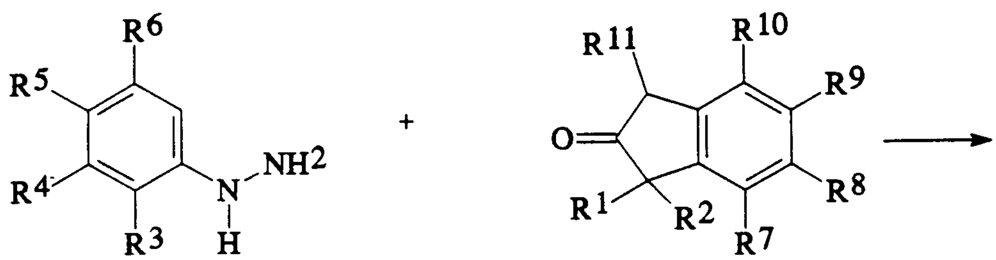

II XII

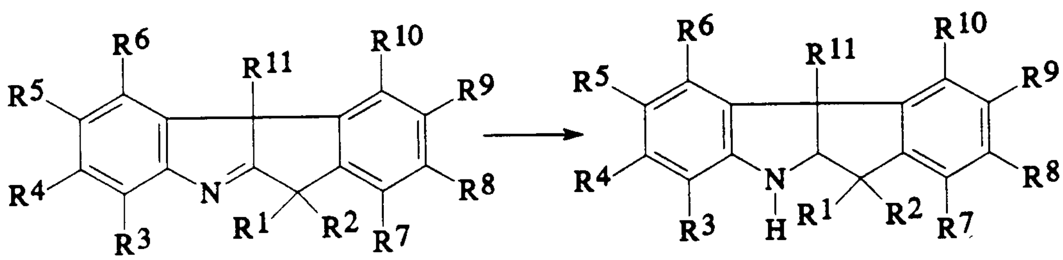

VIII IX

worin $R^1$, $R^2$, $R^3$, $R^4$, $R^5$, $R^6$, $R^7$, $R^8$, $R^9$, $R^{10}$ und $R^{11}$ wie in Formel IB definiert sind oder wobei, wenn geeignet, eine N-Alkylierung mit einem R-halid oder R-sulfat folgt, worin R wie in Formel IB definiert ist.

f. zum Erhalt von 5a,10b-substituierten-5,5a,6,10b-Tetrahydroindeno-[2,1-b]-indolen und Analogen durch Reaktion von Indoleninen der Formel VIII mit Lithiumalkylen $R^{12}Li$

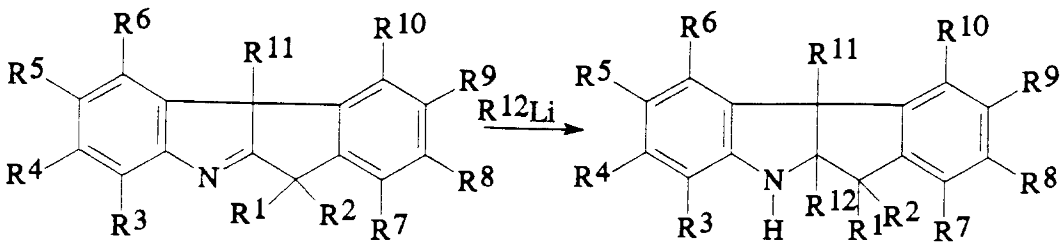


VIII

IB

worin $R^1$, $R^2$, $R^3$, $R^4$, $R^5$, $R^6$, $R^7$, $R^8$, $R^9$, $R^{10}$, $R^{11}$ und $R^{12}$ wie in Formel IB definiert sind oder wobei, wenn geeignet, eine N-Alkylierung mit einem R-halid oder R-sulfat folgt, worin R wie in Formel IB definiert ist.

g. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch N-Alkylierung des THII bzw. iso-THII mit R-halid oder R-sulfat.

h. zum Erhalt von 5-Alkyl-THII oder 6-Alkyl-iso-THII, worin R, $R^1$, $R^2$, $R^3$, $R^4$, $R^5$, $R^6$, $R^7$, $R^8$, $R^9$, $R^{10}$ wie in Formel I definiert sind, durch Reduktion des 5-Acyl-THII bzw. 6-Acyl-iso-THII.

i. zum Erhalt von THII oder iso-THII, worin $R^3$ - $R^6$ und/oder $R^7$ - $R^{10}$ als Mono- oder Di($C_1$-$C_6$-alkyl)-aminogruppe definiert sind und R, $R^1$, $R^2$, $R^{11}$ und $R^{12}$ wie in Formel I definiert sind, durch Reduktion der Nitrokomponenten des 5-Acyl-THII bzw. 6-Acyl-iso-THII, gefolgt von N-Alkylierung und wahlweise von Säurehydrolyse.

j. zum Erhalt von THII oder iso-THII, worin $R^3$ - $R^6$ und/oder $R^7$ - $R^{10}$ als Hydroxyl definiert sind und R, $R^1$, $R^2$, $R^{11}$ und $R^{12}$ wie in Formel I definiert sind, durch Etherdesalkylierung der Alkoxy-substituierten THII- bzw. iso-THII-Verbindungen.

k. zum Erhalt von THII oder iso-THII, worin $R^{12}$ eine niedere Alkylgruppe ist und R, $R^1$ - $R^{11}$ wie in Formel I definiert sind, durch eine Metallierungsabfolge am entsprechenden 4b-unsubstituierten Analog, gefolgt von Alkylierung mit $R^{12}$-halid oder $R^{12}$-sulfat und einer abschließenden Hydrolyse.

**10.** Verfahren nach Anspruch 9, bei welchem cis-4b,5,9b,10-Tetrahydro-8-methoxy-6-methylindeno-[1,2-b]-indol oder ein Enantiomer oder pharmazeutisch geeignetes Salz davon erhalten wird, zur Verwendung bei einer Therapiemethode, die am menschlichen oder tierischen Körper Anwendung findet.

**11.** Verfahren nach Anspruch 9, bei welchem cis-5,5a,6,10b-Tetrahydro-9-methoxy-7-methylindeno-[2,1-b]-indol oder ein Enantiomer oder pharmazeutisch geeignetes Salz davon erhalten wird, zur Verwendung bei einer Therapiemethode, die am menschlichen oder tierischen Körper Anwendung findet.

**12.** Verfahren nach einem der Ansprüche 9 bis 11, bei welchem das Produkt zur Verwendung bei der Behandlung von ischämischen oder Reperfusions-Störungen, Thrombose und Embolie dient.

**13.** Verfahren nach einem der Ansprüche 9 bis 11, bei welchem das Produkt zur Verwendung bei der Behandlung oder Verhütung von Neoplasmen dient.

**14.** Verfahren nach einem der Ansprüche 9 bis 11, bei welchem das Produkt zur Verwendung bei der Behandlung von Parkinson-Krankheit, Alzheimer-Krankheit oder Alterserscheinungen dient.

**15.** Verfahren nach einem der Ansprüche 9 bis 11, bei welchem das Produkt zur Verwendung bei der Behandlung von Arteriosklerose dient.

**16.** Verfahren nach einem der Ansprüche 9 bis 11, bei welchem das Produkt zur Verwendung bei der Behandlung von allergischen/entzündlichen Zuständen, wie z.B. Bronchialasthma und rheumatische Arthritis, dient.

**17.** Verfahren nach einem der Ansprüche 9 bis 11, bei welchem das Produkt zur Verwendung bei der Behandlung von durch Chemikalien, Bestrahlung, antineoplastische oder immunsuppresive Mittel hervorgerufenen Schädigungen dient.

**18.** Verfahren nach einem der Ansprüche 9 bis 17, worin Formel IA oder IB wie in einem der Ansprüche 1-6 definiert ist.

**19.** Verfahren zur Herstellung einer pharmazeutischen Zusammensetzung, welches Verfahren das Mischen eines Wirkstoffs, welcher eine Verbindung wie in einem der Ansprüche 1-6 definiert, ein Enantiomer oder ein pharmazeutisch geeignetes Salz davon ist, mit einem pharmazeutisch geeigneten Träger umfaßt.

**20.** Verfahren zur Herstellung einer pharmazeutischen Zusammensetzung, welches Verfahren das Mischen eines Wirkstoffs, welcher cis-4b,5,9b,10-Tetrahydro-8-methoxy-6-methylindeno-[1,2-b]-indol oder ein Enantiomer oder pharmazeutisch geeignetes Salz davon ist, mit einem pharmazeutisch geeigneten Träger umfaßt.

**21.** Verfahren zur Herstellung einer pharmazeutischen Zusammensetzung, welches Verfahren das Mischen eines Wirkstoffs, welcher cis-5,5a,6,10b-Tetrahydro-9-methoxy-7-methylindeno-[2,1-b]-indol oder ein Enantiomer oder pharmazeutisch geeignetes Salz davon ist, mit einem pharmazeutisch geeigneten Träger umfaßt.

**22.** Verwendung einer Verbindung der Formel IA oder IB, wie in einem der Ansprüche 1 bis 9 definiert, bei der Herstellung eines Medikaments zur Behandlung von ischämischen oder Reperfusions-Störungen, Thrombose, Embolie, Arteriosklerose, Parkinson-Krankheit, Alzheimer-Krankheit, Alterserscheinungen, Neoplasmen, allergischen oder entzündlichen Zuständen, wie z.B. Bronchialasthma und rheumatische Arthritis, und durch Chemikalien, Bestrahlung, antineoplastische oder immunsuppresive Mitteln hervorgerufenen Schädigungen.

**23.** Zusammensetzung, umfassend eine Verbindung, die empfindlich gegenüber oxidativem Verfall ist, und eine Verbindung der Formel IA oder IB

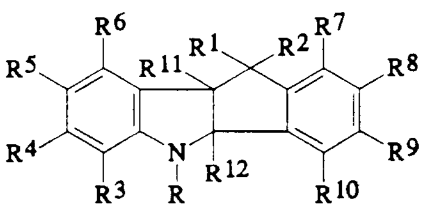

IA oder

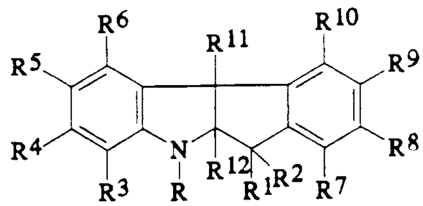

IB,

worin R Wasserstoff, eine $C_1$-$C_{24}$-Alkylgruppe oder $COR^{15}$ ist,
$R^1$, $R^2$, $R^{11}$ und $R^{12}$ unabhängig voneinander ausgewählt sind aus Wasserstoff oder einer niederen Alkylgruppe,
$R^3$, $R^4$, $R^5$ und $R^6$ unabhängig voneinander ausgewählt sind aus Wasserstoff, Hydroxyl, Halogen, einer $C_1$-$C_6$-Alkylgruppe, einer $C_1$-$C_6$-Alkoxygruppe, einer Mono- oder Di-($C_1$-$C_6$-alkyl)-aminogruppe, $NH_2$ oder $NR^{13}COR^{14}$,
$R^7$, $R^8$, $R^9$ und $R^{10}$ unabhängig voneinander ausgewählt sind aus Wasserstoff, Hydroxyl, einer $C_1$-$C_6$-Alkylgruppe, einer $C_1$-$C_6$-Alkoxygruppe oder einer Mono- oder Di-($C_1$-$C_6$-alkyl)-aminogruppe, $NH_2$ oder $NR^{13}COR^{14}$,
$R^{13}$, $R^{14}$ und $R^{15}$ unabhängig voneinander ausgewählt sind aus Wasserstoff oder einer $C_1$-$C_6$-Alkylgruppe,
unter der Bedingung, daß dann, wenn R $COR^{15}$ ist, mindestens eines von $R^3$ bis $R^{10}$ ein Hydroxyl oder eine Mono- oder Di-($C_1$-$C_6$-Alkyl)-aminogruppe ist,
oder ein Enantiomer oder Salz davon.

**24.** Zusammensetzung nach Anspruch 23, worin die Verbindung der Formel IA oder IB wie in jedem der Ansprüche 1-6 definiert ist.

**25.** Zusammensetzung, umfassend eine Verbindung, die empfindlich gegenüber oxidativem Verfall ist, und cis-4b,5,9b,10-Tetrahydro-8-methoxy-6-methylindeno-[1,2-b]-indol oder ein Enantiomer oder Salz davon.

**26.** Zusammensetzung, umfassend eine Verbindung, die empfindlich gegenüber oxidativem Verfall ist, und cis-5,5a,6,10b-Tetrahydro-9-methoxy-7-methylindeno-[2,1-b]-indol oder ein Enantiomer oder Salz davon.

27. Nicht-medizinisches Verfahren zur Stabilisierung einer Verbindung, die empfindlich gegenüber oxidativem Verfall ist, indem die empfindliche Verbindung mit einer Verbindung der Formel IA oder IB, wie in Anspruch 23 oder 24 definiert, zusammengebracht wird.

28. Nicht-medizinisches Verfahren zur Stabilisierung einer Verbindung, die empfindlich gegenüber oxidativem Verfall ist, indem die empfindliche Verbindung mit cis-4b,5,9b,10-Tetrahydro-8-methoxy-6-methylindeno-[1,2-b]-indol oder einem Enantiomer oder Salz davon zusammengebracht wird.

29. Nicht-medizinisches Verfahren zur Stabilisierung einer Verbindung, die empfindlich gegenüber oxidativem Verfall ist, indem die empfindliche Verbindung mit cis-5,5a,6,10b-Tetrahydro-9-methoxy-7-methylindeno-[2,1-b]-indol oder einem Enantiomer oder Salz davon zusammengebracht wird.

30. Verfahren zur Haltbarmachung eines Produkts, das empfindlich gegenüber oxidativer Zerstörung ist, indem das empfindliche Produkt mit einer Verbindung der Formel IA oder IB, wie in Anspruch 23 oder 24 definiert, zusammengebracht wird.

31. Verfahren zur Haltbarmachung eines Produkts, das empfindlich gegenüber oxidativem Verfall ist, indem das empfindliche Produkt mit cis-4b,5,9b,10-Tetrahydro-8-methoxy-6-methylindeno-[1,2-b]-indol oder einem Enantiomer oder Salz davon zusammengebracht wird.

32. Verfahren zur Haltbarmachung eines Produkts, das empfindlich gegenüber oxidativem Verfall ist, indem das empfindliche Produkt mit cis-5,5a,6,10b-Tetrahydro-9-methoxy-7-methylindeno-[2,1-b]-indol oder einem Enantiomer oder Salz davon zusammengebracht wird.

33. Verwendung einer Verbindung der Formel IA oder IB, wie in Anspruch 23 oder 24 definiert, als ein Mittel zur Haltbarmachung eines Produkts, das empfindlich gegenüber oxidativem Verfall ist.

34. Verwendung von cis-4b,5,9b,10-Tetrahydro-8-methoxy-6-methylindeno-[1,2-b]-indol oder einem Enantiomer oder Salz davon, als ein Mittel zur Haltbarmachung eines Produkts, das empfindlich gegenüber oxidativem Verfall ist.

35. Verwendung von cis-5,5a,6,10b-Tetrahydro-9-methoxy-7-methylindeno-[2,1-b]-indol oder einem Enantiomer oder Salz davon, als ein Mittel zur Haltbarmachung eines Produkts, das empfindlich gegenüber oxidativem Verfall ist.

## Revendications

### Revendications pour les Etats contractants suivants : AT, BE, CH, DE, DK, FR, GB, IT, LI, LU, NL, SE

1. Composé de formule IA ou IB

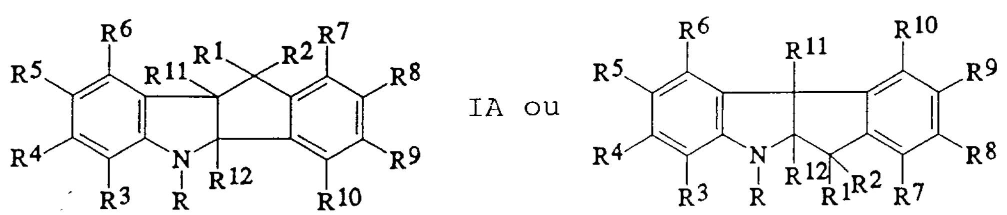

IA ou IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ son choisis indépendamment parmi un atome d'hydrogène, un groupe hydroxy, un atome d'halogène, un groupe alkyle en $C_1$-$C_6$ ou un groupe alcoxy en $C_1$-$C_6$ un groupe mono-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que :

i. lorsque R est un groupe méthyle dans la formule IA, au moins l'un des radicaux $R^1$ à $R^{12}$ n'est pas un atome d'hydrogène,

ii. lorsque R représente un atome d'hydrogène ou une groupe acétyle et $R^{11}$ représente un groupe éthyle dans la formule IA, au moins l'un des radical $R^1$ à $R^{10}$ ou $R^{12}$ n'est pas un atone d'hydrogène ;

et ses énantiomères et sels.

2. Composé selon la revendication 1, dans lequel au moins l'un de $R^3$, $R^5$, $R^7$, $R^9$, $R^{11}$ et $R^{12}$ est un groupe alkyle en $C_1$-$C_6$, de préférence un groupe méthyle, éthyle ou i-propyle.

3. Composé selon la revendication 1 ou 2, dans lequel au moins l'un de $R^5$ et $R^8$ est un groupe mono- ou di(alkyle en $C_1$-$C_6$)amino, de préférence un groupe éthylamino ou diéthylamino.

4. Composé selon la revendication 1 ou 2, dans lequel au moins l'un de $R^5$ et $R^8$ est un groupe hydroxy ou alcoxy en $C_1$-$C_6$, de préférence méthoxy.

5. Composé selon l'une quelconque des revendications précédentes, dans lequel R, $R^1$, $R^2$, $R^4$, $R^6$ et $R^{10}$ représentent un atome d'hydrogène.

6. Composé selon la revendication 1, qui est le
cis-4b,5,9b,10-tétrahydroindéno[1,2-b]indole
cis-4b,5,9b,10-tétrahydro-6,8-diméthylindéno[1,2-b]indole
cis-4b,5,9b,10-tétrahydro-5,8-diméthylindéno[1,2-b]indole
cis-4b,5,9b,10-tétrahydro-8-méthylindéno[1,2-b]indole
cis-4b,5,9b,10-tétrahydro-4b,6,8,9b-tétraméthylindéno[1,2-b]indole
cis-4b,5,9b,10-tétrahydro-8-isopropylindéno[1,2-b]indole
cis-4b,5,9b,10-tétrahydro-8-méthoxy-5-méthylindéno[1,2-b]indole
cis-4b,5,9b,10-tétrahydro-8-méthoxyindéno[1,2-b]indole
cis-4b,5,9b,10-tétrahydro-10,10-diméthylindéno[1,2-b]indole
cis-4b,5,9b,10-tétrahydro-9b-méthylindéno[1,2-b]indole
cis-4b,5,9b,10-tétrahydro-4b,9b-diméthylindéno[1,2-b]indole
cis-4b,5,9b,10-tétrahydro-4b,5,9b-triméthylindéno[1,2-b]indole
cis-4b,5,9b,10-tétrahydro-2-méthoxy-1,3-diméthylindéno[1,2-b]indole
cis-4b,5,9b,10-tétrahydro-2-méthoxy-1,3-diméthyl-8-isopropylindéno[1,2-b]-indole
cis-4b,5,9b,10-tétrahydro-4b-méthylindéno[1,2-b]indole
cis-4b,5,9b,10-tétrahydro-2-hydroxy-1,3-diméthyl-8-isopropylindéno[1,2-b]indole
cis-4b,5,9b,10-tétrahydro-2-hydroxy-1,3-diméthylindéno[1,2-b]indole
cis-4b,5,9b,10-tétrahydro-4b,8,9b-triméthylindéno[1,2-b]indole
cis-4b,5,9b,10-tétrahydro-8-isopropyl-4b,9b-diméthylindéno[1,2-b]indole
cis-4b,5,9b,10-tétrahydro-8-isopropyl-4b-méthylindéno[1,2-b]indole
cis-4b,5,9b,10-tétrahydro-2,8-diméthoxy-1,3-diméthylindéno[1,2-b]indole
cis-4b,5,9b,10-tétrahydro-4b,5,8,9b-tétraméthylindéno[1,2-b]indole
cis-4b,5,9b,10-tétrahydro-8-tert.-butylindéno[1,2-b]indole
cis-4b,5,9b,10-tétrahydro-8-méthoxy-7,9-diméthylindéno[1,2-b]indole
cis-4b,5,9b,10-tétrahydro-8-méthoxy-6-méthylindéno[1,2-b]indole
cis-4b,5,9b,10-tétrahydro-8-diéthylamino-5-éthylindéno[1,2-b]indole
cis-4b,5,9b,10-tétrahydro-2-diéthylaminoindéno[1,2-b]indole
cis-4b,5,9b,10-tétrahydro-8-tert.-butyl-4b-méthylindéno[1,2-b]indole
cis-4b,5,9b,10-tétrahydro-8-fluoroindéno[1,2-b]indole
cis-5,5a,6,10b,-tétrahydroindéno[2,1-b]indole
cis-5,5a,6,10b,-tétrahydro-9-méthoxyindéno[2,1-b]indole
cis-5,5a,6,10b,-tétrahydro-9-isopropylindéno[2,1-b]indole
cis-5,5a,6,10b,-tétrahydro-9-méthoxy-7-méthylindéno[2,1-b]indole
cis-4b,5,9b,10-tétrahydro-4,6-diméthyl-8-méthoxyindéno[1,2-b]indole
cis-4b,5,9b,10-tétrahydro-8-méthoxy-4b,6,9b-triméthylindéno[1,2-b]indole.

7. cis-4b,5,9b,10-tétrahydro-8-méthoxy-6-méthylindéno[1,2-b]indole, ses énantiomères et ses sels.

8. cis-5,5a,6,10b-tétrahydro-9-méthoxy-7-méthylindéno[2,1-b]indole, ses énantiomères et ses sels.

9. Composé de formule IA ou IB

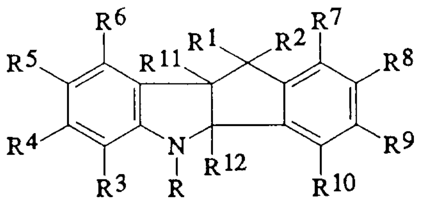

IA ou

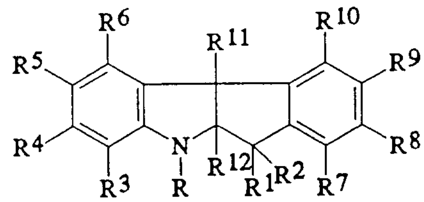

IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$ ou un groupe alcoxy en $C_1$-$C_6$, un groupe mono-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,
ses énantiomères et ses sels pharmaceutiquement acceptables; destinés à être utilisés dans un procédé thérapeutique pratiqué sur le corps humain ou animal.

10. cis-4b,5,9b,10-tétrahydro-8-méthoxy-6-méthylindéno[1,2-b]indole, ou un de ses énantiomères ou sels pharmaceutiquement acceptables, destiné à être utilisé dans un procédé thérapeutique pratiqué sur le corps humain ou animal.

11. cis-5,5a,6,10b-tétrahydro-9-méthoxy-7-méthylindéno[2,1-b]indole, ou un de ses énantiomères ou sels pharmaceutiquement acceptables, destiné à être utilisé dans un procédé thérapeutique pratiqué sur le corps humain ou animal.

12. Composé selon l'une quelconque des revendications 9 à 11, destiné à être utilisé dans le traitement de lésions ischémiques ou de reperfusion, de thromboses et d'embolies.

13. Composé selon l'une quelconque des revendications 9 à 11, destiné à être utilisé dans le traitement curatif ou préventif de néoplasmes.

14. Composé selon l'une quelconque des revendications 9 à 11, destiné à être utilisé dans le traitement de la maladie de Parkinson, de la maladie d'Alzheimer ou du vieillissement.

15. Composé selon l'une quelconque des revendications 9 à 11, destiné à être utilisé dans le traitement de l'athérosclérose.

16. Composé selon l'une quelconque des revendications 9 à 11, destiné à être utilisé dans le traitement d'états allergiques/inflammatoires tels que l'asthme bronchique et le polyarthrite rhumatoïde.

17. Composé selon l'une quelconque des revendications 9 à 11, destiné à être utilisé dans le traitement de lésions dues à des produits chimiques, des radiations, des agents antinéoplasiques ou immunosuppresseurs.

18. Composé selon l'une quelconque des revendications 9 à 17, dans lequel la formule IA ou IB est telle que définie dans l'une quelconque des revendications 1-6.

19. Composition pharmaceutique comprenant un principe actif qui est un composé tel que défini dans l'une quelconque des revendications 1-6, un de ses énantiomères ou sels pharmaceutiquement acceptables, conjointement avec un véhicule pharmaceutiquement acceptable.

**20.** Composition pharmaceutique comprenant un principe actif qui est le cis-4b,5,9b,10-tétrahydro-8-méthoxy-6-méthylindéno[1,2-b]indole, ou un de ses énantiomères ou sels pharmaceutiquement acceptables, conjointement avec un véhicule pharmaceutiquement acceptable.

**21.** Composition pharmaceutique comprenant un principe actif qui est le cis-5,5a,6,10b-tétrahydro-9-méthoxy-7-méthylindéno[2,1-b]indole, ou un de ses énantiomères ou sels pharmaceutiquement acceptables, conjointement avec un véhicule pharmaceutiquement acceptable.

**22.** Utilisation d'un composé de formule IA ou IB, tel que défini dans l'une quelconque des revendications 1 à 9, dans la fabrication d'un médicament destiné au traitement de lésions ischémiques ou de reperfusion, de thromboses, d'embolies, d'athérosclérose, de la maladie de Parkinson, de la maladie d'Alzheimer, du vieillissement, de néoplasmes, d'états allergiques ou inflammatoires tels que l'asthme bronchique et le polyarthrite rhumatoïde, et de lésions dues à des produits chimiques, des radiations, des agents antinéoplasiques ou immunosuppresseurs.

**23.** Procédé de préparation d'un composé de formule IA ou IB, tel que défini dans l'une quelconque des revendications 1 à 8, comprenant les étapes consistant à :

a. réduire le 5,10-dihydroindéno[1,2-b]indole (DHII)

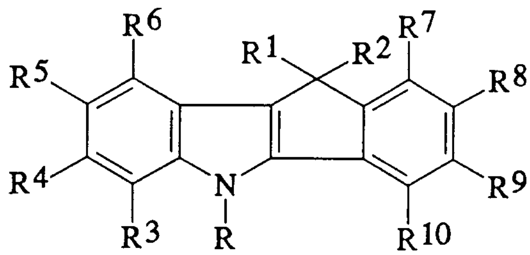

XI

où R, $R^1$, $R^2$, $R^3$, $R^4$, $R^5$, $R^6$, $R^7$, $R^8$, $R^9$ et $R^{10}$ sont définis comme dans la formule IA, si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

b. soumettre à une indolisation de Fischer une phénylhydrazine de formule II et une 1-indanone de formule III portant un substituant en position 2, puis réduire les indolénines (IV)

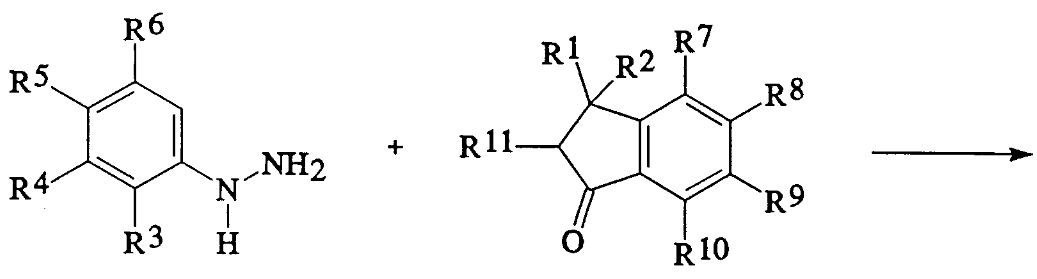

II

III

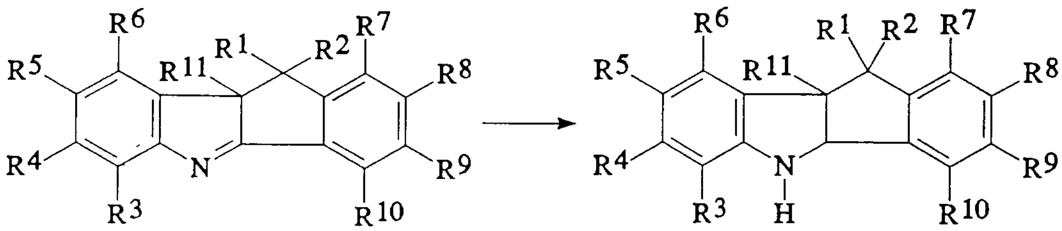

IV V

où $R^1$, $R^2$, $R^3$, $R^4$, $R^5$, $R^6$, $R^7$, $R^8$, $R^9$, $R^{10}$ et $R^{11}$ sont définis comme dans la formule IA, et si nécessaire, effectuer ensuite une N-alkylation avec un R-halogénure ou un R-sulfate, où R est défini comme dans la formule IA,

c. faire réagir des indolénines de formule IV avec des alkyllithiums ($R^{12}$-Li)

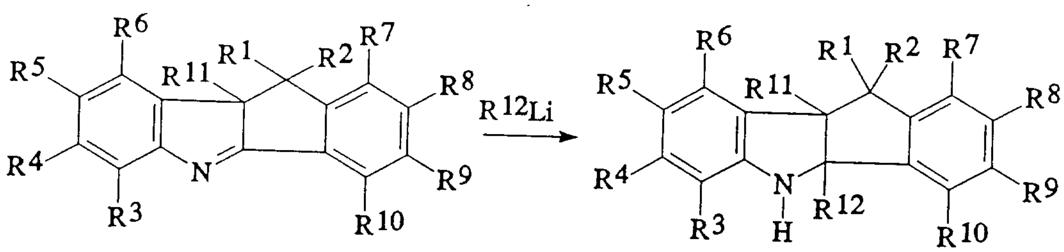


IV VI

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IA, et si nécessaire, effectuer ensuite une N-alkylation avec un R-halogénure ou un R-sulfate, où R est défini comme dans la formule IA,

d. réduire le 5,6-dihydroindéno[2,1-b]indole (iso-DHII), si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

e. pour des 5,5a,6,10b-tétrahydroindéno[2,1-b]indoles (IX) portant un substituant en 10b et leurs analogues, en utilisant des indan-2-ones (XII) portant un groupe substituant en C-3, effectuer une indolisation de Fischer avec des phénylhydrazines de formule (II), suivie d'une réaction du composé intermédiaire VIII avec un agent

réducteur,

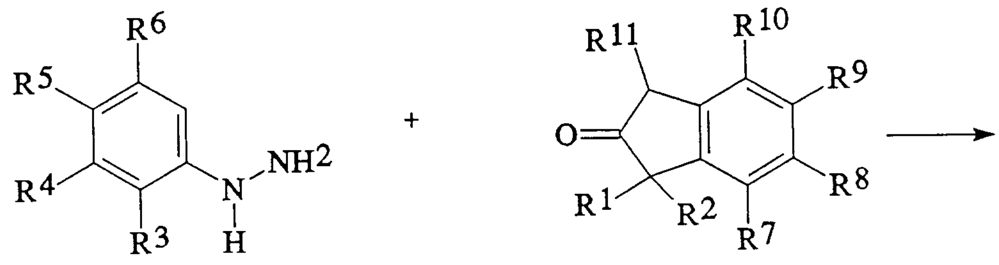

II

XII

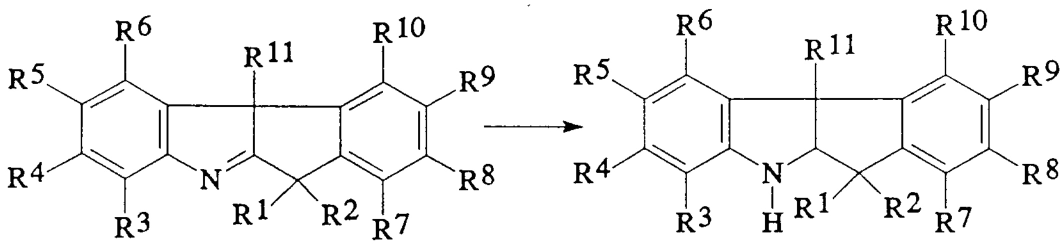

VIII

IX

où $R^1$, $R^2$, $R^3$, $R^4$, $R^5$, $R^6$, $R^7$, $R^8$, $R^9$, $R^{10}$ et $R^{11}$ sont définis comme dans la formule IB, ou si nécessaire, effectuer ensuite une N-alkylation avec un R-halogénure ou un R-sulfate, où R est défini comme dans la formule IB,

f. pour des 5,5a,6,10b-tétrahydroindéno[2,1-b]indoles portant un substituant en 5a,10b et leurs analogues, par réaction d'indolénines de formule VIII avec des alkyllithiums $R^{12}$-Li,

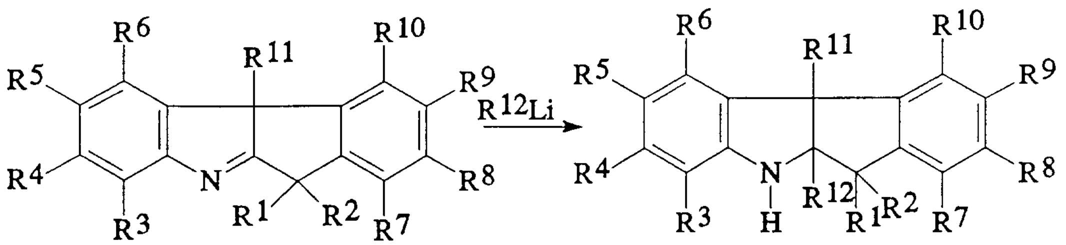


VIII

IB

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IB, ou si nécessaire, effectuer ensuite une N-alkylation avec un R-halogénure ou un R-sulfate, où R est défini comme dans la formule IB,

g. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par N-alkylation du THII ou de l'iso-THII correspondant avec un R-halogénure ou un R-sulfate,

h. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par réduction du 5-acyl-THII ou du 6-acyl-iso-THII correspondant,

i. pour THII ou iso-THII, où $R^3$-$R^6$ et/ou $R^7$-$R^{10}$ sont définis comme un groupe mono- ou di(alkyle en $C_1$-$C_6$)amino, et R, $R^1$, $R^2$, $R^{11}$ et $R^{12}$ sont définis comme dans la formule I, par réduction des composés nitro- 5-acyl-THII ou 6-acyl-iso-THII correspondants, suivie d'une N-alkylation et éventuellement, d'une hydrolyse acide,

j. pour THII ou iso-THII, où $R^3$-$R^6$ et/ou $R^7$-$R^{10}$ sont définis comme un groupe hydroxy, et R, $R^1$, $R^2$, $R^{11}$ et $R^{12}$ sont définis comme dans la formule I, par éther-désalkylation des composés THII ou iso-THII correspondants portant un substituant alcoxy,

k. pour THII ou iso-THII, où $R^{12}$ est un groupe alkyle inférieur et R, $R^1$-$R^{11}$ sont définis comme dans la formule I, par métallation de l'analogue correspondant non substitué en 4b, suivie d'une alkylation avec un $R^{12}$-halogénure ou un $R^{12}$-sulfate et d'une hydrolyse finale.

**24.** Composition comprenant un composé sensible à la détérioration par oxydation et un composé de formule IA ou IB

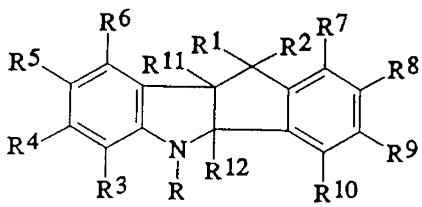

IA ou

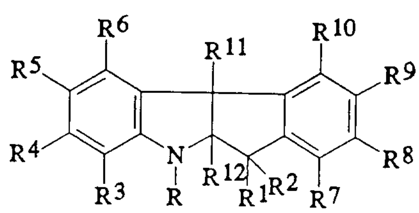

IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle inférieur,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$ ou un groupe alcoxy en $C_1$-$C_6$, un groupe mon-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,
ou ses énantiomères et ses sels.

**25.** Composition selon la revendication 24, dans laquelle le composé de formule IA ou IB est tel que défini dans l'une quelconque des revendications 1-6.

**26.** Composition comprenant un composé sensible à la détérioration par oxydation et le cis-4b,5,9b,10-tétrahydro-8-méthoxy-6-méthylindéno[1,2-b]indole, ou un énantiomère ou un sel de celui-ci.

**27.** Composition comprenant un composé sensible à la détérioration par oxydation et le cis-5,5a,6,10b-tétrahydro-9-méthoxy-7-méthylindéno[2,1-b]indole, ou un énantiomère ou un sel de celui-ci.

**28.** Procédé non médical pour stabiliser un composé sensible à la détérioration par oxydation, par mise en contact du composé sensible avec un composé de formule IA ou IB tel que défini à la revendication 24 ou 25.

**29.** Procédé non médical pour stabiliser un composé sensible à la détérioration par oxydation, par mise en contact du composé sensible avec le cis-4b,5,9b,10-tétrahydro-8-méthoxy-6-méthylindéno[1,2-b]indole ou un énantiomère ou un sel de celui-ci.

**30.** Procédé non médical pour stabiliser un composé sensible à la détérioration par oxydation, par mise en contact du composé sensible avec le cis-5,5a,6,10b-tétrahydro-9-méthoxy-7-méthylindéno[2,1-b]indole ou un énantiomère ou un sel de celui-ci.

**31.** Procédé pour la conservation d'un produit sensible à la détérioration par oxydation, par mise en contact du produit sensible avec un composé de formule IA ou IB, tel que défini dans la revendication 24 ou 25.

**32.** Procédé pour la conservation d'un produit sensible à la détérioration par oxydation, par mise en contact du produit sensible avec le cis-4b,5,9b,10-tétrahydro-8-méthoxy-6-méthylindéno[1,2-b]indole ou un énantiomère ou un sel de celui-ci.

**33.** Procédé pour la conservation d'un produit sensible à la détérioration par oxydation, par mise en contact du produit sensible avec le cis-5,5a,6,10b-tétrahydro-9-méthoxy-7-méthylindéno[2,1-b]indole ou un énantiomère ou un sel de celui-ci.

**34.** Utilisation d'un composé de formule IA ou IB tel que défini dans la revendication 24 ou 25, en tant qu'agent de conservation d'un produit sensible à la détérioration par oxydation.

**35.** Utilisation du cis-4b,5,9b,10-tétrahydro-8-méthoxy-6-méthylindéno-[1,2-b]indole ou d'un énantiomère ou d'un sel de celui-ci, en tant qu'agent de conservation d'un produit sensible à la détérioration par oxydation.

**36.** Utilisation du cis-5,5a,6,10b-tétrahydro-9-méthoxy-7-méthylindéno-[2,1-b]indole ou d'un énantiomère ou d'un sel de celui-ci, en tant qu'agent de conservation d'un produit sensible à la détérioration par oxydation.

**Revendications pour l'Etat contractant suivant : ES**

**1.** Procédé de préparation d'un composé de formule IA ou IB

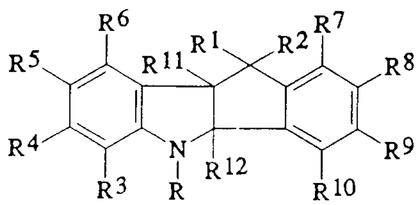
IA ou
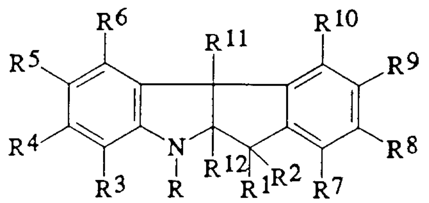
IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ sont choisis indépendamment parmi' un atome d'hydrogène, un groupe hydroxy, un atome d'halogène, un groupe alkyle en $C_1$-$C_6$ ou un groupe alcoxy en $C_1$-$C_6$, un groupe mono-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que :

i. lorsque R est un groupe méthyle dans la formule IA, au moins l'un des radical $R^1$ à $R^{12}$ n'est pas un atome d'hydrogène,

ii. lorsque R représente un atome d'hydrogène ou une groupe acétyle et $R^{11}$ représente un groupe éthyle dans la formule IA, au moins l'un des radical $R^1$ à $R^{10}$ ou $R^{12}$ n'est pas un atome d'hydrogène ;

et de ses énantiomères et sels, comprenant les étapes consistant à :

a. réduire le 5,10-dihydroindéno[1,2-b]indole (DHII)

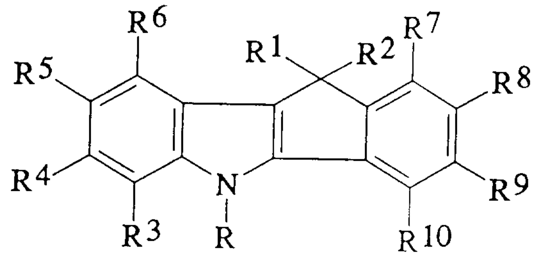

XI

où R, $R^1$, $R^2$, $R^3$, $R^4$, $R^5$, $R^6$, $R^7$, $R^8$, $R^9$ et $R^{10}$ sont définis comme dans la formule IA, si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

b. soumettre à une indolisation de Fischer une phénylhydrazine de formule II et une 1-indanone de formule III portant un substituant en position 2, puis réduire les indolénines (IV)

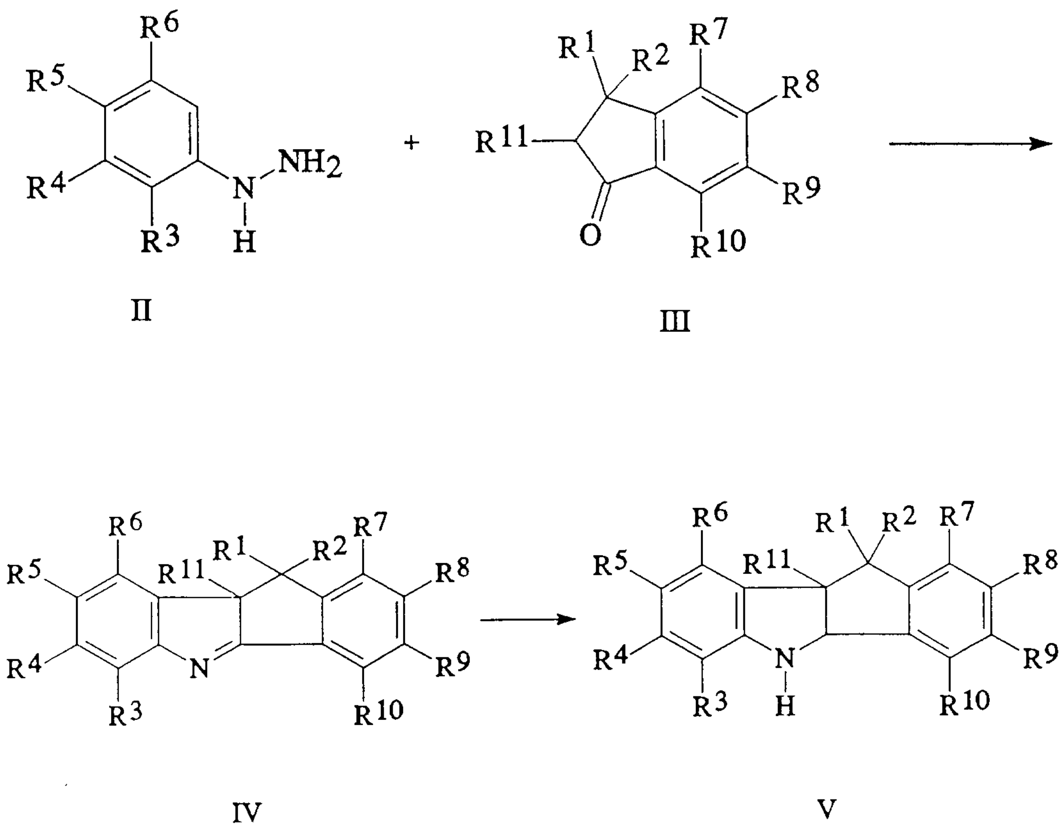

II +

III

IV

V

où $R^1$, $R^2$, $R^3$, $R^4$, $R^5$, $R^6$, $R^7$, $R^8$, $R^9$, $R^{10}$ et $R^{11}$ sont définis comme dans la formule IA, et si nécessaire, effectuer ensuite une N-alkylation avec un R-halogénure ou un R-sulfate, où R est défini comme dans la formule IA,

c. faire réagir des indolénines de formule IV avec des alkyllithiums ($R^{12}$-Li)

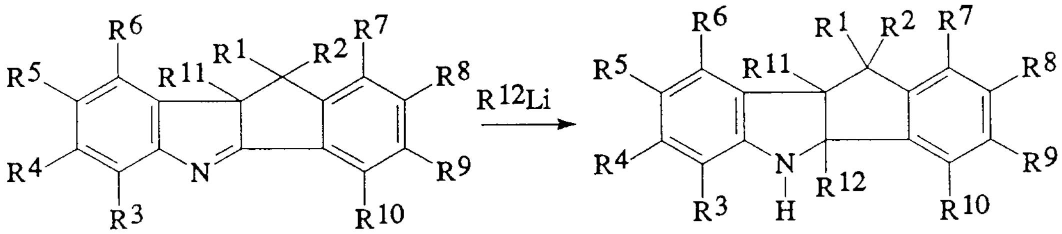


IV VI

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IA, et si nécessaire, effectuer ensuite une N-alkylation avec un R-halogénure ou un R-sulfate, où R est défini comme dans la formule IA,

d. réduire le 5,6-dihydroindéno[2,1-b]indole (iso-DHII), si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

e. pour des 5,5a,6,10b-tétrahydroindéno[2,1-b]indoles (IX) portant un substituant en 10b et leurs analogues, en utilisant des indan-2-ones (XII) portant un groupe substituant en C-3, effectuer une indolisation de Fischer avec des phénylhydrazines de formule (II), suivie d'une réaction du composé intermédiaire VIII avec un agent réducteur,

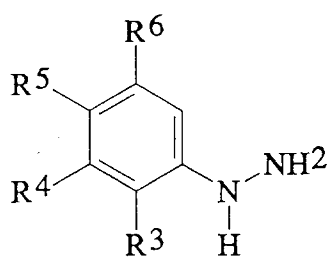

+

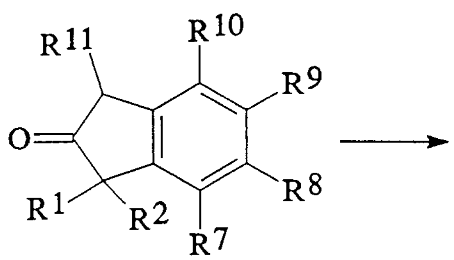

II XII

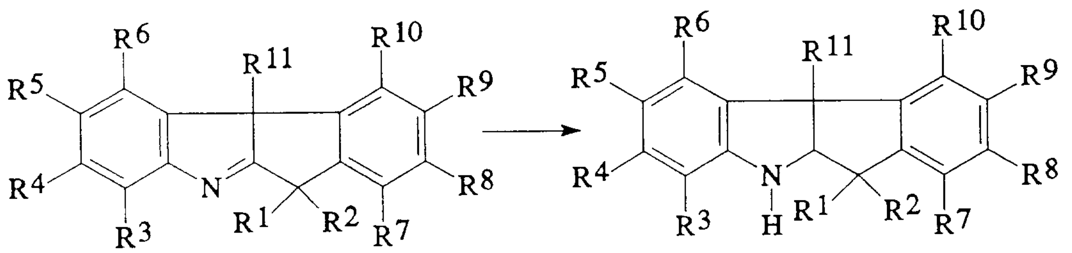

VIII IX

où $R^1$, $R^2$, $R^3$, $R^4$, $R^5$, $R^6$, $R^7$, $R^8$, $R^9$, $R^{10}$ et $R^{11}$ sont définis comme dans la formule IB, ou si nécessaire,

effectuer ensuite une N-alkylation avec un R-halogénure ou un R-sulfate, où R est défini comme dans la formule IB,

f. pour des 5,5a,6,10b-tétrahydroindéno[2,1-b]indoles portant un substituant en 5a,10b et leurs analogues, par réaction d'indolénines de formule VII avec des alkyllithiums $R^{12}$-Li,

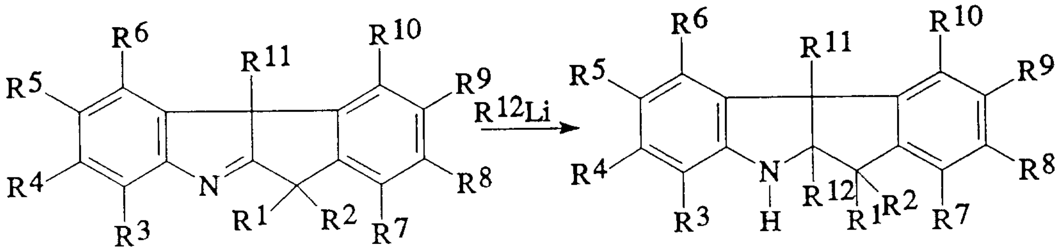

VIII IB

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IB, ou si nécessaire, effectuer ensuite une N-alkylation avec un R-halogénure ou un R-sulfate, où R est défini comme dans la formule IB,

g. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par N-alkylation du THII ou de l'iso-THII correspondant avec un R-halogénure ou un R-sulfate,

h. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par réduction du 5-acyl-THII ou du 6-acyl-iso-THII correspondant,

i. pour THII ou iso-THII, où $R^3$-$R^6$ et/ou $R^7$-$R^{10}$ sont définis comme un groupe mono- ou di(alkyle en $C_1$-$C_6$)amino, et R, $R^1$, $R^2$, $R^{11}$ et $R^{12}$ sont définis comme dans la formule I, par réduction des composés nitro- 5-acyl-THII ou 6-acyl-iso-THII correspondants, suivie d'une N-alkylation et éventuellement, d'une hydrolyse acide,

j. pour THII ou iso-THII, où $R^3$-$R^6$ et/ou $R^7$-$R^{10}$ sont définis comme un groupe hydroxy, et R, $R^1$, $R^2$, $R^{11}$ et $R^{12}$ sont définis comme dans la formule I, par éther-désalkylation des composés THII ou iso-THII correspondants portant un substituant alcoxy,

k. pour THII ou iso-THII, où $R^{12}$ est un groupe alkyle inférieur et R, $R^1$-$R^{11}$ sont définis comme dans la formule I, par métallation de l'analogue correspondant non substitué en 4b, suivie d'une alkylation avec un $R^{12}$-halogénure ou un $R^{12}$-sulfate et d'une hydrolyse finale.

2. Procédé selon la revendication 1, dans lequel au moins l'un de $R^3$, $R^5$, $R^7$, $R^9$, $R^{11}$ et $R^{12}$ est un groupe alkyle en $C_1$-$C_6$, de préférence un groupe méthyle, éthyle ou i-propyle.

3. Procédé selon la revendication 1 ou 2, dans lequel au moins l'un de $R^5$ et $R^8$ est un groupe mono- ou di(alkyle en $C_1$-$C_6$)amino, de préférence un groupe éthylamino ou diéthylamino.

4. Procédé selon la revendication 1 ou 2, dans lequel au moins l'un de $R^5$ et $R^8$ est un groupe hydroxy ou alcoxy en $C_1$-$C_6$, de préférence méthoxy.

5. Procédé selon l'une quelconque des revendications précédentes, dans lequel R, $R^1$, $R^2$, $R^4$, $R^6$ et $R^{10}$ représentent un atome d'hydrogène.

6. Procédé selon la revendication 1, dans lequel on obtient les composés suivants :
cis-4b,5,9b,10-tétrahydroindéno[1,2-b]indole
cis-4b,5,9b,10-tétrahydro-6,8-diméthylindéno[1,2-b]indole
cis-4b,5,9b,10-tétrahydro-5,8-diméthylindéno[1,2-b]indole
cis-4b,5,9b,10-tétrahydro-8-méthylindéno[1,2-b]indole
cis-4b,5,9b,10-tétrahydro-4b,6,8,9b-tétraméthylindéno[1,2-b]indole

cis-4b,5,9b,10-tétrahydro-8-isopropylindéno[1,2-b]indole
cis-4b,5,9b,10-tétrahydro-8-méthoxy-5-méthylindéno[1,2-b]indole
cis-4b,5,9b,10-tétrahydro-8-méthoxyindéno[1,2-b]indole
cis-4b,5,9b,10-tétrahydro-10,10-diméthylindéno[1,2-b]indole
cis-4b,5,9b,10-tétrahydro-9b-méthylindéno[1,2-b]indole
cis-4b,5,9b,10-tétrahydro-4b,9b-diméthylindéno[1,2-b]indole
cis-4b,5,9b,10-tétrahydro-4b,5,9b-triméthylindéno[1,2-b]indole
cis-4b,5,9b,10-tétrahydro-2-méthoxy-1,3-diméthylindéno[1,2-b]indole
cis-4b,5,9b,10-tétrahydro-2-méthoxy-1,3-diméthyl-8-isopropylindéno[1,2-b]-indole
cis-4b,5,9b,10-tétrahydro-4b-méthylindéno[1,2-b]indole
cis-4b,5,9b,10-tétrahydro-2-hydroxy-1,3-diméthyl-8-isopropylindéno[1,2-b]indole
cis-4b,5,9b,10-tétrahydro-2-hydroxy-1,3-diméthylindéno[1,2-b]indole
cis-4b,5,9b,10-tétrahydro-4b,8,9b-triméthylindéno[1,2-b]indole
cis-4b,5,9b,10-tétrahydro-8-isopropyl-4b,9b-diméthylindéno[1,2-b]indole
cis-4b,5,9b,10-tétrahydro-8-isopropyl-4b-méthylindéno[1,2-b]indole
cis-4b,5,9b,10-tétrahydro-2,8-diméthoxy-1,3-diméthylindéno[1,2-b]indole
cis-4b,5,9b,10-tétrahydro-4b,5,8,9b-tétraméthylindéno[1,2-b]indole
cis-4b,5,9b,10-tétrahydro-8-tert.-butylindéno[1,2-b]indole
cis-4b,5,9b,10-tétrahydro-8-méthoxy-7,9-diméthylindéno[1,2-b]indole
cis-4b,5,9b,10-tétrahydro-8-méthoxy-6-méthylindéno[1,2-b]indole
cis-4b,5,9b,10-tétrahydro-8-diéthylamino-5-éthylindéno[1,2-b]indole
cis-4b,5,9b,10-tétrahydro-2-diéthylaminoindéno[1,2-b]indole
cis-4b,5,9b,10-tétrahydro-8-tert.-butyl-4b-méthylindéno[1,2-b]indole
cis-4b,5,9b,10-tétrahydro-8-fluoroindéno[1,2-b]indole
cis-5,5a,6,10b,-tétrahydroindéno[2,1-b]indole
cis-5,5a,6,10b,-tétrahydro-9-méthoxyindéno[2,1-b]indole
cis-5,5a,6,10b,-tétrahydro-9-isopropylindéno[2,1-b]indole
cis-5,5a,6,10b,-tétrahydro-9-méthoxy-7-méthylindéno[2,1-b]indole
cis-4b,5,9b,10-tétrahydro-4,6-diméthyl-8-méthoxyindéno[1,2-b]indole
cis-4b,5,9b,10-tétrahydro-8-méthoxy-4b,6,9b-triméthylindéno[1,2-b]indole.

7. Procédé selon la revendication 1, dans lequel on obtient le cis-4b,5,9b,10-tétrahydro-8-méthoxy-6-méthylindéno[1,2-b]indole, ou un de ses énantiomères ou sels.

8. Procédé selon la revendication 1, dans lequel on obtient le cis-5,5a,6,10b-tétrahydro-9-méthoxy-7-méthylindéno[2,1-b]indole, ou un de ses énantiomères ou sels.

9. Procédé de préparation d'un composé de formule IA ou IB

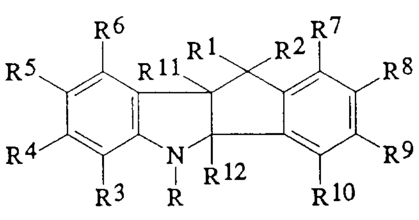 IA ou 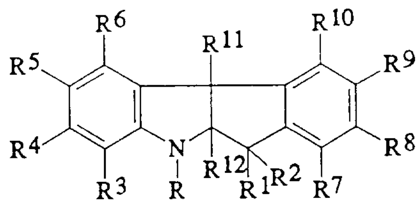 IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$, un groupe alcoxy en $C_1$-$C_6$, ou un groupe mon-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,

ou d'un de ses énantiomères et sels pharmaceutiquement acceptables ; destinés à être utilisés dans un procédé thérapeutique pratiqué sur le corps humain ou animal, comprenant les étapes consistant à :

a. réduire le 5,10-dihydroindéno[1,2-b]indole (DHII)

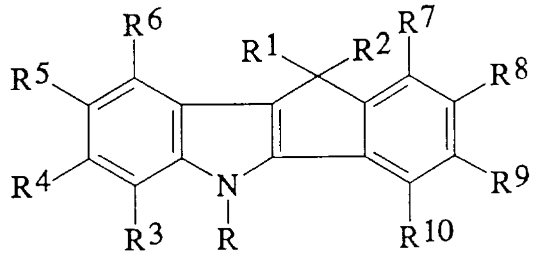

XI

où R, $R^1$, $R^2$, $R^3$, $R^4$, $R^5$, $R^6$, $R^7$, $R^8$, $R^9$ et $R^{10}$ sont définis comme dans la formule IA, si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

b. soumettre à une indolisation de Fischer une phénylhydrazine de formule II et une 1-indanone de formule III portant un substituant en position 2, puis réduire les indolénines (IV)

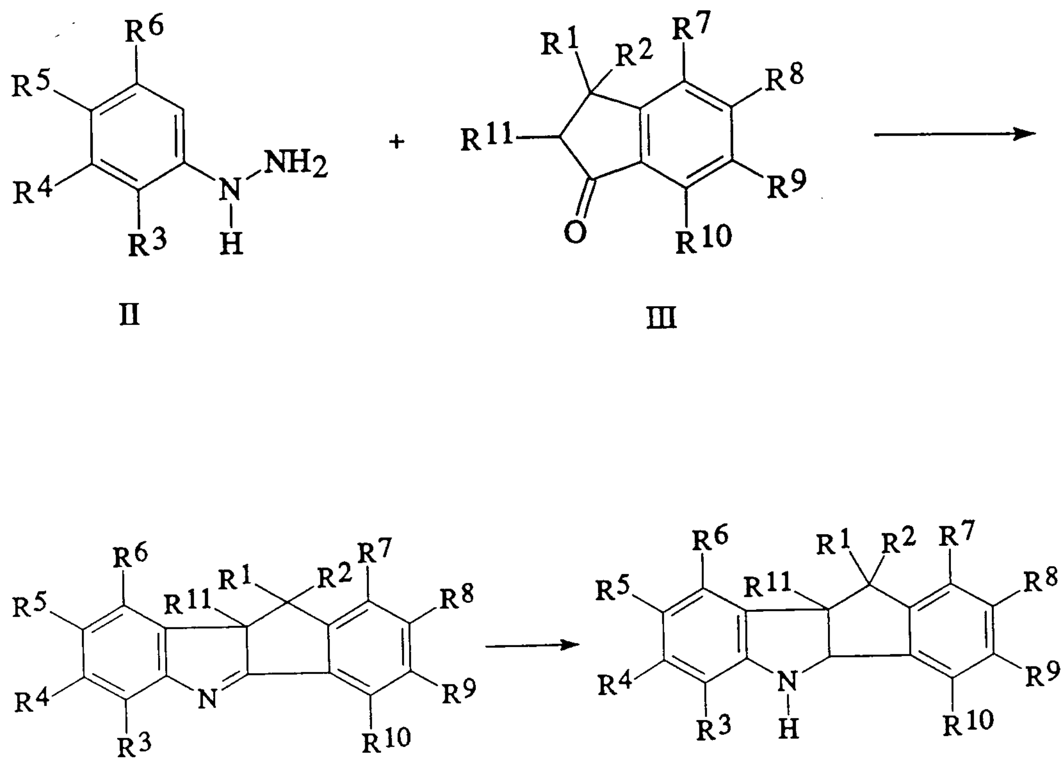

II III

IV V

où $R^1$, $R^2$, $R^3$, $R^4$, $R^5$, $R^6$, $R^7$, $R^8$, $R^9$, $R^{10}$ et $R^{11}$ sont définis comme dans la formule IA, et si nécessaire,

effectuer ensuite une N-alkylation avec un R-halogénure ou un R-sulfate, où R est défini comme dans la formule IA,

c. faire réagir des indolénines de formule IV avec des alkyllithiums ($R^{12}$-Li)

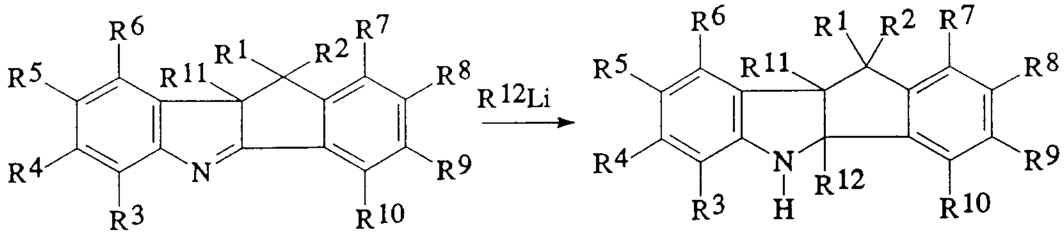


IV VI

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IA, et si nécessaire, effectuer ensuite une N-alkylation avec un R-halogénure ou un R-sulfate, où R est défini comme dans la formule IA,

d. réduire le 5,6-dihydroindéno[2,1-b]indole (iso-DHII), si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

e. pour des 5,5a,6,10b-tétrahydroindéno[2,1-b]indoles (IX) portant un substituant en 10b et leurs analogues, en utilisant des indan-2-ones (XII) portant un groupe substituant en C-3, effectuer une indolisation de Fischer avec des phénylhydrazines de formule (II), suivie d'une réaction du composé intermédiaire VIII avec un agent réducteur,

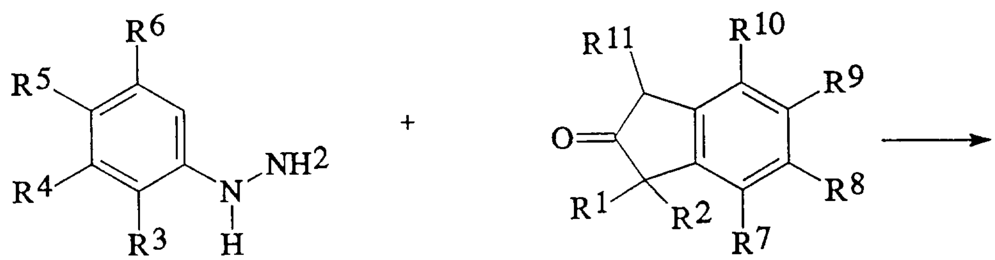

II XII

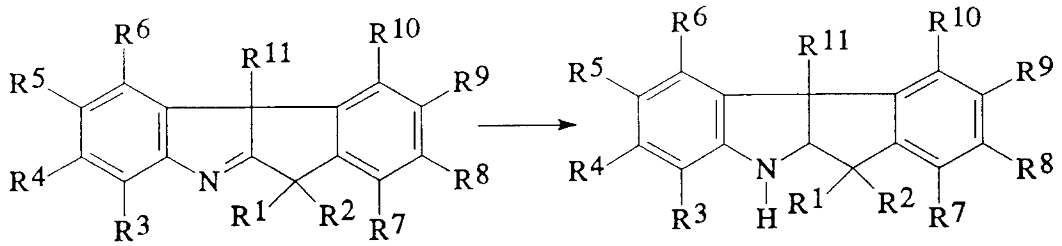

VIII IX

où $R^1$, $R^2$, $R^3$, $R^4$, $R^5$, $R^6$, $R^7$, $R^8$, $R^9$, $R^{10}$ et $R^{11}$ sont définis comme dans la formule IB, ou si nécessaire, effectuer ensuite une N-alkylation avec un R-halogénure ou un R-sulfate, où R est défini comme dans la formule IB,

f. pour des 5,5a,6,10b-tétrahydroindéno[2,1-b]indoles portant un substituant en 5a,10b et leurs analogues, par réaction d'indolénines de formule VII avec des alkyllithiums $R^{12}$-Li,

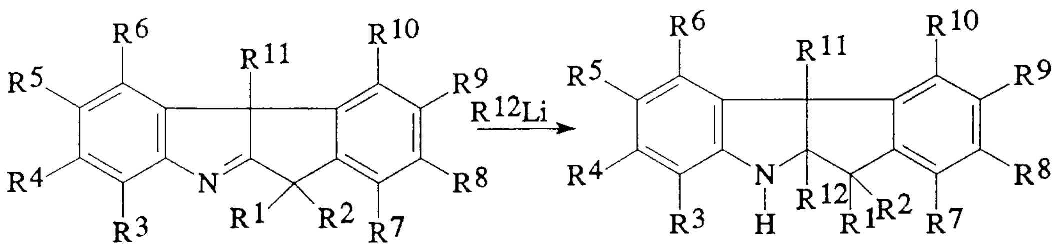


VIII IB

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IB, ou si nécessaire, effectuer ensuite une N-alkylation avec un R-halogénure ou un R-sulfate, où R est défini comme dans la formule IB,

g. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par N-alkylation du THII ou de l'iso-THII correspondant avec un R-halogénure ou un R-sulfate,

h. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par réduction du 5-acyl-THII ou du 6-acyl-iso-THII correspondant,

i. pour THII ou iso-THII, où $R^3$-$R^6$ et/ou $R^7$-$R^{10}$ sont définis comme un groupe mono- ou di(alkyle en $C_1$-$C_6$)amino, et R, $R^1$, $R^2$, $R^{11}$ et $R^{12}$ sont définis comme dans la formule I, par réduction des composés nitro- 5-acyl-THII ou 6-acyl-iso-THII correspondants, suivie d'une N-alkylation et éventuellement, d'une hydrolyse acide,

j. pour THII ou iso-THII, où $R^3$-$R^6$ et/ou $R^7$-$R^{10}$ sont définis comme un groupe hydroxy, et R, $R^1$, $R^2$, $R^{11}$ et $R^{12}$ sont définis comme dans la formule I, par éther-désalkylation des composés THII ou iso-THII correspondants portant un substituant alcoxy,

k. pour THII ou iso-THII, où $R^{12}$ est un groupe alkyle inférieur et R, $R^1$-$R^{11}$ sont définis comme dans la formule I, par métallation de l'analogue correspondant non substitué en 4b, suivie d'une alkylation avec un $R^{12}$-halogénure ou un $R^{12}$-sulfate et d'une hydrolyse finale.

**10.** Procédé selon la revendication 9, dans lequel on obtient le cis-4b,5,9b,10-tétrahydro-8-méthoxy-6-méthylindéno[1,2-b]indole, ou un de ses énantiomères ou sels pharmaceutiquement acceptables, destiné à être utilisé dans un procédé thérapeutique pratiqué sur le corps humain ou animal.

**11.** Procédé selon la revendication 9, dans lequel on obtient le cis-5,5a,6,10b-tétrahydro-9-méthoxy-7-méthylindéno[2,1-b]indole, ou un de ses énantiomères ou sels pharmaceutiquement acceptables, destiné à être utilisé dans un procédé thérapeutique pratiqué sur le corps humain ou animal.

**12.** Procédé selon l'une quelconque des revendications 9 à 11, dans lequel le produit est destiné à être utilisé dans le traitement de lésions ischémiques ou de reperfusion, de thromboses et d'embolies.

**13.** Procédé selon l'une quelconque des revendications 9 à 11, dans lequel le produit est destiné à être utilisé dans le traitement curatif ou préventif de néoplasmes.

**14.** Procédé selon l'une quelconque des revendications 9 à 11, dans lequel le produit est destiné à être utilisé dans le traitement de la maladie de Parkinson, de la maladie d'Alzheimer ou du vieillissement.

**15.** Procédé selon l'une quelconque des revendications 9 à 11, dans lequel le produit est destiné à être utilisé dans le traitement de l'athérosclérose.

**16.** Procédé selon l'une quelconque des revendications 9 à 11, dans lequel le produit est destiné à être utilisé dans le traitement d'états allergiques/inflammatoires tels que l'asthme bronchique et le polyarthrite rhumatoïde.

**17.** Procédé selon l'une quelconque des revendications 9 à 11, dans lequel le produit est destiné à être utilisé dans le traitement de lésions dues à des produits chimiques, des radiations, des agents antinéoplasiques ou immunosuppresseurs.

**18.** Procédé selon l'une quelconque des revendications 9 à 17, dans lequel la formule IA ou IB est telle que définie dans l'une quelconque des revendications 1-6.

**19.** Procédé de préparation d'une composition pharmaceutique, consistant à mélanger un principe actif qui est un composé tel que défini dans l'une quelconque des revendications 1-6, un de ses énantiomères ou sels pharmaceutiquement acceptables, avec un véhicule pharmaceutiquement acceptable.

**20.** Procédé de préparation d'une composition pharmaceutique, consistant à mélanger un principe actif qui est le cis-4b,5,9b,10-tétrahydro-8-méthoxy-6-méthylindéno[1,2-b]indole, ou un de ses énantiomères ou sels pharmaceutiquement acceptables, avec un véhicule pharmaceutiquement acceptable.

**21.** Procédé de préparation d'une composition pharmaceutique, consistant à mélanger un principe actif qui est le cis-5,5a,6,10b-tétrahydro-9-méthoxy-7-méthylindéno[2,1-b]indole, ou un de ses énantiomères ou sels pharmaceutiquement acceptables, avec un véhicule pharmaceutiquement acceptable.

**22.** Utilisation d'un composé de formule IA ou IB, tel que défini dans l'une quelconque des revendications 1 à 9, dans la fabrication d'un médicament destiné au traitement de lésions ischémiques ou de reperfusion, de thromboses, d'embolies, d'athérosclérose, de la maladie de Parkinson, de la maladie d'Alzheimer, du vieillissement, de néoplasmes, d'états allergiques ou inflammatoires tels que l'asthme bronchique et le polyarthrite rhumatoïde, et de lésions dues à des produits chimiques, des radiations, des agents antinéoplasiques ou immunosuppresseurs.

**23.** Procédé non médical pour stabiliser un composé sensible à la détérioration par oxydation, par mise en contact du composé sensible avec un compose de formule IA ou IB

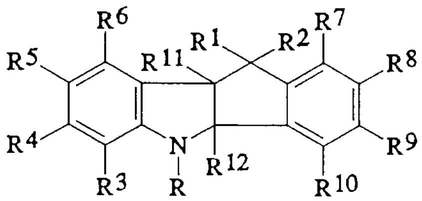

IA ou

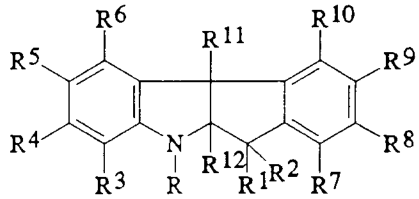

IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle inférieur,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$, un groupe alcoxy en $C_1$-$C_6$, un groupe mon-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$ ou un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,
ou un de ses énantiomères ou sels.

**24.** Procédé selon la revendication 23, dans lequel le composé de formule IA ou IB est tel que défini dans l'une quelconque des revendications 1-6.

**25.** Procédé non médical pour stabiliser un composé sensible à la détérioration par oxydation, par mise en contact du composé sensible avec le cis-4b,5,9b,10-tétrahydro-8-méthoxy-6-méthylindéno[1,2-b]indole ou un énantiomère ou un sel de celui-ci.

**26.** Procédé non médical pour stabiliser un composé sensible à la détérioration par oxydation, par mise en contact du composé sensible avec le cis-5,5a,6,10b-tétrahydro-9-méthoxy-7-méthylindéno[2,1-b]indole ou un énantiomère ou un sel de celui-ci.

**27.** Procédé pour la conservation d'un produit sensible à la détérioration par oxydation, par mise en contact du produit sensible avec un composé de formule IA ou IB, tel que défini dans la revendication 23 ou 24.

**28.** Procédé pour la conservation d'un produit sensible à la détérioration par oxydation, par mise en contact du produit sensible avec le cis-4b,5,9b,10-tétrahydro-8-méthoxy-6-méthylindéno[1,2-b]indole ou un énantiomère ou un sel de celui-ci.

**29.** Procédé pour la conservation d'un produit sensible à la détérioration par oxydation, par mise en contact du produit sensible avec le cis-5,5a,6,10b-tétrahydro-9-méthoxy-7-méthylindéno[2,1-b]indole ou un énantiomère ou un sel de celui-ci.

**30.** Utilisation d'un composé de formule IA ou IB tel que défini dans la revendication 23 ou 24, en tant qu'agent de conservation d'un produit sensible à la détérioration par oxydation.

**31.** Utilisation du cis-4b,5,9b,10-tétrahydro-8-méthoxy-6-méthylindéno-[1,2-b]indole ou d'un énantiomère ou d'un sel de celui-ci, en tant qu'agent de conservation d'un produit sensible à la détérioration par oxydation.

**32.** Utilisation du cis-5,5a,6,10b-tétrahydro-9-méthoxy-7-méthylindéno-[2,1-b]indole ou d'un énantiomère ou d'un sel de celui-ci, en tant qu'agent de conservation d'un produit sensible à la détérioration par oxydation.

**Revendications pour l'Etat contractant suivant : GR**

1. Procédé de préparation d'un composé de formule IA ou IB

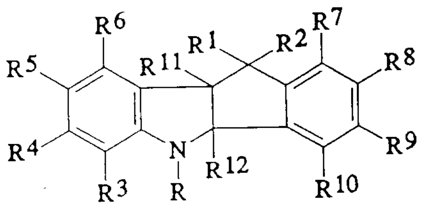
IA ou
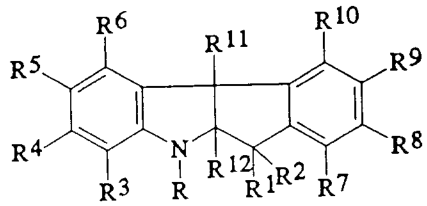
IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$, ou un groupe alcoxy en $C_1$-$C_6$, un groupe mono-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que :

i. lorsque R est un groupe méthyle dans la formule IA, au moins l'un des radical $R^1$ à $R^{12}$ n'est pas un atome d'hydrogène,

ii. lorsque R représente un atome d'hydrogène ou une groupe acétyle et $R^{11}$ représente un groupe éthyle dans la formule IA, au moins l'un des radical $R^1$ à $R^{10}$ ou $R^{12}$ n'est pas un atome d'hydrogène;

et de ses énantiomères et sels, comprenant les étapes consistant à :

a. réduire le 5,10-dihydroindéno[1,2-b]indole (DHII)

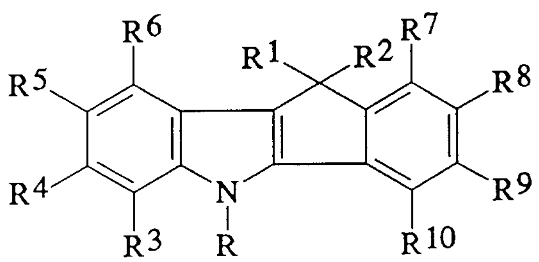

XI

où R, $R^1$, $R^2$, $R^3$, $R^4$, $R^5$, $R^6$, $R^7$, $R^8$, $R^9$ et $R^{10}$ sont définis comme dans la formule IA, si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

b. soumettre à une indolisation de Fischer une phénylhydrazine de formule II et une 1-indanone de formule III portant un substituant en position 2, puis réduire les indolénines (IV)

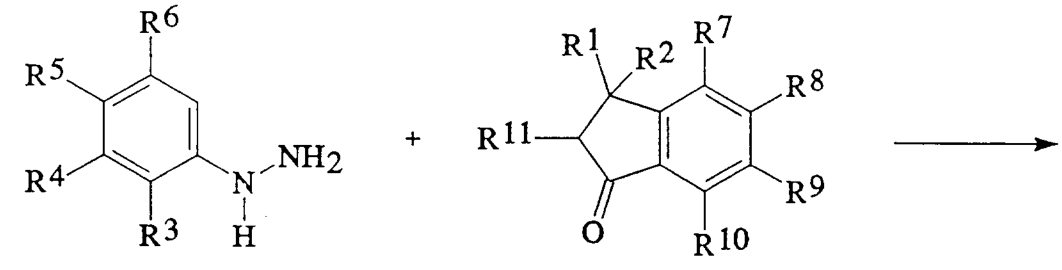

II III

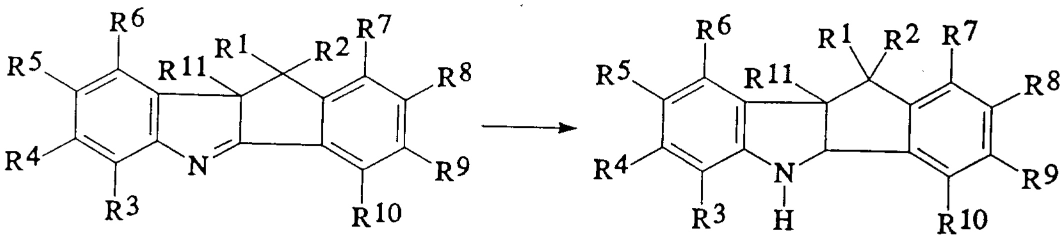

IV V

où $R^1$, $R^2$, $R^3$, $R^4$, $R^5$, $R^6$, $R^7$, $R^8$, $R^9$, $R^{10}$ et $R^{11}$ sont définis comme dans la formule IA, et si nécessaire, effectuer ensuite une N-alkylation avec un R-halogénure ou un R-sulfate, où R est défini comme dans la formule IA,

c. faire réagir des indolénines de formule IV avec des alkyllithiums ($R^{12}$-Li)

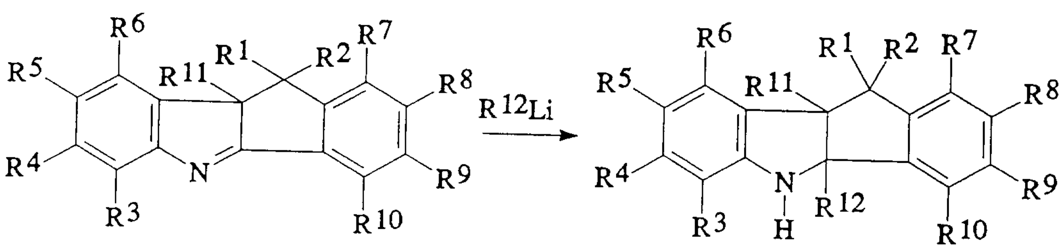


IV VI

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IA, et si nécessaire, effectuer ensuite une N-alkylation avec un R-halogénure ou un R-sulfate, où R est défini comme dans la formule IA,

d. réduire le 5,6-dihydroindéno[2,1-b]indole (iso-DHII), si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

e. pour des 5,5a,6,10b-tétrahydroindéno[2,1-b]indoles (IX) portant un substituant en 10b et leurs analogues, en utilisant des indan-2-ones (XII) portant un groupe substituant en C-3, effectuer une indolisation de Fischer avec des phénylhydrazines de formule (II), suivie d'une réaction du composé intermédiaire VII avec un agent réducteur,

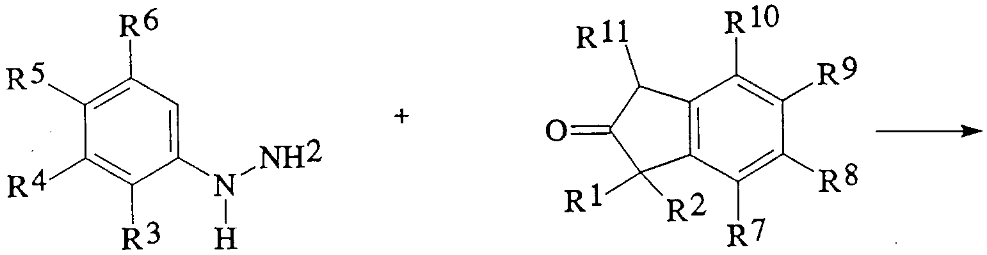

II XII

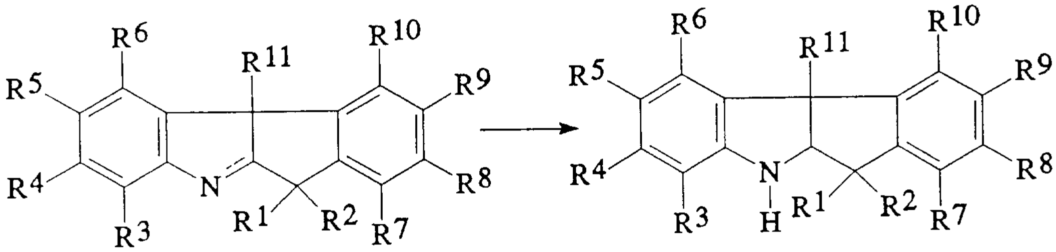

VIII IX

où $R^1$, $R^2$, $R^3$, $R^4$, $R^5$, $R^6$, $R^7$, $R^8$, $R^9$, $R^{10}$ et $R^{11}$ sont définis comme dans la formule IB, ou si nécessaire, effectuer ensuite une N-alkylation avec un R-halogénure ou un R-sulfate, où R est défini comme dans la formule IB,

f. pour des 5,5a,6,10b-tétrahydroindéno[2,1-b]indoles portant un substituant en 5a,10b et leurs analogues, par réaction d'indolénines de formule VII avec des alkyllithiums $R^{12}$-Li,

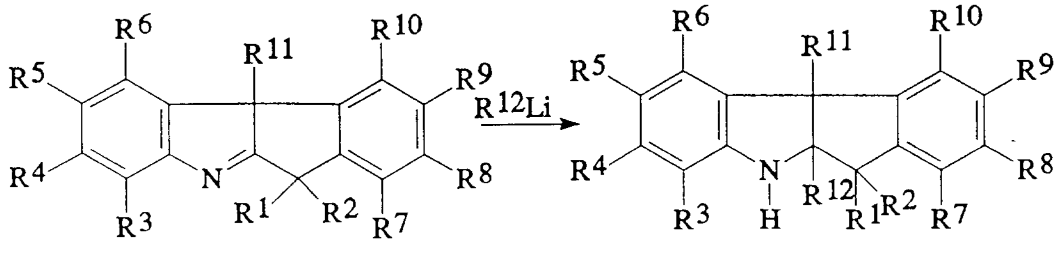


VIII IB

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IB, ou si nécessaire, effectuer ensuite une N-alkylation avec un R-halogénure ou un R-sulfate, où R est défini comme dans la formule IB,

g. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par N-alkylation du THII ou de l'iso-THII correspondant avec un R-halogénure ou un R-sulfate,

h. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par réduction du 5-acyl-THII ou du 6-acyl-iso-THII correspondant,

i. pour THII ou iso-THII, où $R^3$-$R^6$ et/ou $R^7$-$R^{10}$ sont définis comme un groupe mono- ou di(alkyle en $C_1$-$C_6$)amino, et R, $R^1$, $R^2$, $R^{11}$ et $R^{12}$ sont définis comme dans la formule I, par réduction des composés nitro- 5-acyl-THII ou 6-acyl-iso-THII correspondants, suivie d'une N-alkylation et éventuellement, d'une hydrolyse acide,

j. pour THII ou iso-THII, où $R^3$-$R^6$ et/ou $R^7$-$R^{10}$ sont définis comme un groupe hydroxy, et R, $R^1$, $R^2$, $R^{11}$ et $R^{12}$ sont définis comme dans la formule I, par éther-désalkylation des composés THII ou iso-THII correspondants portant un substituant alcoxy,

k. pour THII ou iso-THII, où $R^{12}$ est un groupe alkyle inférieur et R, $R^1$-$R^{11}$ sont définis comme dans la formule I, par métallation de l'analogue correspondant non substitué en 4b, suivie d'une alkylation avec un $R^{12}$-halogénure ou un $R^{12}$-sulfate et d'une hydrolyse finale.

**2.** Procédé selon la revendication 1, dans lequel au moins l'un de $R^3$, $R^5$, $R^7$, $R^9$, $R^{11}$ et $R^{12}$ est un groupe alkyle en $C_1$-$C_6$, de préférence un groupe méthyle, éthyle ou i-propyle.

**3.** Procédé selon la revendication 1 ou 2, dans lequel au moins l'un de $R^5$ et $R^8$ est un groupe mono- ou di(alkyle en $C_1$-$C_6$)amino, de préférence un groupe éthylamino ou diéthylamino.

**4.** Procédé selon la revendication 1 ou 2, dans lequel au moins l'un de $R^5$ et $R^8$ est un groupe hydroxy ou alcoxy en $C_1$-$C_6$, de préférence méthoxy.

**5.** Procédé selon l'une quelconque des revendications précédentes, dans lequel R, $R^1$, $R^2$, $R^4$, $R^6$ et $R^{10}$ représentent un atome d'hydrogène.

**6.** Procédé selon la revendication 1, dans lequel on obtient les composés suivants :
cis-4b,5,9b,10-tétrahydroindéno[1,2-b]indole
cis-4b,5,9b,10-tétrahydro-6,8-diméthylindéno[1,2-b]indole
cis-4b,5,9b,10-tétrahydro-5,8-diméthylindéno[1,2-b]indole
cis-4b,5,9b,10-tétrahydro-8-méthylindéno[1,2-b]indole
cis-4b,5,9b,10-tétrahydro-4b,6,8,9b-tétraméthylindéno[1,2-b]indole
cis-4b,5,9b,10-tétrahydro-8-isopropylindéno[1,2-b]indole
cis-4b,5,9b,10-tétrahydro-8-méthoxy-5-méthylindéno[1,2-b]indole
cis-4b,5,9b,10-tétrahydro-8-méthoxyindéno[1,2-b]indole
cis-4b,5,9b,10-tétrahydro-10,10-diméthylindéno[1,2-b]indole
cis-4b,5,9b,10-tétrahydro-9b-méthylindéno[1,2-b]indole
cis-4b,5,9b,10-tétrahydro-4b,9b-diméthylindéno[1,2-b]indole
cis-4b,5,9b,10-tétrahydro-4b,5,9b-triméthylindéno[1,2-b]indole
cis-4b,5,9b,10-tétrahydro-2-méthoxy-1,3-diméthylindéno[1,2-b]indole
cis-4b,5,9b,10-tétrahydro-2-méthoxy-1,3-diméthyl-8-isopropylindéno[1,2-b]-indole
cis-4b,5,9b,10-tétrahydro-4b-méthylindéno[1,2-b]indole
cis-4b,5,9b,10-tétrahydro-2-hydroxy-1,3-diméthyl-8-isopropylindéno[1,2-b]indole
cis-4b,5,9b,10-tétrahydro-2-hydroxy-1,3-diméthylindéno[1,2-b]indole
cis-4b,5,9b,10-tétrahydro-4b,8,9b-triméthylindéno[1,2-b]indole
cis-4b,5,9b,10-tétrahydro-8-isopropyl-4b,9b-diméthylindéno[1,2-b]indole
cis-4b,5,9b,10-tétrahydro-8-isopropyl-4b-méthylindéno[1,2-b]indole
cis-4b,5,9b,10-tétrahydro-2,8-diméthoxy-1,3-diméthylindéno[1,2-b]indole
cis-4b,5,9b,10-tétrahydro-4b,5,8,9b-tétraméthylindéno[1,2-b]indole
cis-4b,5,9b,10-tétrahydro-8-tert.-butylindéno[1,2-b]indole
cis-4b,5,9b,10-tétrahydro-8-méthoxy-7,9-diméthylindéno[1,2-b]indole

cis-4b,5,9b,10-tétrahydro-8-méthoxy-6-méthylindéno[1,2-b]indole
cis-4b,5,9b,10-tétrahydro-8-diéthylamino-5-éthylindéno[1,2-b]indole
cis-4b,5,9b,10-tétrahydro-2-diéthylaminoindéno[1,2-b]indole
cis-4b,5,9b,10-tétrahydro-8-tert.-butyl-4b-méthylindéno[1,2-b]indole
cis-4b,5,9b,10-tétrahydro-8-fluoroindéno[1,2-b]indole
cis-5,5a,6,10b,-tétrahydroindéno[2,1-b]indole
cis-5,5a,6,10b,-tétrahydro-9-méthoxyindéno[2,1-b]indole
cis-5,5a,6,10b,-tétrahydro-9-isopropylindéno[2,1-b]indole
cis-5,5a,6,10b,-tétrahydro-9-méthoxy-7-méthylindéno[2,1-b]indole
cis-4b,5,9b,10-tétrahydro-4,6-diméthyl-8-méthoxyindéno[1,2-b]indole
cis-4b,5,9b,10-tétrahydro-8-méthoxy-4b,6,9b-triméthylindéno[1,2-b]indole.

**7.** Procédé selon la revendication 1, dans lequel on obtient le cis-4b,5,9b,10-tétrahydro-8-méthoxy-6-méthylindéno[1,2-b]indole, ou un de ses énantiomères ou sels.

**8.** Procédé selon la revendication 1, dans lequel on obtient le cis-5,5a,6,10b-tétrahydro-9-méthoxy-7-méthylindéno[2,1-b]indole, ou un de ses énantiomères ou sels.

**9.** Procédé de préparation d'un composé de formule IA ou IB

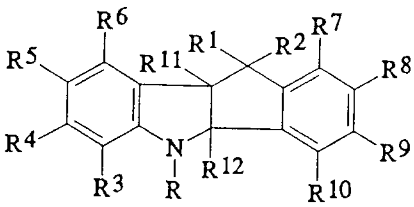
IA ou
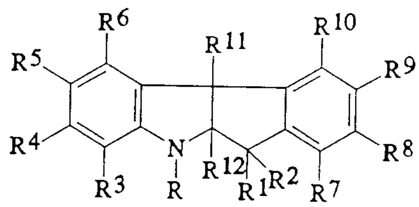
IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle en $C_1$-$C_6$,
$R^3$, $R^4$, $R^5$ et $R^6$ sont choisis indépendamment parmi un atome d'hydrogène, un groupe hydroxy ou halogéno, un groupe alkyle en $C_1$-$C_6$ ou un groupe alcoxy en $C_1$-$C_6$, un groupe mono- ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$, un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,
ou d'un de ses énantiomères et sels pharmaceutiquement acceptables ; destinés à être utilisés dans un procédé thérapeutique pratiqué sur le corps humain ou animal, comprenant les étapes consistant à :

a. réduire le 5,10-dihydroindéno[1,2-b]indole (DHII)

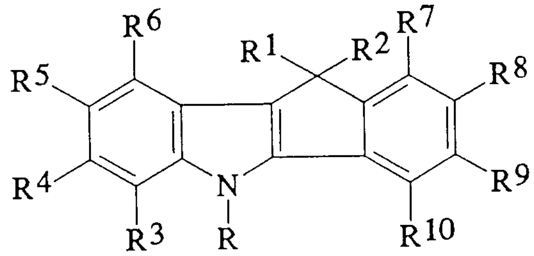

XI

où R, $R^1$, $R^2$, $R^3$, $R^4$, $R^5$, $R^6$, $R^7$, $R^8$, $R^9$ et $R^{10}$ sont définis comme dans la formule IA, si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

b. soumettre à une indolisation de Fischer une phénylhydrazine de formule II et une 1-indanone de formule III portant un substituant en position 2, puis réduire les indolénines (IV)

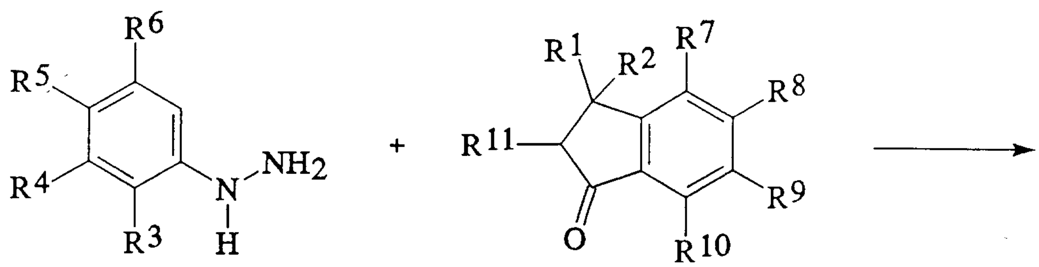

II III

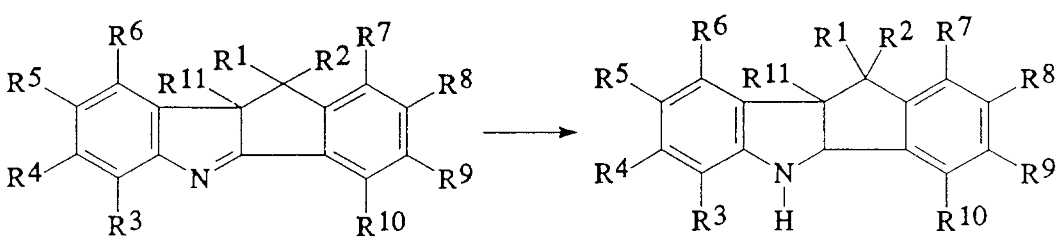

IV V

où $R^1$, $R^2$, $R^3$, $R^4$, $R^5$, $R^6$, $R^7$, $R^8$, $R^9$, $R^{10}$ et $R^{11}$ sont définis comme dans la formule IA, et si nécessaire, effectuer ensuite une N-alkylation avec un R-halogénure ou un R-sulfate, où R est défini comme dans la formule IA,

c. faire réagir des indolénines de formule IV avec des alkyllithiums ($R^{12}$-Li)

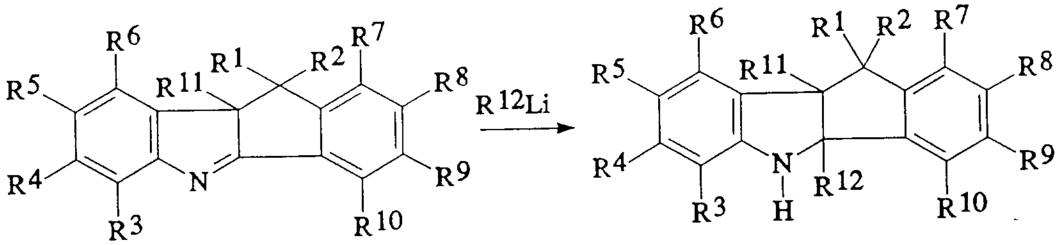


IV VI

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IA, et si nécessaire, effectuer ensuite une N-alkylation avec un R-halogénure ou un R-sulfate, où R est défini comme dans la formule IA,

d. réduire le 5,6-dihydroindéno[2,1-b]indole (iso-DHII), si nécessaire, effectuer auparavant une N-alkylation de l'iso-DHII avant la réduction, ou effectuer ensuite une N-alkylation de l'iso-THII avec un R-halogénure ou un R-sulfate, où R est défini comme dans la formule I,

e. pour des 5,5a,6,10b-tétrahydroindéno[2,1-b]indoles (IX) portant un substituant en 10b et leurs analogues, en utilisant des indan-2-ones (XII) portant un groupe substituant en C-3, effectuer une indolisation de Fischer avec des phénylhydrazines de formule (II), suivie d'une réaction du composé intermédiaire VIII avec un agent réducteur,

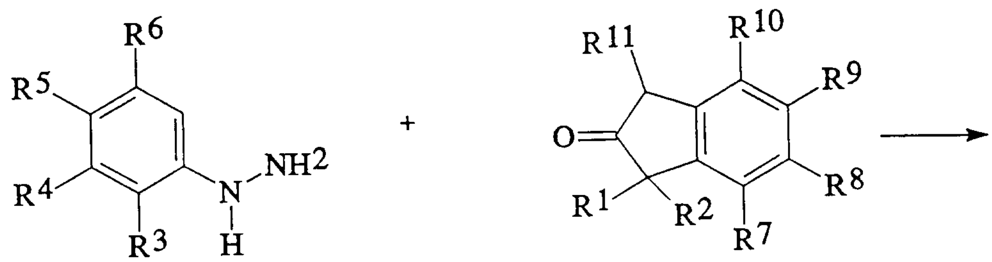

II XII

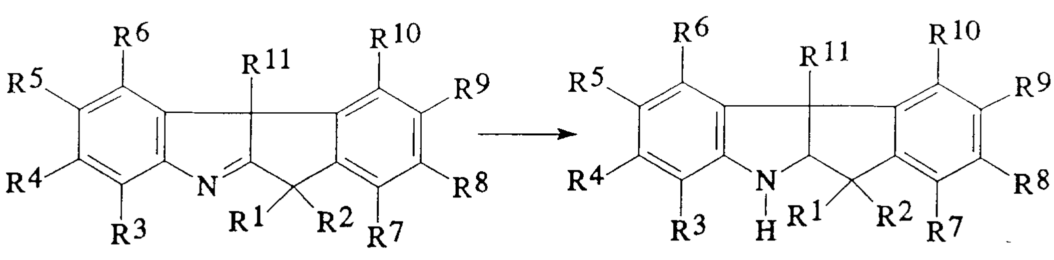

VIII IX

où $R^1$, $R^2$, $R^3$, $R^4$, $R^5$, $R^6$, $R^7$, $R^8$, $R^9$, $R^{10}$ et $R^{11}$ sont définis comme dans la formule IB, ou si nécessaire,

effectuer ensuite une N-alkylation avec un R-halogénure ou un R-sulfate, où R est défini comme dans la formule IB,

f. pour des 5,5a,6,10b-tétrahydroindéno[2,1-b]indoles portant un substituant en 5a,10b et leurs analogues, par réaction d'indolénines de formule VII avec des alkyllithiums $R^{12}$-Li,

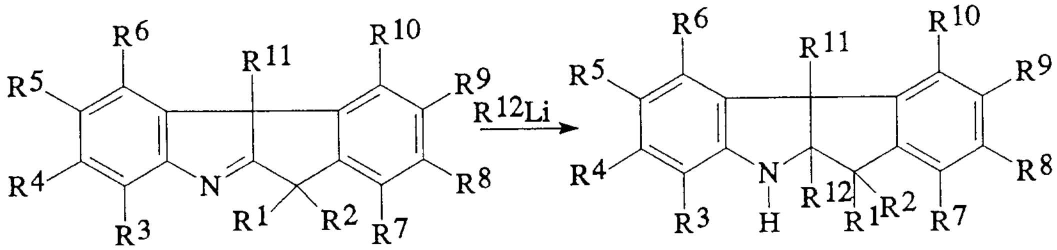

VIII IB

où $R^1$, $R^2$, $R^3$, $R^4$, $R^5$, $R^6$, $R^7$, $R^8$, $R^9$, $R^{10}$, $R^{11}$ et $R^{12}$ sont définis comme dans la formule IB, ou si nécessaire, effectuer ensuite une N-alkylation avec un R-halogénure ou un R-sulfate, où R est défini comme dans la formule IB,

g. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par N-alkylation du THII ou de l'iso-THII correspondant avec un R-halogénure ou un R-sulfate,

h. pour des 5-alkyl-THII ou des 6-alkyl-iso-THII, où R, $R^1$, $R^2$, $R^3$, $R^4$, $R^5$, $R^6$, $R^7$, $R^8$, $R^9$, $R^{10}$ sont définis comme dans la formule I, par réduction du 5-acyl-THII ou du 6-acyl-iso-THII correspondant,

i. pour THII ou iso-THII, où $R^3$-$R^6$ et/ou $R^7$-$R^{10}$ sont définis comme un groupe mono- ou di(alkyle en $C_1$-$C_6$)amino, et R, $R^1$, $R^2$, $R^{11}$ et $R^{12}$ sont définis comme dans la formule I, par réduction des composés nitro- 5-acyl-THII ou 6-acyl-iso-THII correspondants, suivie d'une N-alkylation et éventuellement, d'une hydrolyse acide,

j. pour THII ou iso-THII, où $R^3$-$R^6$ et/ou $R^7$-$R^{10}$ sont définis comme un groupe hydroxy, et R, $R^1$, $R^2$, $R^{11}$ et $R^{12}$ sont définis comme dans la formule I, par éther-désalkylation des composés THII ou iso-THII correspondants portant un substituant alcoxy,

k. pour THII ou iso-THII, où $R^{12}$ est un groupe alkyle inférieur et R, $R^1$-$R^{11}$ sont définis comme dans la formule I, par métallation de l'analogue correspondant non substitué en 4b, suivie d'une alkylation avec un $R^{12}$-halogénure ou un $R^{12}$-sulfate et d'une hydrolyse finale.

**10.** Procédé selon la revendication 9, dans lequel on obtient le cis-4b,5,9b,10-tétrahydro-8-méthoxy-6-méthylindéno[1,2-b]indole, ou un de ses énantiomères ou sels pharmaceutiquement acceptables, destiné à être utilisé dans un procédé thérapeutique pratiqué sur le corps humain ou animal.

**11.** Procédé selon la revendication 9, dans lequel on obtient le cis-5,5a,6,10b-tétrahydro-9-méthoxy-7-méthylindéno[2,1-b]indole, ou un de ses énantiomères ou sels pharmaceutiquement acceptables, destiné à être utilisé dans un procédé thérapeutique pratiqué sur le corps humain ou animal.

**12.** Procédé selon l'une quelconque des revendications 9 à 11, dans lequel le produit est destiné à être utilisé dans le traitement de lésions ischémiques ou de reperfusion, de thromboses et d'embolies.

**13.** Procédé selon l'une quelconque des revendications 9 à 11, dans lequel le produit est destiné à être utilisé dans le traitement curatif ou préventif de néoplasmes.

**14.** Procédé selon l'une quelconque des revendications 9 à 11, dans lequel le produit est destiné à être utilisé dans le traitement de la maladie de Parkinson, de la maladie d'Alzheimer ou du vieillissement.

**15.** Procédé selon l'une quelconque des revendications 9 à 11, dans lequel le produit est destiné à être utilisé dans le traitement de l'athérosclérose.

**16.** Procédé selon l'une quelconque des revendications 9 à 11, dans lequel le produit est destiné à être utilisé dans le traitement d'états allergiques/inflammatoires tels que l'asthme bronchique et le polyarthrite rhumatoïde.

**17.** Procédé selon l'une quelconque des revendications 9 à 11, dans lequel le produit est destiné à être utilisé dans le traitement de lésions dues à des produits chimiques, des radiations, des agents antinéoplasiques ou immunosuppresseurs.

**18.** Procédé selon l'une quelconque des revendications 9 à 17, dans lequel la formule IA ou IB est telle que définie dans l'une quelconque des revendications 1-6.

**19.** Procédé de préparation d'une composition pharmaceutique, consistant à mélanger un principe actif qui est un composé tel que défini dans l'une quelconque des revendications 1-6, un de ses énantiomères ou sels pharmaceutiquement acceptables, avec un véhicule pharmaceutiquement acceptable.

**20.** Procédé de préparation d'une composition pharmaceutique, consistant à mélanger un principe actif qui est le cis-4b,5,9b,10-tétrahydro-8-méthoxy-6-méthylindéno[1,2-b]indole, ou un de ses énantiomères ou sels pharmaceutiquement acceptables, avec un véhicule pharmaceutiquement acceptable.

**21.** Procédé de préparation d'une composition pharmaceutique, consistant à mélanger un principe actif qui est le cis-5,5a,6,10b-tétrahydro-9-méthoxy-7-méthylindéno[2,1-b]indole, ou un de ses énantiomères ou sels pharmaceutiquement acceptables, avec un véhicule pharmaceutiquement acceptable.

**22.** Utilisation d'un composé de formule IA ou IB, tel que défini dans l'une quelconque des revendications 1 à 9, dans la fabrication d'un médicament destiné au traitement de lésions ischémiques ou de reperfusion, de thromboses, d'embolies, d'athérosclérose, de la maladie de Parkinson, de la maladie d'Alzheimer, du vieillissement, de néoplasmes, d'états allergiques ou inflammatoires tels que l'asthme bronchique et le polyarthrite rhumatoïde, et de lésions dues à des produits chimiques, des radiations, des agents antinéoplasiques ou immunosuppresseurs.

**23.** Composition comprenant un composé sensible à la détérioration par oxydation et un composé de formule IA ou IB

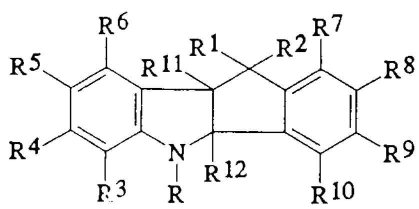

IA ou

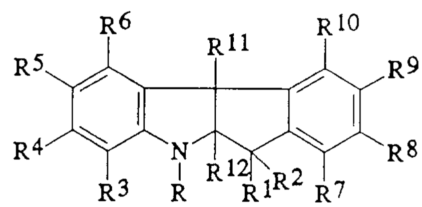

IB

où R représente un atome d'hydrogène, un groupe alkyle en $C_1$-$C_{24}$ ou un groupe $COR^{15}$,
$R^1$, $R^2$, $R^{11}$ et $R^{12}$ sont choisis indépendamment parmi un atome d'hydrogène ou un groupe alkyle inférieur,
$R^3$, $R^4$, $R^5$ et $R^6$ sont choisis indépendamment parmi un atome d'hydrogène, un groupe hydroxy, un atome d'halogène, un groupe alkyle en $C_1$-$C_6$, un groupe alcoxy en $C_1$-$C_6$, un groupe mono-ou di(alkyle en $C_1$-$C_6$)amino, $NH_2$ ou $NR^{13}COR^{14}$,
$R^7$, $R^8$, $R^9$ et $R^{10}$ sont choisis indépendamment parmi un atome d'hydrogène, un groupe hydroxy, un groupe alkyle en $C_1$-$C_6$, un groupe alcoxy en $C_1$-$C_6$ ou un groupe mono- ou di(alkyle en $C_1$-$C_6$)amino, $NH_2$ ou $NR^{13}COR^{14}$,
$R^{13}$, $R^{14}$ et $R^{15}$ sont choisis indépendamment parmi un atome d'hydrogène ou un groupe alkyle en $C_1$-$C_6$,
étant entendu que lorsque R représente un groupe $COR^{15}$, alors au moins l'un de $R^3$ à $R^{10}$ représente un groupe hydroxy ou un groupe mono- ou di(alkyle en $C_1$-$C_6$)amino,
ou un de ses énantiomères ou sels.

**24.** Composition selon la revendication 23, dans laquelle le composé de formule IA ou IB est tel que défini dans l'une quelconque des revendications 1-6.

25. Composition comprenant un composé sensible à la détérioration par oxydation et le cis-4b,5,9b,10-tétrahydro-8-méthoxy-6-méthylindéno[1,2-b]indole, ou un énantiomère ou un sel de celui-ci.

26. Composition comprenant un composé sensible à la détérioration par oxydation et le cis-5,5a,6,10b-tétrahydro-9-méthoxy-7-méthylindéno[2,1-b]indole, ou un énantiomère ou un sel de celui-ci.

27. Procédé non médical pour stabiliser un composé sensible à la détérioration par oxydation, par mise en contact du composé sensible avec un composé de formule IA ou IB tel que défini à la revendication 23 ou 24

28. Procédé non médical pour stabiliser un composé sensible à la détérioration par oxydation, par mise en contact du composé sensible avec le cis-4b,5,9b,10-tétrahydro-8-méthoxy-6-méthylindéno[1,2-b]indole ou un énantiomère ou un sel de celui-ci.

29. Procédé non médical pour stabiliser un composé sensible à la détérioration par oxydation, par mise en contact du composé sensible avec le cis-5,5a,6,10b-tétrahydro-9-méthoxy-7-méthylindéno[2,1-b]indole ou un énantiomère ou un sel de celui-ci.

30. Procédé pour la conservation d'un produit sensible à la détérioration par oxydation, par mise en contact du produit sensible avec un composé de formule IA ou IB, tel que défini dans la revendication 23 ou 24

31. Procédé pour la conservation d'un produit sensible à la détérioration par oxydation, par mise en contact du produit sensible avec le cis-4b,5,9b,10-tétrahydro-8-méthoxy-6-méthylindéno[1,2-b]indole ou un énantiomère ou un sel de celui-ci.

32. Procédé pour la conservation d'un produit sensible à la détérioration par oxydation, par mise en contact du produit sensible avec le cis-5,5a,6,10b-tétrahydro-9-méthoxy-7-méthylindéno[2,1-b]indole ou un énantiomère ou un sel de celui-ci.

33. Utilisation d'un composé de formule IA ou IB tel que défini dans la revendication 23 ou 24, en tant qu'agent de conservation d'un produit sensible à la détérioration par oxydation.

34. Utilisation du cis-4b,5,9b,10-tétrahydro-8-méthoxy-6-méthylindéno[1,2-b]indole ou d'un énantiomère ou d'un sel de celui-ci, en tant qu'agent de conservation d'un produit sensible à la détérioration par oxydation.

35. Utilisation du cis-5,5a,6,10b-tétrahydro-9-méthoxy-7-méthylindéno[2,1-b]indole ou d'un énantiomère ou d'un sel de celui-ci, en tant qu'agent de conservation d'un produit sensible à la détérioration par oxydation.